(19) Europäisches Patentamt
European Patent Office
Office européen des brevets

(11) EP 2 992 910 A1

(12) EUROPEAN PATENT APPLICATION
published in accordance with Art. 153(4) EPC

(43) Date of publication:
**09.03.2016 Bulletin 2016/10**

(21) Application number: **14792315.5**

(22) Date of filing: **02.04.2014**

(51) Int Cl.:
***A61L 31/00*** *(2006.01)* ***A61K 31/23*** *(2006.01)*
***A61K 31/232*** *(2006.01)* ***A61K 47/10*** *(2006.01)*
***A61K 47/26*** *(2006.01)* ***A61K 47/34*** *(2006.01)*
***A61K 47/36*** *(2006.01)* ***A61K 47/44*** *(2006.01)*
***A61L 33/00*** *(2006.01)* ***A61P 41/00*** *(2006.01)*
***C07C 69/587*** *(2006.01)* ***A61K 8/37*** *(2006.01)*

(86) International application number:
**PCT/JP2014/059785**

(87) International publication number:
**WO 2014/178256 (06.11.2014 Gazette 2014/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.05.2013 JP 2013096499**

(71) Applicant: **Farnex Incorporated**
**Ota-ku**
**Tokyo 145-0064 (JP)**

(72) Inventors:
- **TABATA, Yasuhiko**
  **Kyoto-shi**
  **Kyoto 606-8501 (JP)**
- **HIRAI, Kenjiro**
  **Kyoto-shi**
  **Kyoto 606-8501 (JP)**
- **HIJIKURO, Ichiro**
  **Yokohama-shi**
  **Kanagawa 226-8510 (JP)**
- **TANOMURA, Masahisa**
  **Yokohama-shi**
  **Kanagawa 226-8510 (JP)**
- **MORI, Sayaka**
  **Yokohama-shi**
  **Kanagawa 226-8510 (JP)**

(74) Representative: **Webster, Jeremy Mark et al**
**Mewburn Ellis LLP**
**City Tower**
**40 Basinghall Street**
**London EC2V 5DE (GB)**

# (54) ADHESION PREVENTING AGENT

(57) The present invention relates to an adhesion preventing agent comprising an amphipathic compound having the following general formula (I):

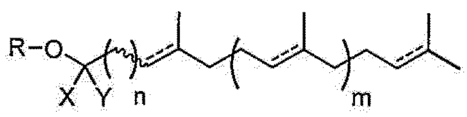

(I)

wherein X and Y each denotes a hydrogen atom or together denote an oxygen atom, n denotes an integer from 0 to 2, m denotes the integer 1 or 2, AA:
AA
denotes a single bond or double bond, and R denotes a hydrophilic group having two or more hydroxyl groups.

EP 2 992 910 A1

## Description

### Technical Field

The present invention relates to an agent for preventing adhesion of tissue in the body.

### Background Art

Organ/tissue adhesion is a typical complication that occurs after surgery. The incidence of the adhesion is 55% or more in abdominal surgery cases. Adhesion causes patients to continuously suffer from serious conditions such as chronic abdominal pain, intestinal obstruction, and infertility. Adhesion is a universal postoperative problem that may occur after chest surgery and brain surgery as well as abdominal surgery.

In order to prevent postoperative adhesion, insertion of a film or sheet of adhesion preventing materials serving as a barrier, between an affected area and an organ that might adhere thereto has been used in clinical practice. For example, Seprafilm (registered trademark), which is a semi-transparent film containing sodium hyaluronate and carboxymethylcellulose at a ratio of 2:1 and developed by Genzyme Corporation, has been used as a bioabsorbable adhesion preventing material after surgery. However, organs have complex shapes that do not have just flat surfaces, which makes it difficult to apply a film or sheet of adhesion preventing material to a tissue/ or organ so as to completely cover an irregular surface thereof or an area in a small operative field. In addition, the film or sheet of adhesion preventing materials are problematic in that they tend to adhere to surgical gloves, and they tend to become torn or displaced during surgery because of the difficulties of properly putting the materials on injured areas, thereby making advanced surgical techniques necessary in handling the materials.

In recent years, an adhesion preventing agent comprising hydrogel containing a polysaccharide derivative has been developed (Patent Document 1). In addition, a gel-like adhesion preventing agent comprising a crosslinkable polysaccharide derivative into which active ester groups have been introduced is known (Patent Document 2). However, most such adhesion preventing agents have high viscosity, and thus it is difficult to apply them using simple techniques such as injection. Therefore, a large-scale apparatus can be required to apply them, and it is difficult to apply them to a small area, both of which are problematic. For such reason, the development of a highly operable adhesion preventing agent that can be applied in a simple manner and to a small area has been desired.

Meanwhile, a variety of amphipathic compounds are known to form liquid crystals in water and are used for various applications in the fields of cosmetics, pharmaceutical products, and the like. For example, drug delivery systems (DDS) using amphipathic compounds are under active development. Various forms of drug delivery carriers have been produced, including a drug delivery system in which a drug is embedded in an intraliposomal aqueous phase or a lipid bilayer prepared from lamellar liquid crystal. In particular, non-lamellar liquid crystal such as cubic liquid crystal or reverse hexagonal liquid crystal has a high degree of structural stability and is capable of stably retaining various drugs within itself, and thus is attracting attention as a particularly useful drug delivery carrier. For example, Patent Document 3 discloses a drug delivery system using a composition prepared by adding a surfactant and ethanol to a lipid mixture of soybean phosphatidylcholine (SPC) and diacylglycerol (GDO), which forms a non-lamellar liquid crystal. However, most of cubic liquid crystals found in amphipathic compound/water systems can remain stable only within narrow ranges of concentration and/or temperature. Recently, amphipathic compounds capable of forming cubic liquid crystals that exhibit high stability at low temperatures (less than 6°C) have been developed, and the use of the liquid crystals in sustained release formulations has also been reported (Patent Document 4). However, such liquid crystal compounds have high viscosity and thus do not allow the compounds to pass through a thin injection needle (e.g., 30 gauge), and their use has difficulty in injections. Amphipathic compounds capable of stably forming cubic liquid crystals and having lower viscosities have been developed as a base for injections (Patent Document 5). However, no medical products using such amphipathic compounds as medical materials but not as drug delivery carriers have been developed.

### Citation List

#### Patent Documents

Patent Document 1: International Patent Publication WO 2010/119994
Patent Document 2: International Patent Publication WO 2005/087289
Patent Document 3: International Patent Publication WO 2006/077362
Patent Document 4: International Patent Publication WO 2006/043705
Patent Document 5: International Patent Publication WO 2011/078383

Summary of the Invention

Problem to Be Solved by the Invention

An object of the present invention is to provide an adhesion preventing agent that can be readily applied.

Means for Solving the Problem

As a result of intensive studies to achieve the above object, the present inventors have found that a certain amphipathic compound (lipid) is capable of exhibiting a tissue adhesion preventing effect by applying it by a simple method such as spraying or spreading. Thus, the present inventors have completed the present invention.
The present invention includes the following [1] to [3].
[1] An adhesion preventing agent comprising an amphipathic compound having the following general formula (I):

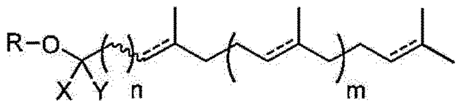

(I)

wherein X and Y each denotes a hydrogen atom or together denote an oxygen atom, n denotes an integer from 0 to 2, m denotes the integer 1 or 2, the designation: denotes a single bond or double bond, and R denotes a hydrophilic group having two or more hydroxyl groups.
In a preferred embodiment, n denotes the integer 1 or 2 in the above formula.
In a preferred embodiment, R in the formula denotes a hydrophilic group generated by removal of one hydroxyl group from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, and xylitol.
In a preferred embodiment, R in the above formula denotes a hydrophilic group generated by removal of one hydroxyl group from glycerol, erythritol, diglycerol, or xylose.
Preferred examples of the amphipathic compound include the following:

mono-O-(5,9,13-trimethyltetradec-4-enoyl)glycerol;
mono-O-(5,9,13,17-tetramethyloctadec-4-enoyl)diglycerol;
mono-O-(5,9,13-trimethyltetradecanoyl)glycerol;
mono-O-(5,9,13,17-tetramethyloctadecanoyl)erythritol;
1-O-(3,7,11,15-tetramethylhexadecyl)-β-D-xylopyranoside; and
mono-O-(5,9,13-trimethyltetradec-4,8,12-trienoyl)glycerol.

In a preferred embodiment, the adhesion preventing agent may further comprise a pharmaceutically acceptable carrier. A preferred example of the carrier is a liquid carrier and/or a gas carrier. Preferably, a liquid carrier comprises at least one selected from the group consisting of silicone oil, alcohol, and an aqueous medium. Preferably, the adhesion preventing agent according to the present invention further comprises a pharmaceutically acceptable surfactant. The adhesion preventing agent according to the present invention may comprise hyaluronic acid or a salt thereof.
[2] An amphipathic compound having the following general formula (II) or a salt thereof:

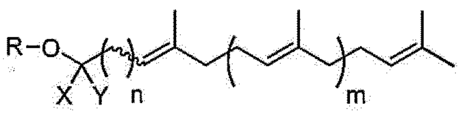

(II)

wherein X and Y each denotes a hydrogen atom or together denote an oxygen atom, n denotes an integer from 0 to 2, m denotes 1 or 2, and R denotes a hydrophilic group generated by removal of one hydroxyl group from any one selected from the group consisting of glycerol, erythritol, pentaerythritol, diglycerol, glyceric acid, and xylose.
In a preferred embodiment, n denotes 1 or 2 and m denotes 2 in the above formula.

Preferred examples of the amphipathic compound include the following:

mono-O-(5,9,13,17-tetramethyloctadec-4,8,12,16-tetraenoyl)glycerol,
mono-O-(5,9,13,17-tetramethyloctadec-4,8,12,16-tetraenoyl)erythritol,
mono-O-(5,9,13,17-tetramethyloctadec-4,8,12,16-tetraenoyl)pentaerythritol,
mono-O-(5,9,13-trimethyltetradec-4,8,12-trienyl)erythritol,
mono-O-(5,9,13-trimethyltetradec-4,8,12-trienyl)pentaerythritol,
mono-O-(3,7,11,15-tetramethylhexadec-2,6,10,14-tetraenoyl)pentaerythritol,
mono-O-(3,7,11,15-tetramethylhexadec-2,6,10,14-tetraenyl)erythritol,
mono-O-(5,9,13,17-tetramethyloctadec-4,8,12,16-tetraenyl)erythritol, and
mono-O-(5,9,13,17-tetramethyloctadec-4,8,12,16-tetraenyl)pentaerythritol.

[3] A method for preventing adhesion of an affected area, comprising applying the adhesion preventing agent according to [1] above to the affected area.

This application includes the disclosure in Japanese Patent Application No. 2013-096499, from which the present application claims priority.

Effects of the Invention

According to the present invention, an adhesion preventing effect can be obtained by an easy application method.

Brief Description of the Drawings

[Fig. 1] Fig. 1 shows the result of SAXS analysis of mono-O-(5,9,13-trimethyltetradec-4,8,12-trienoyl)glycerol.
[Fig. 2] Fig. 2 shows the result of SAXS analysis of mono-O-(5,9,13,17-tetramethyloctadec-4,8,12,16-tetraenoyl)glycerol.
[Fig. 3] Fig. 3 shows the result of SAXS analysis of mono-O-(5,9,13,17-tetramethyloctadec-4,8,12,16-tetraenoyl)erythritol.
[Fig. 4] Fig. 4 shows the result of SAXS analysis of mono-O-(5,9,13,17-tetramethyloctadec-4,8,12,16-tetraenoyl)pentaerythritol.
[Fig. 5] Fig. 5 includes photos showing that tissues were coated by sprayingtest sample 18 (C17 glycerin ester (o/w)). A: rat upper parietal peritoneum before spraying; B: rat upper parietal peritoneum after spraying. C: rat liver before spraying; D: rat liver after spraying.
[Fig. 6] Fig. 6 includes photos showing that tissues were coated by spraying test sample 13 (C17 glycerin ester) and physiological saline. A: rat liver before spraying; B: rat liver after spraying.
[Fig. 7] Fig. 7 shows the result of SAXS analysis of liquid crystal gel of mono-O-(5,9,13-trimethyltetradec-4,8,12-trienyl)erythritol.
[Fig. 8] Fig. 8 shows the result of SAXS analysis of liquid crystal gel of mono-O-(5,9,13-trimethyltetradec-4,8,12-trienyl)pentaerythritol.
[Fig. 9] Fig. 9 shows the result of SAXS analysis of liquid crystal gel of mono-O-(3,7,11,15-tetramethylhexadec-2,6,10,14-tetraenyl)erythritol.
[Fig. 10] Fig. 10 shows the result of SAXS analysis of liquid crystal gel of mono-O-(3,7,11,15-tetramethylhexadec-2,6,10,14-tetraenoyl)pentaerythritol.
[Fig. 11] Fig. 11 shows the result of SAXS analysis of liquid crystal gel of mono-O-(5,9,13,17-tetramethyloctadec-4,8,12,16-tetraenyl)erythritol.
[Fig. 12] Fig. 12 shows the result of SAXS analysis of liquid crystal gel of mono-O-(5,9,13,17-tetramethyloctadec-4,8,12,16-tetraenyl)pentaerythritol.
[Fig. 13] Fig. 13 shows the result of SAXS analysis of an o/w dispersion comprising C17 glycerin ester.

Embodiments for Carrying Out the Invention

The present invention is described in detail as follows.

1. Amphipathic compound

The adhesion preventing agent according to the present invention comprises an amphipathic compound having the following general formula (I):

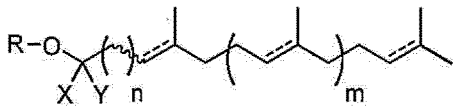

(I) .

In the general formula (I), X and Y each denotes a hydrogen atom or together denote an oxygen atom.
In the general formula (I), n denotes an integer from 0 to 2 (preferably 1 or 2), and m denotes 1 or 2. The combination of n and m for the amphipathic compound having the general formula (I) may be: n=0, m=1; n=0, m=2; n=1, m=1; n=1, m=2; n=2, m=1; or n=2, m=2.
In the above formula, the designation:

denotes a single bond or double bond.

Further, R in the general formula (I) denotes a hydrophilic group having two or more hydroxyl groups. The hydrophilic group may be, but not limited to, for example, a hydrophilic group generated by removal of one hydroxyl group from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, and xylitol. Particularly preferably, R in the general formula (I) denotes a hydrophilic group generated by removal of one hydroxyl group from glycerol, erythritol, diglycerol, glyceric acid, or xylose.

In addition, in the context of the present invention, the designation in the general formula (I): 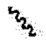 means that the amphipathic compound is an E-(cis-) or Z-(trans-) geometric isomer, or a mixture thereof.
An example of the amphipathic compound having the general formula (I) is an amphipathic compound having the following general formula (II) (polyunsaturated fatty acid ester):

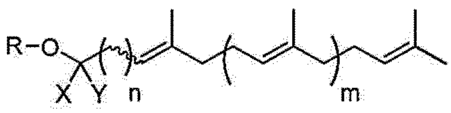

(I I) .

In the general formula (II), X and Y each denotes a hydrogen atom or together denote an oxygen atom, n denotes an integer from 0 to 2 (preferably 1 or 2), and m denotes 1 or 2. R in the general formula (II) denotes a hydrophilic group generated by removal of one hydroxyl group from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, and xylitol. A preferred example of R is a hydrophilic group generated by removal of one hydroxyl group from any one selected from the group consisting of glycerol, erythritol, pentaerythritol, diglycerol, glyceric acid, and xylose. When X and Y each denotes a hydrogen atom, R preferably denotes a hydrophilic group generated by removal of one hydroxyl group from any one selected from the group consisting of glycerol, erythritol, pentaerythritol, diglycerol, glyceric acid, and xylose. When X and Y together denote an oxygen atom (ester bond), R preferably denotes a hydrophilic group generated by removal of one hydroxyl group from any one selected from the group consisting of glycerol, erythritol, pentaerythritol, and diglycerol. However, in the case of n=0, R preferably denotes a hydrophilic group generated by removal of one hydroxyl group from any one selected from the group consisting of erythritol, pentaerythritol, diglycerol, and xylose.

In addition, in the context of the present invention, the designation in the general formula (II): 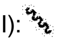 means that the amphipathic compound is an E-(cis-) or Z-(trans-) geometric isomer, or a mixture thereof.
Specific preferred examples of the amphipathic compound having the general formula (II) include the following ester compounds.
- Compounds where n=0 and m=1
  mono-O-(3,7,11-trimethyldodec-2,6,10-trienoyl)glycerol 3,7,11-trimethyldodec-2,6,10-trienyl glycerate
- Compounds where n=0 and m=2
  mono-O-(3,7,11,15-tetramethylhexadec-2,6,10,14-tetraenoyl)glycerol

mono-O-(3,7,11,15-tetramethylhexadec-2,6,10,14-tetraenoyl)erythritol
mono-O-(3,7,11,15-tetramethylhexadec-2,6,10,14-tetraenoyl)pentaerythritol
mono-O-(3,7,11,15-tetramethylhexadec-2,6,10,14-tetraenoyl)diglycerol 3,7,11,15-tetramethylhexadec-2,6,10,14-tetraenyl glycerate

- Compounds where n=1 and m=1
  mono-O-(4,8,12-trimethyltridec-3,7,11-trienoyl)glycerol 4,8,12-trimethyltridec-3,7,11-trienyl glycerate
- Compounds where n=1 and m=2
  mono-O-(4,8,12,16-tetramethylheptadec-3,7,11,15-tetraenoyl)glycerol
  mono-O-(4,8,12,16-tetramethylheptadec-3,7,11,15-tetraenoyl)erythritol
  mono-O-(4,8,12,16-tetramethylheptadec-3,7,11,15-tetraenoyl)pentaerythritol
  mono-O-(4,8,12,16-tetramethylheptadec-3,7,11,15-tetraenoyl)diglycerol 4,8,12,16-tetramethylheptadec-3,7,11,15-tetraenyl glycerate
- Compounds where n=2 and m=1
  mono-O-(5,9,13-trimethyltetradec-4,8,12-trienoyl)glycerol
  mono-O-(5,9,13-trimethyltetradec-4,8,12-trienoyl)erythritol
  mono-O-(5,9,13-trimethyltetradec-4,8,12-trienoyl)pentaerythritol
  mono-O-(5,9,13-trimethyltetradec-4,8,12-trienoyl)diglycerol 5,9,13-trimethyltetradec-4,8,12-trienyl glycerate
- Compounds where n=2 and m=2
  mono-O-(5,9,13,17-tetramethyloctadec-4,8,12,16-tetraenoyl)glycerol
  mono-O-(5,9,13,17-tetramethyloctadec-4,8,12,16-tetraenoyl)erythritol
  mono-O-(5,9,13,17-tetramethyloctadec-4,8,12,16-tetraenoyl)pentaerythritol
  mono-O-(5,9,13,17-tetramethyloctadec-4,8,12,16-tetraenoyl)diglycerol 5,9,13,17-tetramethyloctadec-4,8,12,16-tetraenyl glycerate

Examples of the amphipathic compound having the general formula (II) include the following ether compounds or glycoside compounds.
- Compounds where n=0 and m=1
  mono-O-(,3,7,11-trimethyldodec-2,6,10-trienyl)glycerol
  mono-O-(,3,7,11-trimethyldodec-2,6,10-trienyl)erythritol
  mono-O-(3,7,11-trimethyldodec-2,6,10-trienyl)pentaerythritol 1-O-(3,7,11-trimethyldodec-2,6,10-trienyl)-D-xylopyranoside
- Compounds where n=0 and m=2
  mono-O-(3,7,11,15-tetramethylhexadec-2,6,10,14-tetraenyl)glycerol
  mono-O-(3,7,11,15-tetramethylhexadec-2,6,10,14-tetraenyl)erythritol
  mono-O-(3,7,11,15-tetramethylhexadec-2,6,10,14-tetraenyl)pentaerythritol 1-O-(3,7,11,15-tetramethylhexadec-2,6,10,14-tetraenyl)-D-xylopyranoside
  mono-O-(3,7,11,15-tetramethylhexadec-2,6,10,14-tetraenyl)diglycerol
- Compounds where n=1 and m=1
  mono-O-(4,8,12-trimethyltridec-3,7,11-trienyl)glycerol
  mono-O-(4,8,12-trimethyltridec-3,7,11-trienyl)erythritol
  mono-O-(4,8,12-trimethyltridec-3,7,11-trienyl)pentaerythritol 1-O-(4,8,12-trimethyltridec-3,7,11-trienyl)-D-xylopyranoside
- Compounds where n=1 and m=2
  mono-O-(4,8,12,16-tetramethylheptadec-3,7,11,15-tetraenyl)glycerol
  mono-O-(4,8,12,16-tetramethylheptadec-3,7,11,15-tetraenyl)erythritol
  mono-O-(4,8,12,16-tetramethylheptadec-3,7,11,15-tetraenoyl)pentaerythritol 1-O-(4,8,12,16-tetramethylheptadec-3,7,11,15-tetraenyl)-D-xylopyranoside
- Compounds where n=2 and m=1
  mono-O-(5,9,13-trimethyltetradec-4,8,12-trienyl)glycerol
  mono-O-(5,9,13-trimethyltetradec-4,8,12-trienyl)erythritol
  mono-O-(5,9,13-trimethyltetradec-4,8,12-trienyl)pentaerythritol 1-O-(5,9,13-trimethyltetradec-4,8,12-trienyl)-D-xylopyranoside
  mono-O-(,5,9,13-trimethyltetradec-4,8,12-trienyl)diglycerol
- Compounds where n=2 and m=2
  mono-O-(5,9,13,17-tetramethyloctadec-4,8,12,16-tetraenyl)glycerol
  mono-O-(5,9,13,17-tetramethyloctadec-4,8,12,16-tetraenyl)erythritol

mono-O-(5,9,13,17-tetramethyloctadec-4,8,12,16-tetraenyl)pentaerythritol 1-O-(5,9,13,17-tetramethyloctadec-4,8,12,16-tetraenyl)-D-xylopyranoside
mono-O-(5,9,13,17-tetramethyloctadec-4,8,12,16-tetraenyl)diglycerol

Another example of the amphipathic compound having the general formula (I) is an amphipathic compound having the following general formula (III):

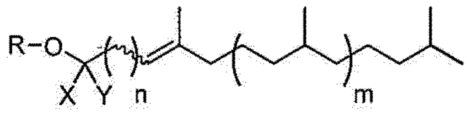

(III).

In the general formula (III), X and Y each denotes a hydrogen atom or together denote an oxygen atom, n denotes an integer from 0 to 2 (preferably 1 or 2), and m denotes 1 or 2.

In the general formula (III), R denotes a hydrophilic group having two or more hydroxyl groups. The hydrophilic group include may be, but not limited to, for example, a hydrophilic group generated by removal of one hydroxyl group from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, and xylitol.

In addition, the designation in the general formula (III): 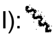 means that the amphipathic compound is an E-(cis-) or Z-(trans-) geometric isomer, or a mixture thereof.

Specific preferred examples of the amphipathic compound having the general formula (III) include the following compounds.

mono-O-(5,9,13-trimethyltetradec-4-enoyl)glycerol
mono-O-(5,9,13,17-tetramethyloctadec-4-enoyl)glycerol
mono-O-(5,9,13,17-tetramethyloctadec-4-enoyl)erythritol
mono-O-(5,9,13,17-tetramethyloctadec-4-enoyl)pentaerythritol
mono-O-(5,9,13,17-tetramethyloctadec-4-enoyl)diglycerol

Further, another example of an amphipathic compound having the general formula (I) is an amphipathic compound having the following general formula (IV):

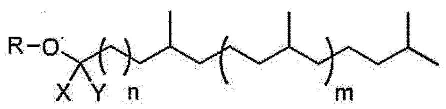

(IV).

In the general formula (IV), X and Y each denotes a hydrogen atom or together denote an oxygen atom, n denotes an integer from 0 to 2 (preferably 1 or 2), and m denotes 1 or 2.

In the general formula (IV), R denotes a hydrophilic group having two or more hydroxyl groups. The hydrophilic group may be, but not limited to, for example, a hydrophilic group generated by removal of one hydroxyl group from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, and xylitol.

Specific preferred examples of the amphipathic compound having the general formula (IV) include the following compounds.

mono-O-(5,9,13-trimethyltetradecanoyl)glycerol 1-O-(3,7,11,15-tetramethylhexadecyl)-β-D-xylopyranoside
mono-O-(5,9,13,17-tetramethyloctadecanoyl)glycerol
mono-O-(5,9,13,17-tetramethyloctadecanoyl)erythritol
mono-O-(5,9,13,17-tetramethyloctadecanoyl)pentaerythritol 1-O-(5,9,13,17-tetramethyloctadecanyl)-β-D-xylopyranoside

2. Properties of amphipathic compounds

The amphipathic compound to be used for the adhesion preventing agent according to the present invention

is a liquid crystal compound and capable of forming a non-lamellar liquid crystal in an aqueous medium. The adhesion preventing effect of the present invention can be obtained as a result of coating of the tissue surface with non-lamellar liquid crystals formed by an amphipathic compound. Herein, an aqueous medium containing an amphipathic compound may be referred to as an "amphipathic compound/water system."

Non-lamellar liquid crystal formed by the amphipathic compound used in the present invention is preferably type II (water-in-oil) liquid crystal wherein hydrophobic groups are oriented outward. Specifically, the non-lamellar liquid crystal is more preferably cubic liquid crystal or reverse hexagonal liquid crystal.

Cubic liquid crystal is preferably type II cubic liquid crystal. Cubic liquid crystal structures are generally classified into type I and type II. Cubic liquid crystal having an "oil-in-water" structure is referred to as type I cubic liquid crystal, and in contrast, cubic liquid crystal having a "water-in-oil" structure is referred to as type II cubic liquid crystal. Type I and type II can be determined on the basis of the phase behavior of an amphipathic compound/water system. For example, in the case of type I, as the water content of an amphipathic compound/water system is increased, it is transformed to another type of liquid crystal (e.g., lamellar liquid crystal) and then to micelles, and it is finally transformed into a uniform aqueous solution. On the other hand, in the case of the type II liquid crystal, when its water content reaches a certain level or higher, it is transformed into a double phase of "liquid crystal + excess water" in which liquid crystal containing a saturated volume of water and excess water coexist. Thus, in the type II, even if the water content is increased, no uniform aqueous solution is formed.

Cubic liquid crystal may also be cubic liquid crystal belonging to the crystallographic space group Ia3d (hereinafter, Ia3d cubic liquid crystal), cubic liquid crystal belonging to the crystallographic space group Pn3m (hereinafter, Pn3m cubic liquid crystal), or cubic liquid crystal belonging to the crystallographic space group Im3m (hereinafter, Im3m cubic liquid crystal). Cubic liquid crystal is more preferably Pn3m cubic liquid crystal.

Aqueous media in which the amphipathic compound according to the present invention can form a non-lamellar liquid crystal include, but not limited to, water such as sterile water, purified water, distilled water, ion exchanged water, or ultrapure water; electrolyte aqueous solutions such as physiological saline, aqueous sodium chloride solution, aqueous calcium chloride solution, aqueous magnesium chloride solution, aqueous sodium sulfate solution, aqueous potassium sulfate solution, aqueous sodium carbonate solution, and aqueous sodium acetate solution; buffer solutions such as phosphate buffer and Tris-HCl buffer; aqueous solutions containing water-soluble organic substances such as glycerin, ethylene glycol, and ethanol; aqueous solutions containing sugar molecules such as glucose, sucrose, and maltose; aqueous solutions containing water soluble polymers such as polyethylene glycol and polyvinyl alcohol; aqueous solutions containing surfactants such as octyl glucoside, dodecyl maltoside, and Pluronic (polyethylene glycol/polypropylene glycol/polyethylene glycol copolymer); and body fluids such as intracellular fluid, extracellular fluid, intercellular fluid, lymph fluid, spinal fluid, blood, gastric juice, serum, blood plasma, saliva, tears, seminal fluid, and urine.

The amphipathic compound according to the present invention exhibits high stability under broad environmental conditions. For example, the amphipathic compound according to the present invention is characterized in that it has, as a hydrophobic group, an isoprenoid chain, and thus, unlike an amphipathic compound having, as a hydrophobic group, a linear chain of fatty acid such as oleic acid, it has high resistance to hydrolysis and relatively high oxidation stability. The amphipathic compound according to the present invention has also a wide temperature range that allows liquid crystal formation and low Krafft temperature, so that it can stably form a liquid crystal even under low temperatures (6°C or less, preferably 0°C or less).

When the amphipathic compound according to the present invention is applied to tissue *in vivo,* it can stably form type II non-lamellar liquid crystals in body fluid, including, but are not limited to, intracellular fluid, extracellular fluid, intercellular fluid, lymph fluid, spinal fluid, blood, serum, and blood plasma, thereby forming a coating. Further, when a liquid mixture of the amphipathic compound of the present invention and the aqueous medium described above is applied to living tissue, the amphipathic compound is capable of stably forming a type II non-lamellar liquid crystal on tissue, thereby forming a coating.

Structural analysis of the liquid crystal formed by the amphipathic compound can be carried out by conventional methods, such as the following methods.

(1) Observation with polarizing microscope

A penetration method can be used as a method for easily determining whether or not an amphipathic compound can form a liquid crystal in an aqueous medium and/or when the amphipathic compound forms cubic liquid crystal whether or not the thus formed cubic liquid crystal is of type I or type II. A small amount (several mg) of an amphipathic compound is placed on microscopic glass slide, and then pressure is gently applied with a cover glass, so that a thin film of the amphipathic compound, of which thickness is about 10 microns, is formed (at a diameter ranging from about 1 mm to 5 mm) in the space between the glass slide and the cover glass. Water or an aqueous solvent is added from the side of the space between the glass slide and the cover glass via capillary action. Water gradually penetrates from the outer edge of the amphipathic compound thin film into the interior, so that a water content gradient is formed from

the amphipathic compound thin film/water interface to the interior of the amphipathic compound thin film. Polarizing microscopic observation thereof enables the determination of a phase type formed depending on the concentration of the amphipathic compound/water system. Through observation that a region that imparts the same isotropic texture as that of a water region, adjacent to the water region (cubic liquid crystals), a region that imparts bright texture (lamellar liquid crystals), and a region that imparts isotropic texture (dry amphipathic compounds) are formed, it is confirmed that the amphipathic compound forms cubic liquid crystal. Also, it can be determined that the amphipathic compound is of type II on the basis of the stable formation of cubic liquid crystal in the interface between the excess water and the amphipathic compound.

(2) Confirmation of liquid crystal structure by small angle X-ray scattering (SAXS) assay

Whether or not a liquid crystal structure has a cubic lattice may be determined by a small-angle X-ray scattering (SAXS) method, for the purpose of confirming liquid crystal formation. First, an amphipathic compound/water system sample with a predetermined concentration can be filled into an X-ray capillary tube made of quartz, for example, and the capillary tube is sealed with an oxy-fuel burner, and subjected to SAXS assay.
Liquid crystal formation can be confirmed by confirming whether or not the following scattering peak ratio (peak interval) peculiar to each liquid crystal structure is exhibited as a result of SAXS measurement.
Ratio of Pn3m cubic liquid crystal:

$$\sqrt{2} : \sqrt{3} : \sqrt{4} : \sqrt{6} : \sqrt{8} : \sqrt{9} : \sqrt{10} : ,,,,$$

Ratio of Ia3d cubic liquid crystal:

$$\sqrt{3} : \sqrt{4} : \sqrt{7} : \sqrt{8} : \sqrt{10} : \sqrt{11} : ,,,,$$

Ratio of Im3m cubic liquid crystal:

$$\sqrt{2} : \sqrt{4} : \sqrt{6} : \sqrt{8} : \sqrt{10} : \sqrt{12} : \sqrt{14} : ,,,,$$

Ratio peculiar to reverse hexagonal liquid crystal:

$$1 : \sqrt{3} : 2$$

When a peak value is calculated from SAXS data and then the reciprocal ratio is calculated therefrom according to a method well known to persons skilled in the art, the space group and the lattice constant can be easily determined based thereon.
In addition, the amphipathic compound to be used for the adhesion preventing agent of the present invention has low viscosity in itself. Specifically, the amphipathic compound to be used for the adhesion preventing agent of the present invention has a viscosity of preferably 15.0 Pa·s or less, more preferably 11.0 Pa·s or less, and further preferably 6.0 Pa·s or less as measured at 25°C in itself. The viscosity can be measured using, for example, a viscosity and viscoelasticity measuring apparatus (Gemini II, Malvern Instruments Ltd.) at 25°C.

3. Synthesis of amphipathic compound

The above amphipathic compounds to be used in the present invention can be synthesized with reference to the Examples described below. Alternatively, the amphipathic compound having the general formula (III) can be synthesized in accordance with, for example, the synthesis method disclosed in International Publication WO 2011/078383. Further, the amphipathic compound having the general formula (IV) can be synthesized in accordance with, for example, the synthesis method disclosed in International Publication WO 2006/043705.
The amphipathic compound having the general formula (II) is more generally an ether or ester compound or a glycoside compound, wherein one molecule of long chain unsaturated hydrocarbon (preferably, long chain unsaturated fatty acid or long chain unsaturated alcohol) is bound to one molecule of polyhydric alcohol (preferably, glycerol, erythritol,

pentaerythritol, diglycerol, glyceric acid, triglycerol, xylose, sorbitol, ascorbic acid, glucose, galactose, mannose, dipentaerythritol, maltose, mannitol, or xylitol, and more preferably, glycerol, erythritol, pentaerythritol, diglycerol, glyceric acid, or xylose) via an ether or ester bond or a glycoside bond, respectively.

The amphipathic compounds having the general formula (II) according to the present invention can be produced (synthesized) as described below, for example.

First, among compounds having the above general formula (II), an ester compound wherein X and Y together denote an oxygen atom (the compound having the following general formula (II-1)) can be produced by transesterification reaction between an ester compound having the following general formula (V) and a hydrophilic compound R-OH, for example. Reaction conditions for transesterification are not particularly limited, and the transesterification is carried out using an acid or base catalyst, for example.

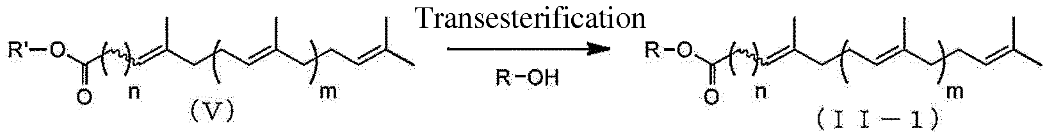


(V) (II-1)

Furthermore, an ester compound (having the general formula (II-1)) can be produced by esterification between a carboxylic acid corresponding to an ester compound having the general formula (V) and a hydrophilic compound R-OH. Reaction conditions for esterification are not particularly limited and, for example, esterification is carried out using an acid or base catalyst, a halogenating agent such as thionyl chloride, or a condensing agent.

Some or all hydroxyl groups within R of a hydrophilic compound R-OH may be protected during transesterification or esterification reaction. In this case, an ester compound (V) can be produced by transesterification or esterification reaction followed by deprotection.

Second, among compounds having the above general formula (II), an ether compound wherein X and Y are both hydrogen atoms (the compound having the following general formula (II-2)) can be produced by etherification reaction between a compound having the following general formula (VI) that has a leaving group Z and a hydrophilic compound R-OH, or by etherification reaction between an alcohol having the following general formula (VII) and a compound R-Z having a leaving group Z, for example. Reaction conditions for etherification are not particularly limited, and, for example, etherification is carried out using a base. Etherification reaction may also be carried out with protecting some or all hydroxyl groups within R of hydrophilic compound R-OH. In this case, the ether compound (II-2) can be produced by etherification reaction followed by deprotection.

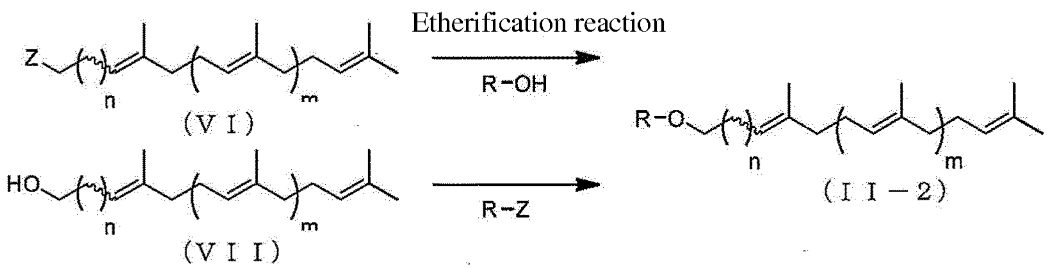


(VI) (VII) (II-2)

Third, among compounds having the above general formula (II), a glycoside compound having the general formula (II-2), wherein X and Y are both hydrogen atoms and R is a sugar residue, can be produced by glycosylation reaction of an alcohol having the general formula (VII) with saccharides R"-Z having a protected hydroxyl group and a leaving group Z at the anomeric position, followed by deprotection (R"→R) as shown below.

Reaction conditions for glycosylation are not particularly limited, and, for example, glycosylation is carried out using Lewis acids. Reaction conditions for deprotection are also not particularly limited, and, for example, deprotection is carried out by using elimination reaction conditions selected so that a glycosidic linkage is not impaired at a particular protecting group.

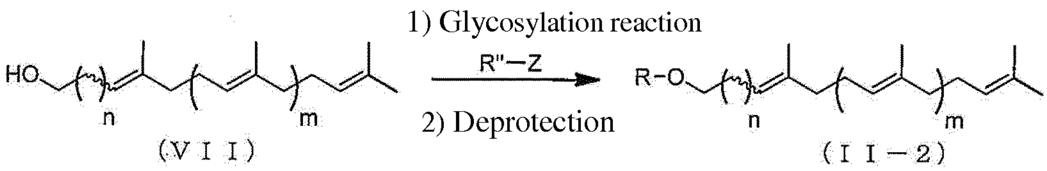


(VII) (II-2)

Further, among the above compounds having the general formula (II), an ester compound having the general formula (II-2), wherein X and Y are both hydrogen atoms and R is a carboxyl group, can be produced by transesterification between an alcohol having the general formula (VII) and a glycerate ester having a protected hydroxyl group, or by esterification between an alcohol having the general formula (VII) and glyceric acid having a protected hydroxyl group, followed by deprotection.

Compounds having the above general formulae (V), (VI), and (VII) can be synthesized by, but not limited to, the followong method.

An ester compound having the formula (V) wherein n=2 and m=2 can be obtained via Johnson-Claisen reaction using orthoacetate ester from 3,7,11,15-tetramethylhexadec-1,6,10,14-tetraen-3-ol (geranyllinalool), for example.

An ester compound having the above formula (V) wherein n=2 and m=1 can be obtained by Johnson-Claisen reaction using orthoacetate ester from 3,7,11-trimethyldodec-1,6,10-trien-3-ol (nerolidol), for example, by the methods as disclosed in JP Patent Publication No. S51-29431 A, JP Patent Publication No. S51-29434 A, JP Patent Publication No. S51-29435 A, JP Patent Publication No. S51-29436 A, and JP Patent Publication No. S51-34108 A, for example.

An ester compound having the formula (V) wherein n=1 and m=2 can be obtained by brominating the hydroxyl group of 3,7,11,15-tetramethylhexadec-2,6,10,14-tetraen-1-ol (geranylgeraniol) and then reacting a Grignard reagent generated by adding metal magnesium with carbon dioxide or carrying out substitution reaction with cyanide followed by hydrolysis to produce carboxylic acids, and then further carrying out esterification, for example.

An ester compound having the formula (V) wherein n=1 and m=1 can be obtained by brominating the hydroxyl group of 3,7,11-trimethyldodec-2,6,10-trien-1-ol (farnesol) and then reacting a Grignard reagent generated by adding metal magnesium with carbon dioxide or carrying out substitution reaction with cyanide followed by hydrolysis to produce carboxylic acids, and then further carrying out esterification, for example.

An ester compound having the formula (V) wherein n=0 and m=2 can be obtained by esterifying 3,7,11,15-tetramethylhexadec-2,6,10,14-tetraenoic acid (geranylgeranoic acid), for example.

An ester compound having the formula (V) wherein n=0 and m=1 can be obtained by esterifying 3,7,11-trimethyldodec-2,6,10-trienoic acid (farnesyl acid), for example.

An alcohol having the formula (VII) wherein n=2 and m=2 can be obtained by reducing the ester compound having the formula (V) wherein n=2 and m=2 or its corresponding carboxylic acid using e.g., lithium aluminum hydride. The alcohol having the formula (VII) wherein n=2 and m=1; n=1 and m=2; or n=1 and m=1 can be similarly obtained by reducing the ester compound having the formula (V) wherein n=2 and m=1; n=1 and m=2; or n=1 and m=1; or its corresponding carboxylic acid using e.g., lithium aluminum hydride.

The alcohol having the formula (VII) wherein n=0 and m=2 is 3,7,11,15-tetramethylhexadec-2,6,10,14-tetraen-1-ol (geranylgeraniol) and is commercially available. However, the alcohol can also be obtained by reducing the ester compound having the formula (V) wherein n=0 and m=2, or its corresponding carboxylic acid, using e.g., lithium aluminum hydride, for example.

The alcohol having the formula (VII) wherein n=0 and m=1 is 3,7,11-trimethyldodec-2,6,10-trien-1-ol (farnesol) and is commercially available. However, the alcohol can also be obtained by reducing the ester compound having the formula (V) wherein n=0 and m=1, or its corresponding carboxylic acid, using e.g., lithium aluminum hydride, for example.

The compound having the formula (VI) with the leaving group Z wherein n=2 and m=2 can be obtained by converting the alcohol having the formula (VII) wherein n=2 and m=2 to a sulfonyloxy group (e.g., tosyl group or mesyl group) or a leaving group such as halogen atom (e.g., chlorine atom, bromine atom, or iodine atom). The compound having the formula (VI) with the leaving group Z wherein n=2, m=1; n=1, m=2; n=1, m=1; n=0, m=2; or n=0, m=1 can be similarly obtained by converting the alcohol having the formula (VII) wherein n=2, m=1; n=1, m=2; n=1, m=1; n=0, m=2; or n=0, m=1, respectively, to a leaving group.

It is preferably verified that the thus synthesized compounds are compounds of interest by using conventional methods such as NMR measurement.

4. Preparation of adhesion preventing agent

The adhesion preventing agent according to the present invention contains an effective amount of the amphipathic compound described above. The concentration of the amphipathic compound contained in the adhesion preventing agent according to the present invention is, but not limited to, for example, 1% to 80% and preferably 10% to 50% of the total amount of the adhesion preventing agent.

The adhesion preventing agent according to the present invention may be in an any dosage form (typically, parenteral formulation). It is preferably formulated into a dosage form that can be directly applied onto tissue and is excellent in handleability, such as spray agent (e.g., an aerosol agent or a pump spray agent), topical formulation, or injection. In the context of the present invention, the term "spray agent" refers to a medicament in a dosage form that enables a substance of interest to be ejected in the form of droplets, mist, fine particles, foam or the like by pressure applied manually, via mechanical power or with a propellant (gas). In the context of the present invention, the term

"aerosol agent" refers to a medicament in a dosage form that enables a substance of interest to be ejected by pressure applied with a propellant filled into a container, together with the substance of interest. In the context of the present invention, the term "pump spray agent" refers to a medicament in a dosage form that enables a substance of interest to be ejected using a atomizer, a powered sprayer, or the like, without using a propellant. In the context of the present invention, the term "medicament" may also refer to a medical material. The present invention also relates to a medicament comprising the adhesion preventing agent according to the present invention used as, for example, a medical material for adhesion prevention.

The adhesion preventing agent according to the present invention is preferably a composition further comprising a pharmaceutically acceptable carrier. Those skilled in the art can adequately select a pharmaceutically acceptable carrier depending on the dosage form to be used, and it may be a gas carrier, a liquid carrier, or the like. Examples of the gas carrier include, but are not limited to, inert gases such as liquefied gas (e.g., butane gas, dimethyl ether, LP gas, carbon dioxide, nitrogen gas, or a mixture thereof), compressed gas, and decomposed gas. Such gas carrier is preferably used for aerosol agents. Examples of the liquid carrier include, but are not limited to, oil such as silicone oil (preferably, e.g., dimethicone), ester such as isopropyl myristate, alcohol (e.g., ethanol or isopropanol), a physiologically acceptable organic solvent (e.g., dimethyl sulfoxide (DMSO)), and an aqueous medium. Such liquid carrier is preferably used for spray agents, topical formulations, injections, and the like.

The adhesion preventing agent according to the present invention contains an aqueous medium, thereby facilitating the formation of non-lamellar liquid crystal of the amphipathic compound, and effectively exhibiting the adhesion preventing effect. Depending on dosage forms to be used, it may be preferred to use an aqueous medium in an amount at which liquid crystal gel is not formed before application to a living body, depending on the formulation to be used, or an aqueous medium may be used in an amount at which liquid crystal gel is formed. Such aqueous medium may be water such as sterile water, purified water, distilled water, ion exchanged water, or ultrapure water; or physiologically acceptable aqueous solutions. Examples of the physiologically acceptable aqueous solution include, for example, physiological saline; aqueous electrolyte solutions such as aqueous sodium chloride solution, aqueous calcium chloride solution, aqueous magnesium chloride solution, aqueous sodium sulfate solution, aqueous potassium sulfate solution, aqueous sodium carbonate solution, and aqueous sodium acetate solution; buffers such as phosphate buffer and Tris-HCl buffer; aqueous solutions containing sugar molecules such as glucose, sucrose, maltose, and hyaluronic acid; and aqueous solutions containing water soluble polymers such as polyethylene glycol and polyvinyl alcohol. Preferred examples of the physiologically acceptable aqueous solutions include hyaluronic acid aqueous solutions comprising hyaluronic acid or a salt thereof (e.g., sodium hyaluronate). The hyaluronic acid aqueous solution may be, but not limited to, 0.01%-5% and preferably 0.1%-1% hyaluronic acid aqueous solution. Thus, it is also preferred for the adhesion preventing agent according to the present invention to contain hyaluronic acid or a salt thereof.

When the adhesion preventing agent according to the present invention comprises an aqueous medium, it may be an o/w dispersion (oil-in-water type) or w/o dispersion (water-in-oil type).

The adhesion preventing agent according to the present invention may further comprise a pharmaceutically acceptable surfactant. Any pharmaceutically acceptable surfactant used in the art of pharmaceutical products or cosmetics can be used. Examples of pharmaceutically acceptable surfactants that can be used include, but are not limited to, Pluronic (Pluronic F127; polyoxyethylene polyoxypropylene (200EO) (70PO)), polysorbate 80 (polyoxyethylene sorbitan oleate; Tween 80), and propylene carbonate.

In a preferred embodiment, the adhesion preventing agent is a mixture of at least one amphipathic compound and at least one liquid carrier selected from the group consisting of oil such as silicone oil (preferably, e.g., dimethicone), ester such as isopropyl myristate, alcohol (e.g., ethanol or isopropanol), and a physiologically acceptable organic solvent (e.g., dimethyl sulfoxide (DMSO)). In another preferred embodiment, the adhesion preventing agent is a mixture of at least one amphipathic compound, an aqueous medium (e.g., water) and at least one liquid carrier selected from the group consisting of oil such as silicone oil (preferably, e.g., dimethicone), ester such as isopropyl myristate, alcohol (e.g., ethanol or isopropanol), and a physiologically acceptable organic solvent (e.g., dimethyl sulfoxide (DMSO)). These mixtures may comprise hyaluronic acid or a salt thereof.

In one preferred embodiment, the adhesion preventing agent is, more specifically, a mixture of an amphipathic compound and silicone oil (preferably, dimethicone) or a mixture of an amphipathic compound, silicone oil (preferably, dimethicone) and water, for example.

In another preferred embodiment, the adhesion preventing agent is a mixture of an amphipathic compound, alcohol (preferably, ethanol), an aqueous medium (preferably, water or a hyaluronic acid aqueous solution) and a surfactant (preferably, Pluronic).

In yet another preferred embodiment, the adhesion preventing agent is a mixture of an amphipathic compound, an aqueous medium (preferably, water or a hyaluronic acid aqueous solution), and a surfactant (preferably, Pluronic).

When the adhesion preventing agent according to the present invention does not comprise an aqueous medium or it comprise an aqueous medium only in an amount at which liquid crystal gel is not formed before application to a living body, it is preferred to apply the adhesion preventing agent to an area of tissue and subsequently further apply

an aqueous medium to the application area. The further application of the aqueous medium allows the amphipathic compound contained in the adhesion preventing agent to favorably form a liquid crystal gel at the application area.

The adhesion preventing agent according to the present invention may further comprise additive(s) such as stabilizer, buffering agent, preservative, flavoring agent, and/or coloring agent that are pharmaceutically commonly used.

The adhesion preventing agent comprising the amphipathic compound according to the present invention can be produced in any dosage form such as aerosol agent, spray agent, topical formulation, or injection by a general formulation technique.

5. Evaluation of adhesion preventing effect

The adhesion preventing agent according to the present invention can exhibit an effect of preventing tissue adhesion by applying it to a tissue that is at risk of adhesion. In the context of to the present invention, the term "adhesion preventing effect" refers to an effect of preventing tissue from adhering to another tissue or organ to result in a state in which it is difficult to peel adhesions, and preventing adhesion completely or reducing the degree of adhesion to low levels.

The adhesion preventing effect of the adhesion preventing agent according to the present invention is obtained by the formation of a coating as a result of formation of non-lamellar liquid crystal of the amphipathic compound contained in the adhesion preventing agent on the tissue surface to which the amphipathic compound has been applied. The formed coating prevents the tissue from being in contact with other tissues or organs, thereby reducing adhesion.

The adhesion preventing effect of the adhesion preventing agent according to the present invention can be confirmed by, for example, applying the adhesion preventing agent to a tissue incision of an animal model subjected to laparotomy, closing the abdominal incision, and carrying out follow-up observation. Specifically, an abdominal median incision (e.g. approximately 30 mm) is made to a rat and an incision of approximately 20 mm in length is further made on the upper parietal peritoneum. After complete hemostasis, the peritoneum incision is closed with a continuous suture (using, e.g., Silk Suture 5-0). The adhesion preventing agent is applied to cover the peritoneal suture site. After the formation of a coating, the abdominal wall is closed with a suture. Then, laparotomy is performed on the rat again after a certain period of time (e.g., 7 days) after surgery to evaluate the adhesion observed at the peritoneal suture site.

The adhesion preventing agent may be applied in a manner appropriate for a dosage form used. The typical amount of the adhesion preventing agent applied for this evaluation is preferably an amount corresponding to 10 mg of the lipids.

Adhesion can be evaluated by, for example, scoring adhesion in accordance with the following evaluation scores.

- Grade 0: No adhesion
- Grade 1: Peelable adhesion with mild traction
- Grade 2: Peelable adhesion with strong traction (impossible to peel with mild traction)
- Grade 3: Adhesion involving tissue damage by peeling (strong adhesion of adipose tissue), but without adhesion to other organs
- Grade 4: Adhesion to other organs, with difficulty in peeling (including impossibility of peeling)

If the adhesion evaluation score is lower than that of an untreated group, it is determined that an adhesion preventing effect can be obtained. That is, in the context of the present invention, the "adhesion prevention" refers to a decrease in frequency and/or degree of adhesion compared with those in an untreated group.

6. Adhesion prevention method

According to the present invention, a method for preventing tissue adhesion at an affected area, comprising applying an effective amount of the adhesion preventing agent according to the present invention to an affected area of a patient, specifically an area at risk of adhesion, i.e., an area at which tissue repair is expected to occur (e.g., an *in vivo* inflammatory area or injured area), is also provided. Specific examples of such area at risk of adhesion include exogenous or endogenous inflammatory sites, wound sites such as surgical incisions, and areas at which the tissue surface has been damaged due to artificial treatment involving, e.g., contact during surgery. In the context of the present invention, the term "injured area" refers to an area of a tissue or organ damaged as a result of surgery, trauma, diseases, or the like. Examples of a tissue or organ to which the adhesion preventing agent is applied include, but are not limited to, peritoneum, small intestine, large intestine, rectum, stomach, duodenum, cecum, liver, uterus, fallopian tube, lymphatic vessels, heart, pericardium, lung, brain, ovary, and tendons. In typical cases, the adhesion preventing agent according to the present invention is applied to an incision, an area surrounding an incision, or an organ having an incision as a whole upon surgery. The adhesion preventing agent according to the present invention may be applied to an area *in vivo* in contact with a wound site or an inflammatory area.

The adhesion preventing agent can be applied to an affected area such as an injured area (e.g., a wound site) or an inflammatory area in a manner suitable for the dosage form of the adhesion preventing agent. For example, if the adhesion preventing agent is an aerosol agent, the adhesion preventing agent can be sprayed onto an affected area such as an injured area (e.g., a wound site) or an inflammatory area using a gas-injection-type aerosol container. Alternatively, if the adhesion preventing agent is a pump spray agent, the adhesion preventing agent can be sprayed onto an affected area such as an injured area (e.g., a wound site) or an inflammatory area using a general-use non-gas-injection-type (e.g., manual) spray container, for example. In the case of endoscopic surgery or laparoscopic surgery, the adhesion preventing agent can be sprayed onto an affected area such as an injured area (e.g., a wound site) using, for example, a spray nozzle used for endoscopic surgery or laparoscopic surgery. In the context of the present invention, the term "spray" or "spraying" refers to causing an ejection (spray and/or squirt) of a substance of interest in the form of droplets, mist, fine particles, foam or the like by pressure). If the adhesion preventing agent is a topical formulation, an adequate amount of the adhesion preventing agent can be applied by spreading it to an affected area such as an injured area (e.g., a wound site) or an inflammatory area. If the adhesion preventing agent is an injection, it can be injected into an affected area such as an injured area (e.g., a wound site) or an inflammatory area.

It is preferred to apply the adhesion preventing agent according to the present invention to an affected area such as an injured area (e.g., a wound site) or an inflammatory area in a sufficient amount to cover the affected area. In a preferred embodiment, a specific dosage amount of the adhesion preventing agent according to the present invention is 50 mg to 50 g (and more preferably 600 mg to 1500 mg) for humans.

When the adhesion preventing agent according to the present invention contains a sufficient amount of an aqueous medium, the amphipathic compound contained in the adhesion preventing agent can form a non-lamellar liquid crystal on the tissue surface to which the adhesion preventing agent has been applied. Even if the adhesion preventing agent according to the present invention does not contain a sufficient amount of an aqueous medium, a non-lamellar liquid crystal is formed with water in the body. However, in order to promote coating formation, it is preferred to apply an aqueous medium in addition to the adhesion preventing agent to an affected area such as an injured area (e.g., a wound site) or an inflammatory area. The aqueous medium described above as a component of the adhesion preventing agent can be used as the aqueous medium herein. Examples of such aqueous medium include water such as sterile water, purified water, distilled water, ion exchanged water, or ultrapure water; and physiologically acceptable aqueous solutions. Examples of a physiologically acceptable aqueous solution include physiological saline; aqueous electrolyte solutions such as aqueous sodium chloride solution, aqueous calcium chloride solution, aqueous magnesium chloride solution, aqueous sodium sulfate solution, aqueous potassium sulfate solution, aqueous sodium carbonate solution, and aqueous sodium acetate solution; buffers such as phosphate buffer and Tris-HCl buffer; aqueous solutions containing sugar molecules such as glucose, sucrose, maltose, and hyaluronic acid; and aqueous solutions containing water soluble polymers such as polyethylene glycol and polyvinyl alcohol. Preferred examples of the physiologically acceptable aqueous solutions include hyaluronic acid aqueous solutions containing hyaluronic acid or a salt thereof (e.g., sodium hyaluronate). Examples of hyaluronic acid aqueous solutions include, but are not limited to, 0.01%-5% and preferably 0.1%-1% hyaluronic acid aqueous solutions.

It is preferred to apply the adhesion preventing agent and then apply an aqueous medium on it, but there is no limitation thereto. It is possible to apply an aqueous medium in a manner similar to that for the adhesion preventing agent, for example, by spraying, spreading, or injecting. After applying an aqueous medium to a tissue or organ in the above manner, it is preferred to allowing it to stand for a certain period of time (which may be, but is not limited to, for example, 1 to 30 minutes and preferably 5 to 10 minutes) to promote coating formation.

Typical examples of a subject (patient) to which the method for preventing adhesion using the adhesion preventing agent according to the present invention is applied include mammals such as humans, livestock, pet animals, and laboratory animals. A subject who has received or is expected to receive an injury to a tissue (organ) due to surgery, trauma, diseases, or the like is particularly preferred. Examples of surgery include endoscopic surgery and laparoscopic surgery as well as laparotomy.

7. Polyunsaturated fatty acid ester and use thereof

The present invention also relates to the above amphipathic compound having the general formula (II) (polyunsaturated fatty acid ester) itself, which is among the amphipathic compounds of the present invention.

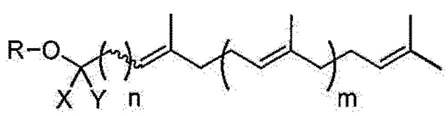

(I I)

In the general formula (II), X and Y each denotes a hydrogen atom or together denote an oxygen atom, n

denotes an integer from 0 to 2, and m denotes 1 or 2. In a preferred embodiment, in the general formula (II), n denotes 1 or 2, and m denotes 2. R denotes a hydrophilic group generated by removal of one hydroxyl group from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, and xylitol. Preferably, for example, R denotes a hydrophilic group generated by removal of one hydroxyl group from any one selected from the group consisting of glycerol, erythritol, pentaerythritol, diglycerol, glyceric acid, and xylose. When X and Y each denotes a hydrogen atom, R preferably denotes a hydrophilic group generated by removal of one hydroxyl group from any one selected from the group consisting of glycerol, erythritol, pentaerythritol, diglycerol, glyceric acid, and xylose. When X and Y together denote an oxygen atom, R preferably denotes a hydrophilic group generated by removal of one hydroxyl group from any one selected from the group consisting of glycerol, erythritol, pentaerythritol, and diglycerol. In the case of n=0, R preferably denotes a hydrophilic group generated by removal of one hydroxyl group from any one selected from the group consisting of erythritol, pentaerythritol, diglycerol, glyceric acid, and xylose. In light of the adhesion preventing effect, R particularly preferably denotes a hydrophilic group generated by removal of one hydroxyl group from any one selected from the group consisting of glycerol, erythritol, pentaerythritol, and diglycerol. Examples of the compound are as described above.

The present invention further relates to a salt of the above amphipathic compound having the general formula (II). The salt of the amphipathic compound having the general formula (II) of the present invention may be any type of salt, including alkali metal or alkaline-earth metal salt such as sodium, potassium, calcium, or magnesium salt and preferably sodium or potassium salt. The salt of the amphipathic compound having the general formula (II) of the present invention may be a salt acceptable for production of foods, cosmetics, pharmaceuticals, or pesticides, to be selected depending on the intended use. For example, it may be a pharmacologically acceptable salt.

Not only does the amphipathic compound itself have low viscosity, but a gel (liquid crystal gel) formed by adding water to the amphipathic compound also has significantly low viscosity. The viscosity of the liquid crystal gel is, but is not limited to, preferably 100 Pa·s (Pas·sec) or less and more preferably 50 Pa·s or less as measured at a shear velocity of approximately 100 1/s using a viscosity and viscoelasticity measuring apparatus (Gemini II, Malvern Instruments Ltd.). Therefore, the amphipathic compound can be easily formulated into a variety of preparations such as an injection and thus it can be advantageously used as a base for preparations. Moreover, since the amphipathic compound has low viscosity, it can be used in cosmetics for the purpose of improving feeling upon use.

Preferred examples of the amphipathic compound having the general formula (II) include, but are not limited to, the following:

mono-O-(5,9,13,17-tetramethyloctadec-4,8,12,16-tetraenoyl)glycerol,
mono-O-(5,9,13,17-tetramethyloctadec-4,8,12,16-tetraenoyl)erythutol,
mono-O-(5,9,13,17-tetramethyloctadec-4,8,12,16-tetraenoyl)pentaerythritol,
mono-O-(5,9,13-trimethyltetradec-4,8,12-trienyl)erythritol,
mono-O-(5,9,13-trimethyltetradec-4,8,12-trienyl)pentaerythritol,
mono-O-(3,7,11,15-tetramethylhexadec-2,6,10,14-tetraenoyl)pentaerythritol,
mono-O-(3,7,11,15-tetramethylhexadec-2,6,10,14-tetraenyl)erythritol,
mono-O-(5,9,13,17-tetramethyloctadec-4,8,12,16-tetraenyl)erythritol, and
mono-O-(5,9,13,17-tetramethyloctadec-4,8,12,16-tetraenyl)pentaerythritol.

## Examples

The present invention will be explained more specifically with reference to the following Examples. However, the technical scope of the present invention is not limited to these Examples.

The viscosity of each of the compounds discribed in the Examles 1-7 was measured using a viscosity/viscoelasticity measuring instrument (Gemini II, Malvern) at the temperature of 25°C.

[Example 1]

Synthesis of mono-O-(5,9,13-trimethyltetradeca-4,8,12-trienoyl)glycerol

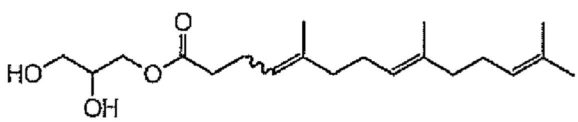

Under reduced pressure of 200-250 mmHg, 13.9 g (50.0 mmol) of methyl 5,9,13-trimethyltetradeca-4,8,12-trienoate (methyl farnesylacetate) was slowly added dropwise at 85°C to a solution of 9.2 g (0.10 mol) of glycerol and 0.28 g (2.0 mmol) of potassium carbonate in dry N,N-dimethylformamide (20 mL), followed by stirring at the same temperature for 3 hours. In this reaction, the methanol produced was distilled off. The resulting reaction solution was diluted with a mixed solvent of ethyl acetate/hexane (1:1, 150 mL), washed with water, saturated sodium bicarbonate aqueous solution, and saturated brine (twice), and dried over magnesium sulfate. After filtration, the filtrate was concentrated, and the resulting residue was purified by silica gel column chromatography (hexane/ethyl acetate = 100:0 to 0:100) to obtain 8.22 g of the title compound (49% yield) as a colorless transparent liquid. $^1$H-NMR and viscosity of the obtained compound were measured. The results were as follows.

$^1$H-NMR spectrum (270 MHz, $CDCl_3$, TMS) δ: 1.5-1.8 (m, 12H), 1.9-2.1 (m, 8H), 2.1 (brs, 1H, OH), 2.25-2.45 (m, 4H), 2.56 (brs, 1H, OH), 3.59 (dd, J=5.6, 11.2Hz, 1H), 3.68 (dd, J=3.6, 11.2Hz, 1H), 3.92 (m, 1H), 4.14 (dd, J=6.0, 11.6Hz, 1H), 4.21 (dd, J=4.8, 11.6Hz, 1H), 5.02-5.16 (m, 3H).

Viscosity: 0.26 Pa·s (at shear velocity of 92 1/s).

Mono-O-(5,9,13-trimethyltetradeca-4,8,12-trienoyl)glycerol synthesized was also referred to below as glyceryl farnesylacetate.

[Example 2]

Synthesis of mono-O-(,5,9,13-trimethyltetradeca-4,8,12-trienoyl)erythritol

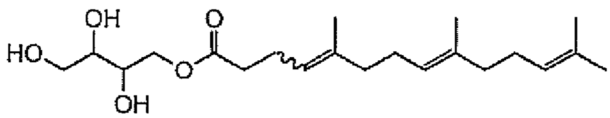

The title compound was synthesized and mesured using the same procedure as employed in Example 1, but with 12.2 g (0.100 mol) of erythritol instead of 9.2 g (0.10 mol) of glycerol. The compound was obtained (6.68 g, 36% yield) as a colorless transparent liquid , which has the following $^1$H-NMR spectrum and viscosity:

$^1$H-NMR spectrum (270 MHz, $CDCl_3$, TMS) δ: 1.58-1.74 (m, 12H), 1.92-2.14 (m, 8H), 2.3-2.5 (m, 5H), 2.80 (m, 1H, OH), 2.98 (m, 1H, OH), 3.63 (m, 1H), 3.81 (m, 2H), 3.88 (m, 1H), 4.29 (dd, J=3.4, 11.9Hz, 1H), 4.34 (dd, J=5.6, 11.9Hz, 1H), 5.05-5.15 (m, 3H).

Viscosity: 4.7 Pa·s (at shear velocity of 92 1/s).

Mono-O-(5,9,13-trimethyltetradeca-4,8,12-trienoyl)erythritol synthesized had a low viscosity.

[Example 3]

Synthesis of mono-O-(5,9,13-trimethyltetradeca-4,8,12-trienoyl)pentaerythritol

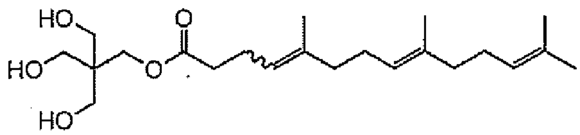

The title compound was synthesized and mesured using the same procedure as employed in Example 1, but with 13.6 g (0.100 mol) of pentaerythritol instead of 9.2 g (0.10 mol) of glycerol. The compound was obtained (6.97 g, 36% yield) as a colorless transparent liquid, which has the following $^1$H-NMR spectrum and viscosity:

$^1$H-NMR spectrum (270 MHz, $CDCl_3$, TMS) δ: 1.58-1.72 (m, 12H), 1.9-2.15 (m, 8H), 2.28-2.45 (m, 4H), 2.66 (brs, 3H, OH), 3.63 (s, 6H), 4.22 (s, 2H), 5.05-5.15 (m, 3H).

Viscosity: 2.5 Pa·s (at shear velocity of 92 1/s).

Mono-O-(5,9,13-trimethyltetradeca-4,8,12-trienoyl)pentaerythritol synthesized had a low viscosity.

[Example 4]

Synthesis of mono-O-(5,9,13,17-tetramethyloctadeca-4,8,12,16-tetraenoyl)glycerol

(1) Synthesis of methyl 5,9,13,17-tetramethyloctadeca-4,8,12,16-tetraenoate (methyl geranylgeranylacetate)

Under a nitrogen atmosphere, a mixture of 53 mL (0.42 mol) of trimethyl orthoacetate and 5.0 mL (40 mmol) of n-hexanoic acid was slowly added dropwise at 135°C for 8 hours to a solution of 58.1 g (200 mmol) of 3,7,11,15-tetramethylhexadeca-1,6,10,14-tetraen-3-ol (geranyl linalool) and 19 mL (0.15 mol) of trimethyl orthoacetate. After the reaction mixture was stirred for 6 hours at the same temperature, a mixture of 5.3 mL (42 mmol) of trimethyl orthoacetate and 0.5 mL (4 mmol) of n-hexanoic acid was added dropwise, and the mixture was further stirred for 2 hours at the same temperature. The resulting reaction solution was dilluted with a mixed solvent of ethyl acetate/hexane (3:1, 300 mL), washed with saturated sodium bicarbonate aqueous solution (twice), and saturated brine, and dried over magnesium sulfate. After filtration, the filtrate was concentrated to obtain 67.24 g of the title compound as a crude liquid product. The crude product was directly used for the next reaction.

(2) Synthesis of mono-O-(5,9,13,17-tetramethyloctadeca-4,8,12,16-tetraenoyl)glycerol

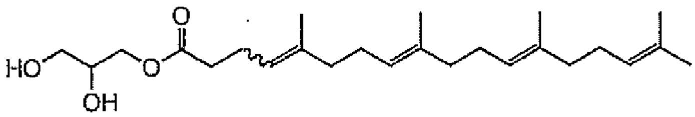

Under reduced pressure of 200-250 mmHg, 13.9 g (40.0 mmol) of methyl 5,9,13,17-tetramethyloctadeca-4,8,12,16-tetraenoate (methyl geranylgeranylacetate) synthesized in Example 4 (1) was slowly added dropwise at 85°C to a solution of 7.4 g (80 mmol) of glycerol and 5.5 g (40 mmol) of potassium carbonate in dry N,N-dimethylformamide (16 mL), followed by stirring at the same temperature for 6 hours. In this reaction, the methanol produced was distilled off. The resulting reaction solution was diluted with a mixed solvent of ethyl acetate/hexane (1:1, 200 mL), washed with water, saturated sodium bicarbonate aqueous solution, and saturated brine (twice), and dried over magnesium sulfate. After filtration, the filtrate was concentrated, and the resulting residue was purified by silica gel column chromatography (hexane/ethyl acetate = 100:0 to 0:100) to obtain 5.44 g of the title compound (33% yield) as a transparent liquid. $^1$H-NMR and viscosity of the obtained compound were measured. The results were as follows.

$^1$H-NMR spectrum (270 MHz, $CDCl_3$, TMS) $\delta$: 1.55-1.72 (m, 15H), 1.9-2.2 (m, 13H), 2.27-2.45 (m, 4H), 2.53 (brs, 1H, OH), 3.59 (dd, J=5.4, 11.4Hz, 1H), 3.68 (dd, J=3, 11.4Hz, 1H), 3.92 (m, 1H), 4.15 (dd, J=6.0, 11.6Hz, 1H), 4.21 (dd, J=4.8, 11.6Hz, 1H), 5.05-5.15 (m, 4H).

Viscosity: 0.37 Pa·s (at shear velocity of 92 1/s).

Mono-O-(5,9,13,17-tetramethyloctadeca-4,8,12,16-tetraenoyl)glycerol synthesized had a very low viscosity.

[Example 5]

Synthesis of mono-O-(5,9,13,17-tetramethyloctadeca-4,8,12,16-tetraenoyl)erythritol

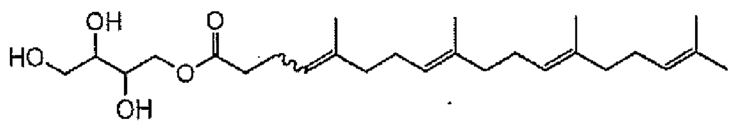

The title compound was synthesized and mesured using the same procedure as employed in Example 4 (2), but with 9.8 g (80 mmol) of erythritol instead of 7.4 g (80 mmol) of glycerol. The compound was obtained (4.01 g, 23% yield) as a transparent liquid, which has the following $^1$H-NMR spectrum and viscosity:

$^1$H-NMR spectrum (270 MHz, $CDCl_3$, TMS) $\delta$: 1.55-1.75 (m, 15H), 1.9-2.15 (m, 12H), 2.27-2.46 (m, 5H), 2.81 (m, 1H, OH), 2.99 (m, 1H, OH), 3.63 (m, 1H), 3.81 (m, 2H), 3.87 (m, 1H), 4.29 (dd, J=3.4, 11.9Hz, 1H), 4.34 (dd, J=5.6, 11.9Hz, 1H), 5.05-5.15 (m, 4H).

Viscosity: 5.8 Pa·s (at shear velocity of 92 1/s).

Mono-O-(5,9,13,17-tetramethyloctadeca-4,8,12,16-tetraenoyl)erythritol synthesized had a low viscosity.

[Example 6]

Synthesis of mono-O-(5,9,13,17-tetramethyloctadeca-4,8,12,16-tetraenoyl)pentaerythritol

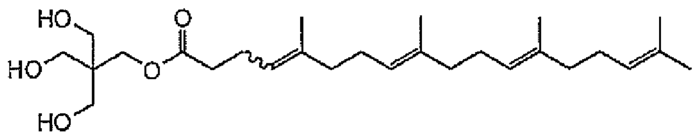

The title compound was synthesized and mesured using the same procedure as employed in Example 4 (2), but with 10.9 g (80 mmol) of pentaerythritol instead of 7.4 g (80 mmol) of glycerol. The compound was obtained (2.53 g, 14% yield) as a transparent liquid, which has the following $^1$H-NMR spectrum and viscosity:

$^1$H-NMR spectrum (270 MHz, $CDCl_3$, TMS) δ: 1.57-1.71 (m, 15H), 1.9-2.13 (m, 12H), 2.27-2.45 (m, 4H), 2.64 (brs, 3H, OH), 3.62 (s, 6H), 4.20 (s, 2H), 5.04-5.16 (m, 4H).

Viscosity: 3.3 Pa·s (at shear velocity of 92 1/s).

Mono-O-(5,9,13,17-tetramethyloctadeca-4,8,12,16-tetraenoyl)pentaerythritol synthesized had a low viscosity.

[Example 7]

Synthesis of mono-O-(5,9,13,17-tetramethyloctadeca-4,8,12,16-tetraenoyl)diglycerol

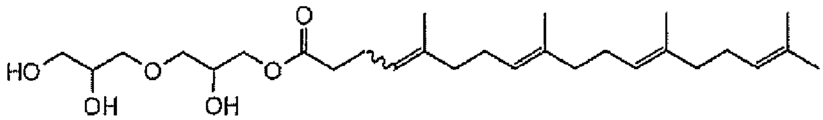

The title compound was synthesized and mesured using the same procedure as employed in Example 4 (2), but with 13.3 g (80 mmol) of diglycerol instead of 7.4 g (80 mmol) of glycerol. The compound was obtained (3.34 g, 17% yield) as a transparent liquid, which has the following $^1$H-NMR spectrum and viscosity:

$^1$H-NMR spectrum (270 MHz, $CDCl_3$, TMS) δ: 1.55-1.7 (m, 15H), 1.88-2.21 (m, 12H), 2.22-2.4 (m, 4H), 3.4-3.8 (m, 8H), 3.8-4.1 (m, 2H), 4.11 (m, 2H), 4.3-4.5 (m, 1H), 5.02-5.14 (m, 4H).

Viscosity: 2.6 Pa·s (at shear velocity of 92 1/s).

Mono-O-(5,9,13,17-tetramethyloctadeca-4,8,12,16-tetraenoyl)diglycerol synthesized had a low viscosity.

[Example 8]

Synthesis of mono-O-(5,9,13-trimethyltetradecanoyl)glycerol

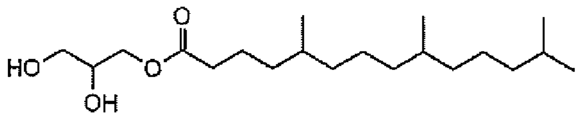

70 g (0.53 mol) of 2,2-dimethyl-1,3-dioxolane-4-methanol and 36.7 g (266 mmol) of potassium carbonate was added to 50.3 g (177 mmol) of methyl 5,9,13-trimethyltetradecanoate, followed by stirring at 85°C for 3 hours under reduced pressure of 200-250 mmHg. In this reaction, the methanol produced was distilled off. After the resulting reaction solution was subjected to vacuum concentration (from 50 to 210°C, from 1.4 to 0.38 kPa), the resulting residue was purified by silica gel column chromatography (hexane/ethyl acetate) to obtain 43.0 g of (2,2-dimethyl-1,3-dioxolane-4-yl)methyl 5,9,13-trimethyltetradecanoate (63% yield).

3M hydrochloric acid (85 mL) was added to a solution of 32.7 g (85.0 mol) of (2,2-dimethyl-1,3-dioxolane-4-yl)methyl 5,9,13-trimethyltetradecanoate in tetrahydrofuran (340 mL) at room temperature, followed by stirring at the same temperature for 5 hours. The reaction mixture was added to ethyl acetate (300mL) and saturated sodium bicarbonate aqueous solution (400 mL), followed by separation. The separated organic layer was washed with saturated brine, and dried over magnesium sulfate. After filtration, the filtrate was concentrated, and the resulting residue was purified by silica gel column chromatography (hexane/ethyl acetate) to obtain 28.7 g of the title compound (98% yield) as a colorless transparent liquid. $^1$H-NMR of the obtained compound was measured. The result was as follows.

$^1$H-NMR spectrum (270 MHz, $CDCl_3$, TMS) $\delta$: 0.7-0.9 (m, 12H), 0.95-1.45 (m, 16H), 1.45-1.75 (m, 3H), 3.60 (dd, J=5.8, 11.5Hz, 1H), 3.70 (dd, J=4.0, 11.5Hz, 1H), 3.94 (m, 1H), 4.15 (dd, J=5.9, 11.7Hz, 1H), 4.21 (dd, J=4.7, 11.7Hz, 1H).

Mono-O-(5,9,13-trimethyltetradecanoyl)glycerol synthesized was also referred to below as saturated C17 glycerin ester.

[Example 9]

Formation of a liquid crystal by mono-O-(5,9,13-trimethyltetradeca-4,8,12-trienoyl)glycerol and analysis thereof

Mono-O-(5,9,13-trimethyltetradeca-4,8,12-trienoyl)glycerol synthesized in Example 1 and pure water were introduced into a mixing device at the concentration of 50 % by mass of mono-O-(5,9,13-trimethyltetradeca-4,8,12-trienoyl)glycerol (in water-excess condition), and they were mixed at room temperature (25°C) and left for 24 hours to obtain a homogeneous mixture. Then the separated water was removed. Thus, a sample of mono-O-(5,9,13-trimethyltetradeca-4,8,12-trienoyl)glycerol/water system was obtained as a white turbid to colorless transparent gel composition in appearance (it was referred to below as the gel sample).

Subsequently, the gel sample was analyzed by small-angle x-ray scattering (SAXS). The NANO-Viewer nanoscale X-ray structure analysis equipment (Rigaku) was used for SAXS analysis, which was performed by X-ray irradiation at room temperature (25°C), 40 kV, 50 mA, wavelength $\lambda$ = 0.1542 nm (Cu-K$\alpha$).

As a result, at least 6 sharp scattering peaks were observed. The peak value ratio indicated the following ratio peculiar to the cubic liquid crystal belonging to the crystallographic space group Pn3m:

$$\sqrt{2}:\sqrt{3}:\sqrt{4}:\sqrt{6}:\sqrt{8}:\sqrt{9}\ .$$

Thus, the gel sample was confirmed to form a cubic liquid crystal that belongs to the crystallographic space group Pn3m. The result of SAXS analysis is shown in Figure 1.

[Example 10]

Formation of a liquid crystal by mono-O-(5,9,13,17-tetramethyloctadeca-4,8,12,16-tetraenoyl)glycerol and analysis thereof

Mono-O-(5,9,13,17-tetramethyloctadeca-4,8,12,16-tetraenoyl)glycerol synthesized in Example 4 and water were homogeneously mixed in accordance with the same procedure as in Example 9 to obtain a sample of mono-O-(5,9,13,17-tetramethyloctadeca-4,8,12,16-tetraenoyl)glycerol/water system as a white turbid gel composition in appearance. SAXS analysis of the gel sample was performed in the same manner as in Example 9. As a result, at least 3 scattering peaks were observed. The peak value ratio indicated the following ratio peculiar to the reverse hexagonal liquid crystal:

$$1:\sqrt{3}:2\ .$$

Thus, the sample of mono-O-(5,9,13,17-tetramethyloctadeca-4,8,12,16-tetraenoyl)glycerol/water system was confirmed to form a reverse hexagonal liquid crystal. The result of SAXS analysis is shown in Figure 2.

[Example 11]

Formation of a liquid crystal by mono-O-(5,9,13,17-tetramethyloctadeca-4,8,12,16-tetraenoyl)erythritol and analysis thereof

Mono-O-(5,9,13,17-tetramethyloctadeca-4,8,12,16-tetraenoyl)erythritol synthesized in Example 5 and water were homogeneously mixed in accordance with the same procedure as in Example 9 to obtain a sample of mono-O-(5,9,13,17-tetramethyloctadeca-4,8,12,16-tetraenoyl)erythritol/water system as a colorless transparent gel composition in appearance. SAXS analysis of the gel sample was performed in the same manner as in Example 9. As a result, at least 6 scattering peaks were observed. The peak value ratio indicated the following ratio peculiar to the cubic liquid crystal belonging to the crystallographic space group Pn3m:

$$\sqrt{2}:\sqrt{3}:\sqrt{4}:\sqrt{6}:\sqrt{8}:\sqrt{9}\ .$$

Thus, the sample of mono-O-(5,9,13,17-tetramethyloctadeca-4,8,12,16-tetraenoyl)erythritol/water system was confirmed to form a cubic liquid crystal that belongs to the crystallographic space group Pn3m. The result of SAXS analysis is shown in Figure 3.

[Example 12]

Formation of a liquid crystal by mono-O-(5,9,13,17-tetramethyloctadeca-4,8,12,16-tetraenoyl)pentaerythritol and analysis thereof

Mono-O-(5,9,13,17-tetramethyloctadeca-4,8,12,16-tetraenoyl)pentaerythritol synthesized in Example 6 and water were homogeneously mixed in accordance with the same procedure as in Example 9 to obtain a sample of mono-O-(5,9,13,17-tetramethyloctadeca-4,8,12,16-tetraenoyl)pentaerythritol/water system as a white turbid gel composition in appearance. SAXS analysis of the gel sample was performed in the same manner as in Example 9. As a result, at least 6 scattering peaks were observed. The peak value ratio indicated the following ratio peculiar to the cubic liquid crystal belonging to the crystallographic space group Pn3m:

$$\sqrt{2}:\sqrt{3}:\sqrt{4}:\sqrt{6}:\sqrt{8}:\sqrt{9}\ .$$

Thus, the sample of mono-O-(5,9,13,17-tetramethyloctadeca-4,8,12,16-tetraenoyl)pentaerythritol/water system was confirmed to form a cubic liquid crystal that belongs to the crystallographic space group Pn3m. The result of SAXS analysis is shown in Figure 4.

[Example 13]

Synthesis of mono-O-(5,9,13-trimethyltetradec-4-enoyl)glycerol

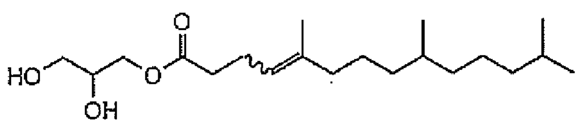

1.0 g (3.5 mmol) of methyl 5,9,13-trimethyltetradec-4-enoate was slowly added dropwise to a solution of 0.65 g (7.1 mmol) of glycerol and 0.59 g (4.3 mmol) of potassium carbonate in dry N,N-dimethylformamide (3.5 mL) at 80°C. After the reaction mixture was stirred at 100°C for 18 hours, 1M hydrochloric acid was added. The resulting solution was extracted with ether, and the extract was washed with saturated sodium bicarbonate aqueous solution and saturated brine, successively, and dried over anhydrous sodium sulfate. After filtration, the filtrate was concentrated, and the resulting residue was purified by silica gel column chromatography (ethyl acetate/hexane mixture) to obtain the title compound as a colorless transparent liquid.

$^{1}$H-NMR and viscosity of the obtained compound were measured. The results were as follows.

$^1$H-NMR spectrum (300 MHz, $CDCl_3$, TMS) δ: 0.80-0.90 (m, 9H), 1.00-1.70 (m, 15H), 1.97 (td, J=7.8, 17.0Hz, 2H), 2.13 (t, J=6.1Hz, 1H, OH), 2.25-2.45 (m, 4H), 2.55 (d, J=5.2Hz, 1H, OH), 3.50-4.00 (m, 3H), 4.10-4.25 (m, 2H), 5.08 (t, J=6.7Hz, 1H).

Viscosity: 0.48 Pa·s (at shear velocity of 92 1/s).

Mono-O-(5,9,13-trimethyltetradec-4-enoyl)glycerol synthesized was also referred to below as C17 glycerin ester.

[Example 14]

Synthesis of mono-O-(5,9,13,17-tetramethyloctadec-4-enoyl)diglycerol

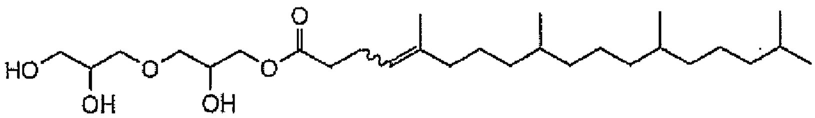

Under reduced pressure of 60-70 mmHg and nitrogen gas stream, 199 g (0.564 mol) of methyl 5,9,13,17-tetramethyloctadec-4-enoate was slowly added dropwise at 78-83°C to a solution of 259 g (1.56 mol) of diglycerol and 1.58 g (1.15 mmol) of potassium carbonate in dry N,N-dimethylformamide (700 mL). After the reaction mixture was stirred at the same temperature for 10 hours, formic acid was added at 75°C to adjust the pH to 4. After the resulting solution was subjected to vacuum concentration, the residue was dilluted with t-butylmethylether (1.5L), and the resulting insoluble matter was separated by filtration. The filtrate was washed with 10% sodium bicarbonate aqueous solution twice, and decolorized with activated carbon (8 g). After filtration, the filtrate was concentrated, and the residue was dissolved in ethanol, followed by filtration through cellulose powder. After the filtrate was concentrated, the resulting residue was purified by silica gel column chromatography (hexane/ethyl acetate mixture) to obtain the title compound as a transparent viscous liquid.

$^1$H-NMR of the obtained compound was measured. The result was as follows.

$^1$H-NMR spectrum (300 MHz, $CDCl_3$, TMS) δ: 0.80-0.90 (m, 12H), 1.00-1.70 (m, 22H), 1.97 (ddd, J=6.9, 7.8, 17.4Hz, 2H), 2.20-2.45 (m, 4H), 3.50-4.10 (m, 8H), 4.10-4.25 (m, 2H), 5.08 (dd, J=6.6, 6.6Hz,1H).

Mono-O-(5,9,13,17-tetramethyloctadec-4-enoyl)diglycerol synthesized was also referred to below as C22 diglycerin ester.

[Example 15]

Synthesis of 1-O-(3,7,11,15-tetramethylhexadecyl)-β-D-xylopyranoside

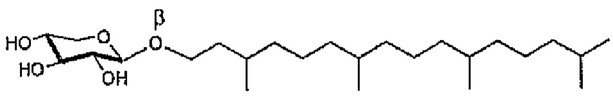

Under an argon atmosphere, 5 g (15.7 mM) of vacuum-dried tetra-O-acetyl-β-D-xylopyranoside and 100 ml of methylene chloride were added to 2 g of dried molecular sieve 4A, and the resultant was agitated for 10 to 30 minutes. The product was cooled to 5°C to 8°C, 16 ml of a solution of 1M tin chloride in methylene chloride was added dropwise thereto, and the mixture was agitated at room temperature for 20 minutes. After the resultant was cooled to -10°C, 16 ml of a solution of 4.69 g (15.7 mM) of 3,7,11,15-tetramethylhexadecanol in methylene chloride was added dropwise over about 30 minutes, and agitation was continued in that state for 4 hours. The resulting solution was introduced into a saturated aqueous solution of sodium bicarbonate, and extraction was carried out 3 times with 100 ml of methylene chloride, followed by washing with water. The organic phase was dried over anhydrous sodium sulfate, filtrated, and then concentrated. Subsequently, the mixture was purified by silica gel column chromatography (eluent: a mixed solvent of hexane/ethyl acetate).

The resulting 1-O-(3,7,11,15-tetramethylhexadecyl)-β-D-xylopyranoside triacetate was dissolved in 5.5 ml of methanol, and 2.5 ml of 0.05M sodium methylate was added thereto. After the mixture was agitated at room temperature for 4.5 hours, the equal amount of 1N hydrochloric acid was added for neutralization. After the solution was concentrated, the concentrate was purified by silica gel column chromatography (eluent: a mixed solvent of chloroform/methanol), and

the resultant was dried under reduced pressure to obtain the title compound, 1-O-(3,7,11,15-tetramethylhexadecyl)-β-D-xylopyranoside as a white waxy solid. $^1$H-NMR measurement demonstrated that contamination by 1-O-(3,7,11,15-tetramethylhexadecyl)-α-D-xylopyranoside did not take place.

$^1$H-NMR spectrum (270 MHz, $CDCl_3$, TMS) δ: 0.8-0.9 (m, 15H), 0.95-1.75 (m, 24H), 2.71 (brs, 1H, OH), 2.87 (brs, 1H, OH), 3.26 (brs, 1H, OH), 3.37 (dd, J=8.0, 11.9Hz, 1H), 3.4-3.65 (m, 3H), 3.74 (m, 1H), 3.90 (m, 1H), 4.04 (dd, J=4.2, 11.9Hz, 1H), 4.37 (d, J=6.0Hz, 1H).

1-O-(3,7,11,15-tetramethylhexadecyl)-β-D-xylopyranoside synthesized was also referred to below as β-XP.

[Example 16]

Synthesis of mono-O-(,5,9,13,17-tetramethyloctadecanoyl)erythritol

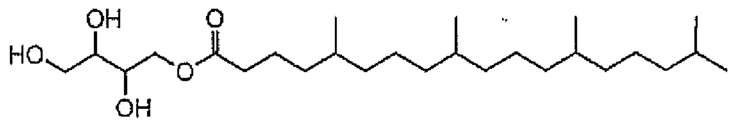

Under a nitrogen atmosphere, one drop of pyridine was added to 10 g of 5,9,13,17-tetramethyloctadecanoic acid and 20 ml of methylene chloride, and 5.2 g of thionyl chloride was added dropwise thereto at room temperature. After the completion of dropwise addition, the mixture was refluxed for 1 hour and concentrated under reduced pressure to obtain 10.5 g of 5,9,13,17-tetramethyloctadecanoic acid chloride.

2.56 g of erythritol, 2.21 g of pyridine, and 70 ml of dry DMF were mixed and dissolved with heating. The resultant was cooled to room temperature, a solution of 5 g of 5,9,13,17-tetramethyloctadecanoic acid chloride obtained above in 10 ml of methylene chloride was added dropwise thereto, and the mixture was then agitated at room temperature for 1 hour. 100 ml of methylene chloride was added to the resulting reaction solution, and the mixture was washed 3 times with saturated brine, and dried over anhydrous sodium sulfate. Following filtration and concentration under reduced pressure, the concentrate was purified by silica gel column chromatography to obtain 2.83 g of transparent and semisolid mono-O-(5,9,13,17-tetramethyloctadecanoyl)erythritol. As a result of HPLC analysis of the obtained product, the purity of 1-O-(5,9,13,17-tetramethyloctadecanoyl)erythritol was 91.6% and that of 2-O-(5,9,13,17-tetramethyloctadecanoyl)erythritol was 8.4%. The results of $^1$H-NMR measurement are as shown below.

$^1$H-NMR spectrum (270 MHz, $CDCl_3$, TMS) δ: 0.8-0.9 (m, 15H), 1.0-1.7 (m, 26H), 2.11 (br.s, 1H), 2.33 (t, J=7.9Hz, 2H), 2.66 (br.s, 1H), 2.75 (br.s, 1H), 3.6-3.9 (m, 4H), 4.29-4.36 (m, 2H).

Mono-O-(5,9,13,17-tetramethyloctadecanoyl)erythritol synthesized was also referred to below as saturated C22 erythritol ester.

[Example 17]

Preparation of samples of topical formulations

(1) Test Samples 1-5: lipid/water/0.65cs dimethicone

In accordance with the amounts shown in Table 1, the compounds (lipids) synthesized in the above Examples were mixed with distilled water, followed by adding 0.65cs dimethicone (Dow Coming Q7-9180 Silocon Fluid 0.65CST), and stirring. Thus, test samples 1-5 were prepared as topical formulations.

Table 1

| | Lipid | Distilled water | 0.65cs Dimethycone |
|---|---|---|---|
| Test sample 1 | C17 glycerin ester 2.50 g | 0.70 g | 7.60 g |
| Test sample 2 | C22 glycerin ester 2.50 g | 0.51 g | 7.60 g |
| Test sample 3 | Saturated C17 glycerin ester 2.50 g | 0.80 g | 7.60 g |
| Test sample 4 | β-XP 2.50g | 1.25 g | 7.60 g |
| Test sample 5 | Saturated C22 erythritol ester 2.50 g | 0.36 g | 7.60 g |

(2) Test Samples 7-12: lipid/Pluronic/EtOH/water

In accordance with the amounts shown in Table 2, Pluronic (Unilube 70DP-950B, NOF Corporation) and ethanol (EtOH) were added to the compounds (lipids) synthesized in the above Examples, followed by stirring for 1 hour using a stirrer chip. Then, distilled water was added and the mixture was further stirred for 1 hour. Thus, test samples 7-12 were prepared as topical formulations.

Table 2

| | Lipid | Pluronic | EtOH | Distilled water |
|---|---|---|---|---|
| Test sample 7 | C17 glycerin ester 1.00 g | 0.25 g | 0.139 g | 6.00 g |
| Test sample 8 | C17 glycerin ester 1.00 g | 0.25 g | 0.417 g | 2.00 g |
| Test sample 9 | C22 diglycerin ester 1.00 g | 0.25 g | 0.417 g | 2.00 g |
| Test sample 10 | Saturated C17 glycerin ester 1.00 g | 0.25 g | 0.417 g | 2.00 g |
| Test sample 11 | β-XP 1.00 g | 0.25 g | 0.417 g | 2.00 g |
| Test sample 12 | Saturated C22 erythritol ester 1.00 g | 0.25 g | 0.417 g | 2.00 g |

[Example 18]

Preparation of pump spray agent samples

(1) Test samples 17, 18, 22, 24, 25: lipid/Pluronic/EtOH/water

In accordance with the amounts shown in Table 3, Pluronic (Unilube 70DP-950B, NOF Corporation) and ethanol were added to the compounds (lipids) synthesized in the above Examples, followed by stirring for 1 hour using a stirrer chip. Then, distilled water was added and the mixture was further stirred for 1 hour. Commercially available manual simple spray bottles were filled with 2 to 4 mL of the resulting solution, respectively, to prepare test samples 17, 18, 22, 24 and 25 as pump spray agents.

Each test sample was sprayed once onto a test surface from a distance of about 2 cm. The ranges coated with the solutions all became almost circular shapes. The diameters of these almost circular shapes, and ejection amounts, and corresponding amounts of lipid contained therein are shown in Table 3.

Table 3

| | Lipid | Pluronic | EtOH | Distilled water | Diameter (spray amount) | Corresponding lipid amount |
|---|---|---|---|---|---|---|
| Test sample 18 | C17 glycerin ester 1.00 g | 0.25 g | 0.139 g | 6.00 g | 2.5 cm (38.2 mg) | 5.1 mg |
| Test sample 24 | C22 diglycerin ester 1.00 g | 0.25 g | 1.68 g | 4.46 g | 2 cm (37.3 mg) | 5.0 mg |
| Test sample 22 | Glyceryl farnesylacetate 1.00 g | 0.25 g | 0.139 g | 6.00 g | 3 cm (31.4 mg) | 4.2 mg |
| Test sample 25 | Saturated C17 glycerin ester 1.00 g | 0.25 g | 0.139 g | 6.00 g | 2.2 cm (33.5 mg) | 4.5 mg |
| Test sample 17 | β-XP 1.00 g | 0.25 g | 0.139 g | 6.00 g | 2.5 cm (33.5 mg) | 4.5 mg |

(2) Test samples 19 and 20: lipid/Pluronic/EtOH/water/sodium hyaluronate

In accordance with the amounts shown in Table 3, Pluronic (Unilube 70DP-950B, NOF Corporation) and ethanol were added to the compounds (lipids) synthesized in the above Examples, followed by stirring for 1 hour using a stirrer chip. Then, an aqueous solution of sodium hyaluronate (hyaluronic acid FCH (FCH-80), Kikkoman Biochemifa Company) prepared in advance was added and the mixture was further stirred for 1 hour. Commercially available manual simple

spray bottles were filled with 2 to 4 mL of the resulting solution, respectively, to prepare test samples 19 and 20 as pump spray agents.

Each test sample was sprayed once onto a test surface from a distance of about 2 cm. The ranges coated with the solutions all became almost circular shapes. The diameters of these almost circular shapes, and spray amounts, and corresponding amounts of lipid contained therein are shown in Table 4.

Table 4

| | Lipid | Pluronic | EtOH | Distilled water | Sodium hyaluronate | Diameter (spray amount) | Corresponding lipid amount |
|---|---|---|---|---|---|---|---|
| Test sample 19 | C17 glycerin ester 1.00g | 0.25 g | 0.139 g | 5.99 g | 7.4 mg | 2.5 cm (42.1 mg) | 5.7 mg |
| Test sample 20 | C17 glycerin ester 1.00g | 0.25 g | 0.139 g | 5.96 g | 37 mg | 2.5 cm (44.2 mg) | 6.0 mg |

(3) Test Samples 13, 14, 21, 23: Preparation of lipid/dimethicone

In accordance with the amounts shown in Table 5, 0.65cs dimethicone (Dow Coming Q7-9180 Silocon Fluid 0. 65CST) was added to the compounds (lipids) synthesized in the above Examples, followed by stirring. Commercially available manual simple spray bottles were filled with 2 to 4 mL of the resulting solution, respectively, to prepare test samples 13, 14, 21, 23 as pump spray agents.

Each test sample was sprayed once onto a test surface from a distance of about 2 cm. The ranges coated with the solutions all became almost circular shapes. The diameters of these almost circular shapes, and spray amounts, and corresponding amounts of lipid contained therein are shown in Table 5.

Table 5

| | Lipid | 0.65cs dimethicone | Diameter (spray amount) | Corresponding lipid amount |
|---|---|---|---|---|
| Test sample 13 | C17 glycerin ester 1.00 g | 2.33 g | 3 cm (26 mg) | 7.9 mg |
| Test sample 23 | C22 diglycerin ester 1.00 g | 2.33 g | 2.3 cm (24.5 mg) | 7.3 mg |
| Test sample 14 | β-XP 1.00 g | 2.33 g | 2.5 cm (30 mg) | 9.0 mg |
| Test sample 21 | β-XP 1.00 g | 5.67 g | 2.6 cm (24 mg) | 4.2 mg |

(4) Preparation of physiological saline

A commercially available manual simple spray bottle was filled with 2 to 4 mL of physiological saline (Otsuka normal saline, Otsuka Pharmaceutical Factory Inc.) to prepare a pump spray agent of physiological saline.

(5) Preparation of sodium hyaluronate aqueous solution

50 mg of sodium hyaluronate (hyaluronic acid FCH (FCH-80), Kikkoman Biochemifa Company) was dissolved in 9.95 g of distilled water, and 2 to 4 mL of the resulting solution was filled in a commercially available manual simple spray bottle to prepare a pump spray agent of 0.5% sodium hyaluronate aqueous solution.

Furthermore, 2.00 g of 0.5% sodium hyaluronate aqueous solution was diluted with 8.00 g of distilled water, and the resulting solution was filled in a spray bottle in a similar manner to prepare a pump spray agent of 0.1% sodium hyaluronate aqueous solution.

[Example 19]

Preparation of aerosol agents

(1) C17 glycerin ester/n-butane = 5:95

The vessel equipped with an upright metering valve and a straight button (pore size 0.9 mm) was filled with 1.2 g of C17 glycerin ester, 22.8 g of liquefied petroleum gas (0.15 MPa, 20°C) to prepare an aerosol agent. The internal pressure (25°C) of the aerosol agent was 0.17 MPa.

The aerosol agent was sprayed once onto a test surface from a distance of 10 cm. The range coated with the solution became an almost circular shape with a diameter of 2.5 cm. The amount of C17 glycerin ester was 3.3 mg after the liquefied petroleum gas was sufficiently volatilized.

(2) C17 glycerin ester/n-butane = 10:90

The vessel equipped with an upright metering valve and a straight button (pore size 0.9 mm) was filled with 2.4 g of C17 glycerin ester, 21.6 g of liquefied petroleum gas (0.15 MPa, 20°C) to prepare a aerosol agent. The internal pressure (25°C) of the aerosol agent was 0.23 MPa.

The aerosol agent was sprayed once onto a test surface from a distance of 10 cm. The range coated with the solution became an almost circular shape with a diameter of 2.5 cm. The amount of C17 glycerin ester was 6.7 mg after the liquefied petroleum gas was sufficiently volatilized.

[Example 20]

Coating of tissues by pump spray agents

Test sample 18 was sprayed five times onto the upper parietal peritoneum and the liver of 10-week-old male Wistar rats. As a result, it was observed that the tissue surfaces of the peritoneum and the liver were coated therewith. Photographs of the peritoneum (Fig. 5A and B) and the liver (Fig. 5C and D) before and after spray of test sample 18 are shown in Figure 5.

Furthermore, test sample 13 (free from water) was sprayed five times onto the liver of a 10-week-old male Wistar rat, followed by spraying physiological saline thereon sufficiently. As a result, it was observed that the tissue surface of the liver was coated therewith. Photographs of the liver (Fig. 6A and B) before and after spray of test sample 13 are shown in Figure 6.

As shown in these Figures, it was observed that the tissue surfaces before spraying reflected light strongly by being covered with water, whereas the tissue surfaces after spraying reflected light weakly by being covered with a coat of a liquid crystal.

[Example 21]

Evaluation of adhesion preventing effect

(1) Evaluation method

In this Example, the adhesion preventing effect of each of the test samples was evaluated using 10-week-old male Wistar rats. First, the rats underwent general anesthesia with pentobarbital, and underwent laparotomy with an approximately 30 mm abdominal midline incision in the supine position. Approximately 20 mm incisions were underwent on the left and right upper parietal peritoneums, and complete hemostasis was performed. These peritoneum incisions were closed with a continuous suture of 6 stitches using Silk Suture 5-0.

Subsequently, the test samples were applied to cover the suture site of the right peritoneum (refer to the sections (2) to (4) below). After the formation of a coating was observed, two layers of the abdominal walls were each closed with a suture to finish the surgery.

After 7 days of the surgery, the rats underwent general anesthesia with pentobarbital and underwent laparotomy again. In accordance with the adhesion evaluation scores below, adhesions of the suture sites of peritoneum were evaluated.

Adhesion evaluation scores:

- Grade 0: No adhesion

- Grade 1: Peelable adhesion with mild traction
- Grade 2: Peelable adhesion with strong traction (impossible to peel with mild traction)
- Grade 3: Adhesion involving tissue damage by peeling (strong adhesion of adipose tissue), but without adhesion to other organs
- Grade 4: Adhesion to other organs, with difficulty in peeling (including impossibility of peeling)

In addition, the average score of adhesion evaluation for the untreated rat group (10 samples) was 2.90±1.10 (mean ± standard deviation).

(2) Application of topical formulations and adhesion evaluation

A solution of each of test samples 1 to 12 prepared in Example 17 was measured out by a pipettor at an amount corresponding to 10 mg of the lipid and added dropwise to the suture sites. Then, the test sample was spreaded in an almost circle with a diameter of 2.5 to 3 cm by fingers with latex gloves, for applying test samples 1 to 12 to the suture sites.

The adhesion to other organs (grade 4) was not observed for test samples 1 to 12 applied above.

The average scores of adhesion evaluation obtained for three rat subjects per each test sample, for example, were 2.67 ± 1.15 (mean ± standard deviation) for test sample 1, 2.33 ± 0.58, 2.67 ± 0.58, and 2.00 ± 1.73 (mean ± standard deviation) for test samples 4, 5 and 7, respectively. In addition, the average scores were 2.33 ± 0.58 and 1.0 ± 1.73 (mean ± standard deviation), for test samples 10 and 11, respectively.

(3) Application of pump spray agents and adhesion evaluation

Each test sample (pump spray agent) prepared in Example 18(1) to (3) was sprayed a predefined number of times (any of 1 to 3 times) onto the suture site from a distance of about 2 cm to apply the test sample so as to cover the suture site by the range coated with the solution in an almost circular shape.

Test samples 17 to 20, 22, 24, and 25 prepared in Example 18(1) and (2) weresprayed onto the suture sites, followed by allowing to stand for 5 to 10 minutes. Then, two layers of the abdominal walls were each closed with a suture to finish the surgery.

In addition, test samples 13, 14, 21, and 23 prepared in Example 18(3) were sprayed onto the suture sites, followed by allowing to stand for 2 minutes. Then, after the physiological saline prepared in Example 18(4) was sprayed sufficiently onto the suture sites, followed by allowing to stand for 5 to 10 minutes. Two layers of the abdominal walls were each closed with a suture to finish the surgery.

Furthermore, test sample 13 was sprayed, and then 0.1% or 0.5% sodium hyaluronate aqueous solution prepared in Example 18(5) instead of the above-mentioned physiological saline was sprayed onto the suture site and the same evaluation was performed.

As a result, the adhesion to other organs (grade 4) was not observed for three or six rat subjects each in the tests using the pump spray agents. In particular, the average scores of adhesion evaluation were 1.17 ± 0.75 and 1.33 ± 1.15 (mean ± standard deviation) for test sample 18 (C17 glycerin ester (o/w), two sprays), and test sample 22 (glyceryl farnesylacetate (o/w), two sprays), respectively, which indicates that adhesion was highly prevented (reduced). In addition, the average score of adhesion evaluation was 1.67 ± 0.82 (mean ± standard deviation) for test sample 13 sprayed followed by spraying of the physiological saline, which indicates that a good adhesion-preventing effect was exhibited. In addition, the average scores of adhesion evaluation were 2.67 ±1.15 and 2.67 ± 0.58 (mean ± standard deviation) for test samples 19 and 20, respectively, which indicates that adhesion was clearly prevented (reduced).

(4) Application of aerosol agents and adhesion evaluation

Each test sample (aerosol agent) prepared in Examples 19(1) and (2) was sprayed a predefined number of times (any of 1 to 3 times) onto the suture site from a distance of about 10 cm, to apply the test sample so as to cover the suture site of the right peritoneum, followed by allowing to stand for 2 minutes. Then, the physiological saline prepared in Example 18(4) was sprayed sufficiently onto the suture site, followed by allowing to stand for 5 to 10 minutes. Two layers of the abdominal walls were each closed with a suture to finish the surgery.

The adhesion to other organs (grade 4) was not observed in the tests using these aerosol agents. The average score of adhesion evaluation was 2.67 ± 0.58 (mean ± standard deviation) for the aerosol agent containing 10% C17 glycerin ester (C17 glycerin ester/n-butane = 10:90), which indicates that adhesion was clearly prevented (reduced).

[Comparative Example 1]

Preparation of glyceryl monooleate (GMO)

2.33 g of 0.65cs dimethicone (Dow Corning Q7-9180 Silocon Fluid 0.65CST) was added to 1.00 g of GMO (Rikemal XO-100L, Riken Vitamin Co., Ltd.), followed by stirring to prepare a solution.
However, crystals were precipitated in the solution and it became a white turbid solution or a semisolid, and therefore it was not appropriate as a subject of the adhesion evaluation.
Additionally, an o/w dispersion of GMO having the same composition as test sample 18 as described above was prepared, and stored at a refrigerated temperature (7°C). As a result, it became a gel with no fluidity at all. On the other hand, no change in state was observed for test sample 18 (o/w dispersion) not only at the room temperature but also at refrigeration temperature. In addidion, the o/w dispersion of GMO generated fine aggregates by heating to 80°C. Thus, it is shown that the o/w dispersion of GMO has low temperature stability.

[Comparative Example 2]

Preparation of liquid crystal precursor composition

In accordance with Example 14 described in JP Patent Publication No. 2008-528463A, GDO (4.30 g), SPC (Lecinol S-10E, 2.90 g), P80 (1.80 g), and ethanol (1.00g) were mixed to prepare a liquid crystal precursor composition (GDO/SPC/P80/EtOH = 43: 29: 18:10).
6 mL of distilled water was added to 1.388 g of the liquid crystal precursor composition, followed by stirring for 1 hour to prepare test sample 6 (GDO/SPC/P80/EtOH/water = 10.8: 2.7: 3.4: 1.9: 81.2).
However, GDO/SPC as components of test sample 6 were insoluble in dimethicone with causing phase separation. Thus, it was impossible to prepare a lipid/dimethicone preparation, unlike test samples 13, 14, 21 and 23. Moreover, although test sample 6 was filled in a spray bottle (WO00/47079) and was tried to be sprayed, it was impossible to spray it uniformly. It was considered that this was due to high viscosity of the sample.

[Comparative Example 3]

Evaluation of adhesion preventing effect of Seprafilm

Using the procedure shown in Example 21(1), Seprafilm (Kaken Pharmaceutical Co., Ltd.) instead of the test samples, was applied and its adhesion preventing effect was evaluated (10 rat subjects). The average score of adhesion evaluation was 2.30 ± 1.70 (mean ± standard deviation).

[Example 22]

Measurement of viscosity of gels

The viscosity was measured for the gels prepared by addition of water to the compounds synthesized in Examples 1, 4, and 5. Specifically, for the gel samples obtained by mixing homogeneously according to the same procedure as in Example 9, the shear viscosity was measured at a temperature of 25°C, using a viscosity and viscoelasticity measuring apparatus (Gemini II, Malvern Instruments Ltd.; cone plate φ25, cone angle 1°).
The measurement results at shear velocity of 12 1/s and 100.8 1/s are shown in Table 6.

Table 6

| Compound | Shear velocity (1/s) | Viscosity (Pas·sec) |
|---|---|---|
| Example 1 | 12<br>100.8 | 461<br>37 |
| Example 4 | 12<br>100.8 | 39<br>1.4 |
| Example 5 | 12<br>100.8 | 333<br>17 |

These compounds showed quite low viscosities as liquid crystal gels. On the other hand, since when compared with the viscosities of the lipid compounds themselves (see Examples 1, 4, and 5), the viscosities of the gels were greatly increased, it was verified that liquid crystal gels were actually formed.

[Example 23]

Synthesis of 3,7,11-trimethyldodeca-2,6,10-trienoic acid

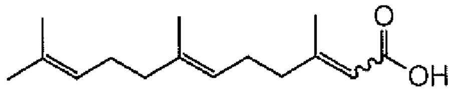

Under a nitrogen atmosphere, 23.7 mL (276 mmol) of oxalyl chloride was dissolved in methylene chloride (460 mL), and 49 mL (0.69 mol) of dimethyl sulfoxide was slowly added dropwise at -78°C. After the mixture was stirred for 15 min, 51.1 g (230 mmol) of farnesol was added, followed by stirring for 1 hour at the same temperature. After addition of 128 mL (0.920 mol) of trimethylamine, the reaction mixture was heated to room temperature, and was stirred for 1 hour. The mixture was diluted with 460 mL of hexane, and washed with water, 1M hydrochloric acid, saturated sodium bicarbonate aqueous solution, and saturated brine, successively, and dried over magnesium sulfate. After filtration, the filtrate was concentrated to obtain 47.84 g of a crude product of 3,7,11-trimethyldodeca-2,6,10-trien-1-al.

The above-mentioned crude product of 3,7,11-trimethyldodeca-2,6,10-trien-1-al was dissolved in t-butanol (326 mL) and water (217 mL). 69 mL (0.65 mol) of 2-methyl-2-butene, 67.7 g (434 mmol) of sodium dihydrogen phosphate dihydrate, and 39.3 g (434 mmol) of sodium chlorite were added sequentially. After stirring for 2 hours at room temperature, the reaction solution was diluted with ethyl acetate/hexane (1:3, 434 mL) and water (217 mL). The resulting separated organic layer was washed with 1M hydrochloric acid, saturated sodium bicarbonate aqueous solution, and saturated brine, successively, and dried over magnesium sulfate. After filtration, the filtrate was concentrated, and the resulting residue was purified by silica gel column chromatography (ethyl acetate/hexane mixture) to obtain 33.52 g of the title compound (62% yield in 2 steps)as a yellow transparent liquid, which has the following $^1$H-NMR spectrum.

$^1$H-NMR spectrum (270 MHz, $CDCl_3$, TMS) $\delta$: 1.6-1.7 (m, 9H), 1.9-2.2 (m, 11H), 5.09 (brs, 2H), 5.69 (s, 1H).

[Example 24]

Synthesis of 1-chloro-3,7,11-trimethyldodeca-2,6,10-triene

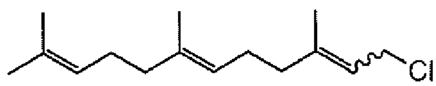

24.0 g (160 mmol) of N-chlorosuccinimide was suspended in methylene chloride (180 mL). After addition of 14.0 mL (192 mmol) of dimethylsulfide at 0°C, the mixture was stirred for 20 min. After addition of 26.7 g (120 mmol) of farnesol, the mixture was stirred for 30 min at 0°C and further stirred for 2 hours at room temperature. Saturated sodium bicarbonate aqueous solution was added to the reaction mixture and then extraction with hexane (240 mL) was performed. The extract was washed with saturated brine, and dried over magnesium sulfate. After filtration, the filtrate was concentrated to obtain 28.32 g of the title compound (98% yield) as a pale orange transparent liquid.

[Example 25]

Synthesis of 5,9,13-trimethyltetradeca-4,8,12-tuen-1-ol

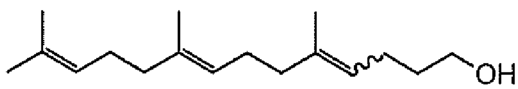

Under a nitrogen atmosphere, 12.5 g (330 mmol) of lithium aluminum hydride was suspended in dry tetrahydrofuran (600 mL). 167 g (600 mmol) of methyl farnesylacetate in dry tetrahydrofuran (300 mL) was added little by little to the solution at 0°C. The mixture was stirred for 30 min at 0°C, and further stirred for 4 hours at 60°C. Sodium sulfate and water were added to the resulting reaction mixture at 0°C, followed by stirring for 3 hours at room temperature. After the mixture was dried over sodium sulfate, and filtered, the filtrate was concentrated to obtain 132.3 g of the title compound (88% yield) as a pale yellow transparent liquid, which has the following $^1$H-NMR spectrum.

$^1$H-NMR spectrum (270 MHz, $CDCl_3$, TMS) $\delta$: 1.5-1.8 (m, 14H), 1.9-2.2 (m, 10H), 3.65 (t, J = 6Hz, 2H), 5.15 (m, 3H)

[Example 26]

Synthesis of 5,9,13-trimethyltetradeca-4,8,12-trien-1-yl p-toluenesulfonate

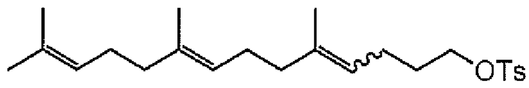

Under a nitrogen atmosphere, 58.7 g (308 mmol) of p-toluenesulfonyl chloride was added little by little at 0°C to a solution of 70.1 g (280 mmol) of 5,9,13-trimethyltetradeca-4,8,12-trien-1-ol, 43 mL (0.31 mol) of triethylamine, and 0.80 g (8.4 mmol) of trimethylamine hydrochloride in dry methylene chloride (140 mL). After stirring for 1 hour at 0°C, 7.0 mL (56 mmol) of N,N-dimethyl-1,3-propanediamine was added to the reaction mixture. After stirring for 10 min at the same temperature, the mixture was diluted with hexane (470 mL). The resulting solution was washed with water, 1M hydrochloric acid, saturated sodium bicarbonate aqueous solution, and saturated brine, successively, and dried over magnesium sulfate. After filtration, the filtrate was concentrated to obtain 108.2 g of the title compound (96% yield) as a pale yellow transparent liquid, which has the following $^1$H-NMR spectrum.

$^1$H-NMR spectrum (270 MHz, $CDCl_3$, TMS) $\delta$: 1.4-1.8 (m, 14H), 1.8-2.1 (m, 10H), 2.45 (s, 3H), 4.03 (t, J = 6.4Hz, 2H), 4.9-5.2 (m, 3H), 7.34 (d, J = 8.2Hz, 2H), 7.79 (d, J = 8.2Hz, 2H)

[Example 27]

Synthesis of mono-O-(3,7,11-trimethyldodeca-2,6,10-trienoyl)glycerol

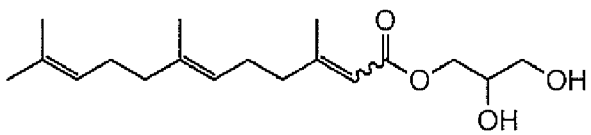

Under a nitrogen atmosphere, 4.36 mL (60.0 mmol) of thionyl chloride was added dropwise carefully at room temperature to a solution of 9.45 g (40.0 mmol) of 3,7,11-trimethyldodeca-2,6,10-trienoic acid and 0.16 mL (2.0 mmol) of pyridine in dry methylene chloride (140 mL), followed by stirring for 1 hour at 55°C. After the solution was concentrated, the concentrate was diluted with methylene chloride (36 mL) to obtain a solution of 3,7,11-trimethyldodeca-2,6,10-trienic acid chloride.

The above-mentioned solution of 3,7,11-trimethyldodeca-2,6,10-trienic acid chloride was added dropwise at 0°C to a solution of 5.53 g (60.0 mmol) of glycerol, 6.44 mL (80.0 mmol) of pyridine in dry N,N-dimethylformamide (200 mL), followed by stirring overnight at room temperature. The resulting reaction solution was diluted with methylene chloride (400 ml), and washed with water (400 mL). The aqueous layer was further extracted with methylene chloride (400 ml). The combined extracts were washed with 1M hydrochloric acid, saturated sodium bicarbonate aqueous solution, and saturated brine, successively, and dried over magnesium sulfate. After filtration, the filtrate was concentrated, and the resulting residue was purified by silica gel column chromatography (ethyl acetate/hexane mixture) to obtain 5.07 g of the title compound (41% yield) as a pale brown transparent liquid. $^1$H-NMR and viscosity of the obtained compound were measured. The results were as follows.

$^1$H-NMR spectrum (270 MHz, $CDCl_3$, TMS) $\delta$: 1.4-1.8 (m, 12H), 1.8-2.3 (m, 8H), 2.65 (brs, OH), 3.5-4.0 (m, 3H), 4.0-4.25 (m, 2H), 4.9-5.2 (m, 2H), 5.70 (brs, 1H).

Viscosity: 3.0 Pa·s (at shear velocity of 92 1/s).

[Example 28]

Synthesis of mono-O-(3,7,11-trimethyldodeca-2,6,10-trienyl)glycerol

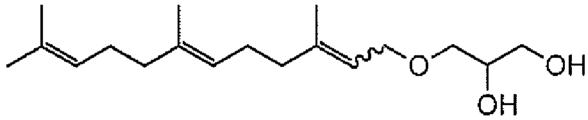

2.38 g (55%, 54.6 mmol) of sodium hydride was added to a solution of 5.03 g (54.6 mmol) of glycerol in dry N,N-dimethylformamide/tetrahydrofuran (1:1, 54 mL) with cooling on ice. After stirring for 30 min at 50°C, 8.77 g (36.4 mmol) of 1-chloro-3,7,11-trimethyldodeca-2,6,10-triene was added, followed by stirring overnight at the same temperature. After addition of saturated ammonium chloride aqueous solution (73 mL) at 0°C, the reaction mixture was extracted with ethyl acetate. The extract was washed with saturated sodium bicarbonate aqueous solution, and saturated brine, successively, and dried over magnesium sulfate. After filtration, the filtrate was concentrated, and the resulting residue was purified by silica gel column chromatography (ethyl acetate/hexane mixture) to obtain 2.16 g of the title compound (20% yield) as a pale yellow transparent liquid. $^1$H-NMR and viscosity of the obtained compound were measured. The results were as follows.

$^1$H-NMR spectrum (270 MHz, $CDCl_3$, TMS) $\delta$: 1.5-1.8 (m, 12H), 1.8-2.2 (m, 8H), 2.22 (brs, OH), 2.66 (brs, OH), 3.45-3.9 (m, 5H), 4.0-4.2 (m, 2H), 5.10 (brs, 2H), 5.33 (m,1H).

Viscosity: 0.20 Pa·s (at shear velocity of 92 1/s).

[Example 29]

Synthesis of mono-O-(3,7,11-trimethyldodeca-2,6,10-trienyl)erythritol

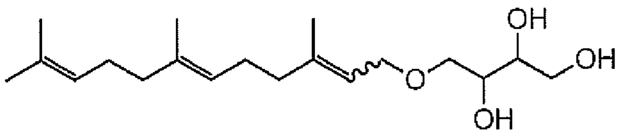

The title compound was synthesized using the same procedure and the same relative amounts of reagents as employed in Example 28, but with 7.33 g (60 mmol) of erythritol instead of 5.03 g (54.6 mmol) of glycerol. The compound was obtained (0.803 g, 6.1% yield) as a pale yellow transparent liquid, which has the following $^1$H-NMR spectrum:

$^1$H-NMR spectrum (270 MHz, $CDCl_3$, TMS) $\delta$: 1.5-1.8 (m, 12H), 1.8-2.2 (m, 8H), 2.56 (brs, OH), 2.83 (brs, OH), 2.96 (brs, OH), 3.59 (m, 2H), 3.7-3.9 (m, 4H), 4.05 (d, J=6.8Hz, 2H), 5.09 (m, 2H), 5.33 (t, J=6.8Hz, 1H).

[Example 30]

Synthesis of mono-O-(3,7,11-trimethyldodeca-2,6,10-trienyl)pentaerythritol

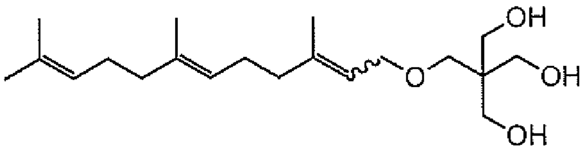

The title compound was synthesized using the same procedure and the same relative amounts of reagents as employed in Example 28, but with 8.17 g (60 mmol) of pentaerythritol instead of 5.03 g (54.6 mmol) of glycerol. The

compound was obtained (2.75 g, 20% yield) as a pale yellow transparent liquid, which has the following $^1$H-NMR spectrum and viscosity:

$^1$H-NMR spectrum (270 MHz, $CDCl_3$, TMS) δ: 1.6-1.8 (m, 12H), 1.9-2.2 (m, 8H), 2.69 (brs, 30H), 3.46 (s, 2H), 3.72 (d, J=4.8Hz, 6H), 3.99 (d, J=6.6Hz, 2H), 5.09 (m, 2H), 5.30 (t, J=6Hz, 1H).

Viscosity: 1.6 Pa·s (at shear velocity of 92 1/s).

[Example 31]

Synthesis of mono-O-(5,9,13-trimethyltetradeca-4,8,12-trienyl)glycerol

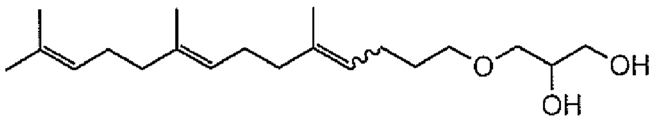

The title compound was synthesized using the same procedure and the same relative amounts of reagents as employed in Example 28, but with 16.2 g (40.0 mmol) of 5,9,13-trimethyltetradeca-4,8,12-trien-1-yl p-toluenesulfonate and 5.53 g (60.0 mmol) of glycerol instead of 8.77 g (36.4 mmol) of 1-chloro-3,7,11-trimethyldodeca-2,6,10-triene and 5.03 g (54.6 mmol) of glycerol, respectively. The compound was obtained (2.19 g, 17% yield) as a colorless transparent liquid, which has the following $^1$H-NMR spectrum and viscosity:

$^1$H-NMR spectrum (270 MHz, $CDCl_3$, TMS) δ: 1.55-1.7 (m, 14H), 1.95-2.15 (m, 10H), 2.23 (t, J=6Hz, OH), 2.66 (d, J=5Hz, OH), 3.4-3.9 (m, 7H), 5.10 (brs, 3H).

Viscosity: 0.23 Pa·s (at shear velocity of 92 1/s).

[Example 32]

Synthesis of mono-O-(5,9,13-trimethyltetradeca-4,8,12-trienyl)erythritol

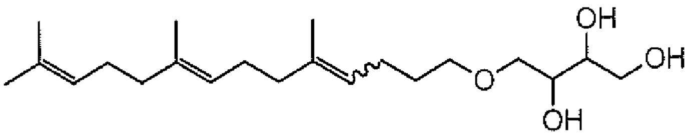

The title compound was synthesized using the same procedure and the same relative amounts of reagents as employed in Example 28, but with 16.2 g (40.0 mmol) of 5,9,13-trimethyltetradeca-4,8,12-trien-1-yl p-toluenesulfonate and 7.33 g (60.0 mmol) of erythritol instead of 8.77 g (36.4 mmol) of 1-chloro-3,7,11-trimethyldodeca-2,6,10-triene and 5.03 g (54.6 mmol) of glycerol, respectively. The compound was obtained (2.05 g, 14% yield) as a colorless transparent liquid, which has the following $^1$H-NMR spectrum and viscosity:

$^1$H-NMR spectrum (270 MHz, $CDCl_3$, TMS) δ: 1.55-1.8 (m, 14H), 1.9-2.1 (m, 10H), 2.49 (brs, OH), 2.78 (d, J=5Hz, OH), 2.90 (m, OH), 3.4-3.9 (m, 8H), 5.10 (brs, 3H).

Viscosity: 1.2 Pa·s (at shear velocity of 92 1/s).

[Example 33]

Synthesis of mono-O-(5,9,13-trimethyltetradeca-4,8,12-trienyl)pentaerythritol

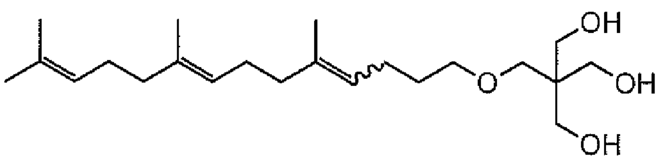

The title compound was synthesized using the same procedure and the same relative amounts of reagents as employed in Example 28, but with 16.2 g (40.0 mmol) of 5,9,13-trimethyltetradeca-4,8,12-trien-1-yl p-toluenesulfonate and 8.17 g (60.0 mmol) of pentaerythritol instead of 8.77 g (36.4 mmol) of 1-chloro-3,7,11-trimethyldodeca-2,6,10-triene and 5.03 g (54.6 mmol) of glycerol, respectively. The compound was obtained (3.09 g, 21% yield) as a colorless transparent liquid, which has the following $^1$H-NMR spectrum and viscosity:

$^1$H-NMR spectrum (270 MHz, $CDCl_3$, TMS) δ: 1.55-1.7 (m, 14H), 1.9-2.15 (m, 10H), 2.60 (m, 30H), 3.42 (t, J=6.5Hz, 2H), 3.46 (s, 2H), 3.72 (d, J=4.8Hz, 6H), 5.10 (t, J=6.6Hz, 3H).

Viscosity: 0.63 Pa·s (at shear velocity of 92 1/s).

[Example 34]

Synthesis of mono-O-(5,9,13-triomethyltetradeca-4,8,12-trienyl)diglycerol

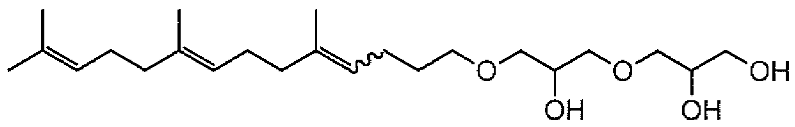

The title compound was synthesized using the same procedure and the same relative amounts of reagents as employed in Example 28, but with 16.2 g (40.0 mmol) of 5,9,13-trimethyltetradeca-4,8,12-trien-1-yl p-toluenesulfonate and 10.0 g (60.0 mmol) of diglycerol instead of 8.77 g (36.4 mmol) of 1-chloro-3,7,11-trimethyldodeca-2,6,10-triene and 5.03 g (54.6 mmol) of glycerol, respectively. The compound was obtained (3.97 g, 25% yield) as a pale yellow transparent liquid, which has the following $^1$H-NMR spectrum and viscosity:

$^1$H-NMR spectrum (270 MHz, $CDCl_3$, TMS) δ: 1.5-1.8 (m, 14H), 1.9-2.15 (m, 10H), 2.2-2.8 (m, 30H), 3.4-4.0 (m, 12H), 5.10 (brs, 3H).

Viscosity: 0.37 Pa·s (at shear velocity of 92 1/s).

[Example 35]

Synthesis of 3,7,11-trimethyldodeca-2,6,10-trienyl glycerate

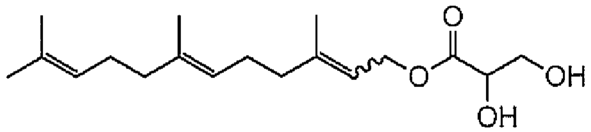

A solution of 0.222 g (1.00 mmol) of farnesol, 0.109 mL (0.75 mmol) of methyl (±)-2,2-dimethyl-1,3-dioxolane-4-carboxylate, and 0.138 g (1.00 mmol) of potassium carbonate in dry N,N-dimethylformamide (1.5 mL) was stirred for 2 hours at 95°C under reduced pressure (60 to 70 kPa). Then, under the same pressure and at the same temperature, addition of 0.109 mL (0.75 mmol) of methyl (±)-2,2-dimethyl-1,3-dioxolane-4-carboxylate and 2 hours of stirring repeated twice. The reaction mixture was cooled to room temperature, followed by dilution with ethyl acetate/hexane mixture (1:2). The solution was washed with water, saturated sodium bicarbonate aqueous solution, and saturated brine, successively, and dried over magnesium sulfate. After filtration, the filtrate was concentrated, and the resulting residue was purified by silica gel column chromatography (ethyl acetate/hexane mixture) to obtain 0.283 g of acetonide-protected glycerate ester (81% yield).

A solution of 0.283 g (0.80 mmol) of above-mentioned acetonide-protected glycerate ester in 0.80 mL (2.4 mmmol) of 3M hydrochloric acid and tetrahydrofuran (3.2 mL) was stirred for 10 hours at room temperature. The resulting reaction mixture was diluted with ethyl acetate, and washed with water, saturated sodium bicarbonate aqueous solution, and saturated brine, successively, and dried over magnesium sulfate. After filtration, the filtrate was concentrated, and the resulting residue was purified by silica gel column chromatography (ethyl acetate/hexane mixture) to obtain 58.1 mg of the title compound (23% yield) as a pale yellow transparent liquid. $^1$H-NMR of the obtained compound was measured. The result was as follows.

$^1$H-NMR spectrum (270 MHz, $CDCl_3$, TMS) δ: 1.5-1.8 (m, 12H), 1.9-2.2 (m, 8H+OH), 3.12 (d, J=4.7Hz, OH),

3.87 (m, 2H), 4.25 (m, 1H), 4.74 (m, 2H), 5.09 (m, 2H), 5.36 (t, J=7Hz, 1H).

[Example 36]

Synthesis of 5,9,13-trimethyltetradeca-4,8,12-trienyl glycerate

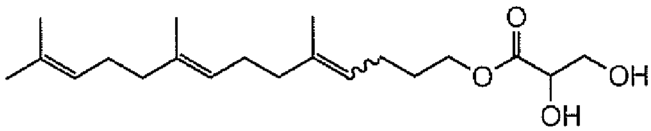

The title compound was synthesized using the same procedure and the same relative amounts of reagents as employed in Example 35, but with 5.01 g (20.0 mmol) of 5,9,13-trimethyltetradeca-4,8,12-trien-1-ol instead of 0.222 g (1.00 mmol) of farnesol. The compound was obtained (1.81 g, 27% yield in 2 steps) as a pale yellow transparent liquid, which has the following $^{1}$H-NMR spectrum and viscosity:
$^{1}$H-NMR spectrum (270 MHz, $CDCl_3$, TMS) δ: 1.55-1.8 (m, 14H), 1.9-2.15 (m, 10H), 2.20 (brs, OH), 3.20 (brs, OH), 3.89 (m, 2H), 4.22 (t, J=6.7Hz, 2H), 4.24 (m, 1H), 5.09 (m, 3H).
Viscosity: 0.16 Pa·s (at shear velocity of 92 1/s).

[Example 37]

Synthesis of geranylgeranic acid (i.e., 3,7,11,15-tetramethylhexadeca-2,6,10,14-tetraenoic acid)

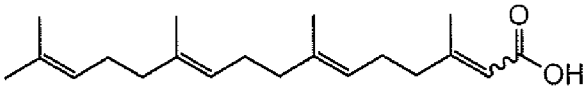

The title compound was synthesized using the same procedure and the same relative amounts of reagents as employed in Example 23, but with 20.3 g (70.0 mmol) of geranylgeraniol instead of 51.1 g (230 mmol) of farnesol. The compound was obtained (16.6 g, 78% yield in 2 steps) as a pale yellow transparent liquid, which has the following $^{1}$H-NMR spectrum:
$^{1}$H-NMR spectrum (270 MHz, $CDCl_3$, TMS) δ: 1.55-1.75 (m, 12H), 1.9-2.2 (m, 15H), 5.10 (m, 3H), 5.68 (s, 1H).

[Example 38]

Synthesis of 1-chloro-3,7,11,15-tetramethylhexadeca-2,6,10,14-tetraene

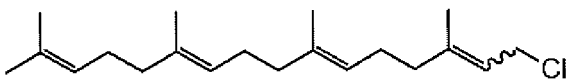

The title compound was synthesized using the same procedure and the same relative amounts of reagents as employed in Example 24, but with 17.4 g (60.0 mmol) of geranylgeraniol instead of 26.7 g (120 mmol) of farnesol. The compound was obtained (18.8 g, 100% yield) as an orange transparent liquid, which has the following $^{1}$H-NMR spectrum:
$^{1}$H-NMR spectrum (270 MHz, $CDCl_3$, TMS) δ: 1.5-1.9 (m, 15H), 1.9-2.2 (m, 12H), 4.11 (d, J=7.9Hz, 2H), 5.11 (m, 3H), 5.45 (t, J=7.9Hz, 1H).

[Example 39]

Synthesis of 5,9,13,17-tetramethyloctadeca-4,8,12,16-tetraen-1-ol

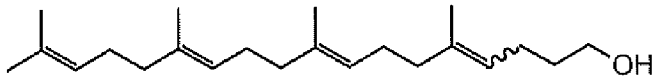

The title compound was synthesized using the same procedure and the same relative amounts of reagents as employed in Example 25, but with 138.6 g (400.0 mmol) of methyl 5,9,13,17-tetramethyloctadeca-4,8,12,16-tetraenoate (or methyl geranylgeranylacetate) instead of 167 g (600 mmol) of methyl farnesylacetate. The compound was obtained (105.47 g, 83% yield) as a colorless transparent liquid, which has the following $^1$H-NMR spectrum:

$^1$H-NMR spectrum (270 MHz, $CDCl_3$, TMS) $\delta$: 1.55-1.7 (m, 17H), 1.9-2.15 (m, 14H), 3.65 (t, J=6.4Hz, 2H), 5.12 (m, 4H).

[Example 40]

Synthesis of 5,9,13,17-tetramethyloctadeca-4,8,12,16-tetraen-1-yl p-toluenesulfonate

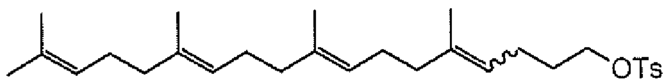

The title compound was synthesized using the same procedure and the same relative amounts of reagents as employed in Example 26, but with 41.4 g (130 mmol) of 5,9,13,17-tetramethyloctadeca-4,8,12,16-tetraen-1-ol instead of 70.1 g (280 mmol) of 5,9,13-trimethyltetradeca-4,8,12-trien-1-ol. The compound was obtained (54.9 g, 100% yield), which has the following $^1$H-NMR spectrum:

$^1$H-NMR spectrum (270 MHz, $CDCl_3$, TMS) $\delta$: 1.55-1.7 (m, 17H), 1.9-2.15 (m, 14H), 2.45 (s, 3H), 4.02 (m, 2H), 4.9-5.2 (m, 4H), 7.34 (d, J=8.2Hz, 2H), 7.79 (d, J=8.2Hz, 2H).

[Example 41]

Synthesis of mono-O-(3,7,11,15-tetramethylhexadeca-2,6,10,14-tetraenoyl)glycerol

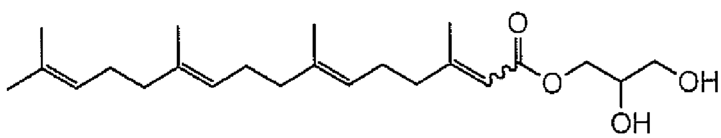

The title compound was synthesized using the same procedure and the same relative amounts of reagents as employed in Example 27, but with 4.57 g (15.0 mmol) of geranylgeranic acid (3,7,11,15-tetramethylhexadeca-2,6,10,14-tetraenoic acid) and 2.07 g (22.5 mmol) of glycerol instead of 9.45 g (40.0 mmol) of 3,7,11-trimethyldodeca-2,6,10-trienoic acid and 5.53 g (60.0 mmol) of glycerol, respectively. The compound was obtained (1.38 g, 24% yield) as a brown transparent liquid, which has the following $^1$H-NMR spectrum and viscosity:

$^1$H-NMR spectrum (270 MHz, $CDCl_3$, TMS) $\delta$: 1.4-1.8 (m, 15H), 1.8-2.3 (m, 12H), 2.62 (m, OH), 3.5-4.0 (m, 3H), 4.0-4.25 (m, 2H), 4.9-5.2 (m, 3H), 5.70 (brs, 1H).

Viscosity: 3.8 Pa·s (at shear velocity of 92 1/s).

[Example 42]

Synthesis of mono-O-(3,7,11,15-tetramethylhexadeca-2,6,10,14-tetraenoyl)erythritol

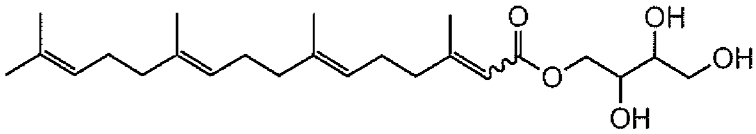

The title compound was synthesized using the same procedure and the same relative amounts of reagents as employed in Example 27, but with 4.57 g (15.0 mmol) of geranylgeranic acid (3,7,11,15-tetramethylhexadeca-2,6,10,14-tetraenoic acid) and 2.75 g (22.5 mmol) of erythritol instead of 9.45 g (40.0 mmol) of 3,7,11-trimethyldodeca-2,6,10-trienoic acid and 5.53 g (60.0 mmol) of glycerol, respectively. The compound was obtained (1.25 g, 20% yield) as a brown transparent liquid, which has the following $^1$H-NMR spectrum:

$^1$H-NMR spectrum (270 MHz, $CDCl_3$, TMS) $\delta$: 1.4-1.8 (m, 15H), 1.8-2.3 (m, 12H), 2.66 (t, J=10Hz, OH), 2.78 (m, OH), 2.93 (brs, OH), 3.63 (m, 1H), 3.7-4.0 (m, 3H), 4.29 (dd, J=2.9, 12.2Hz, 1H), 4.39 (dd, J=5.4, 12.2Hz, 1H), 4.9-5.2 (m, 3H), 5.72 (brs, 1H).

[Example 43]

Synthesis of mono-O-(3,7,11,15-tetramethylhexadeca-2,6,10,14-tetraenoyl)pentaerythritol

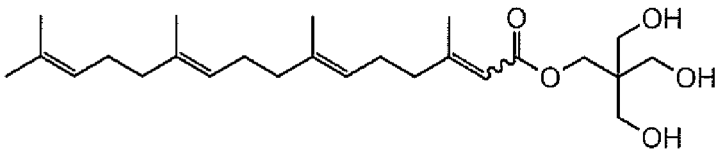

The title compound was synthesized using the same procedure and the same relative amounts of reagents as employed in Example 27, but with 4.57 g (15.0 mmol) of geranylgeranic acid (3,7,11,15-tetramethylhexadeca-2,6,10,14-tetraenoic acid) and 3.06 g (22.5 mmol) of pentaerythritol instead of 9.45 g (40.0 mmol) of 3,7,11-trimethyldodeca-2,6,10-trienoic acid and 5.53 g (60.0 mmol) of glycerol, respectively. The compound was obtained (1.53 g, 24% yield) as a brown transparent liquid, which has the following $^1$H-NMR spectrum and viscosity:

$^1$H-NMR spectrum (270 MHz, $CDCl_3$, TMS) $\delta$: 1.4-1.8 (m, 15H), 1.8-2.3 (m, 12H), 2.65 (m, 30H), 3.65 (m, 6H), 4.24 (m, 2H), 4.9-5.2 (m, 3H), 5.70 (brs, 1H).

Viscosity: 14 Pa·s (at shear velocity of 92 1/s).

[Example 44]

Synthesis of mono-O-(3,7,11,15-tetramethylhexadeca-2,6,10,14-tetraenyl)glycerol

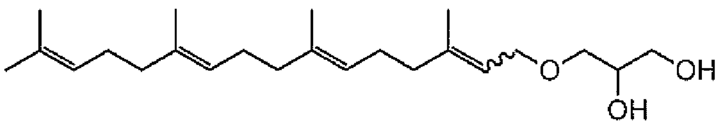

The title compound was synthesized using the same procedure and the same relative amounts of reagents as employed in Example 28, but with 5.56 g (18.0 mmol) of 1-chloro-3,7,11,15-tetramethylhexadeca-2,6,10,14-tetraene and 2.49 g (27.0 mmol) of glycerol instead of 8.77 g (36.4 mmol) of 1-chloro-3,7,11-trimethyldodeca-2,6,10-triene and 5.03 g (54.6 mmol) of glycerol, respectively. The compound was obtained (1.12 g, 17% yield) as a pale yellow transparent liquid, which has the following $^1$H-NMR spectrum and viscosity:

$^1$H-NMR spectrum (270 MHz, $CDCl_3$, TMS) $\delta$: 1.5-1.8 (m, 15H), 1.9-2.2 (m, 12H), 2.2 (m, OH), 2.64 (brs, OH), 3.45-3.9 (m, 5H), 4.0-4.2 (m, 2H), 5.10 (brs, 3H), 5.33 (m, 1H).
Viscosity: 0.26 Pa·s (at shear velocity of 92 1/s).

[Example 45]

Synthesis of mono-O-(3,7,11,15-tetramethylhexadeca-2,6,10,14-tetraenyl)erythritol

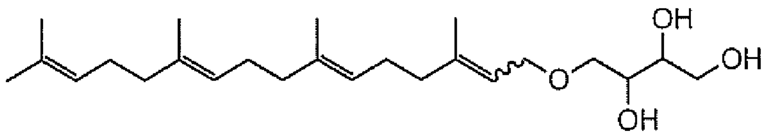

The title compound was synthesized using the same procedure and the same relative amounts of reagents as employed in Example 28, but with 6.18 g (20.0 mmol) of 1-chloro-3,7,11,15-tetramethylhexadeca-2,6,10,14-tetraene and 3.66 g (30.0 mmol) of erythritol instead of 8.77 g (36.4 mmol) of 1-chloro-3,7,11-trimethyldodeca-2,6,10-triene and 5.03 g (54.6 mmol) of glycerol, respectively. The compound was obtained (1.47 g, 19% yield) as a pale yellow transparent liquid, which has the following $^1$H-NMR spectrum and viscosity:
$^1$H-NMR spectrum (270 MHz, $CDCl_3$, TMS) $\delta$: 1.5-1.8 (m, 15H), 1.8-2.2 (m, 12H), 2.38 (m, OH), 2.70 (d, J=5Hz, OH), 2.82 (d, J=5Hz, OH), 3.4-3.7 (m, 2H), 3.7-3.9 (m, 4H), 4.0-4.2 (m, 2H), 5.10 (brs, 3H), 5.33 (m, 1H).
Viscosity: 1.9 Pa·s (at shear velocity of 92 1/s).

[Example 46]

Synthesis of mono-O-(3,7,11,15-tetramethylhexadeca-2,6,10,14-tetraenyl)pentaerythritol

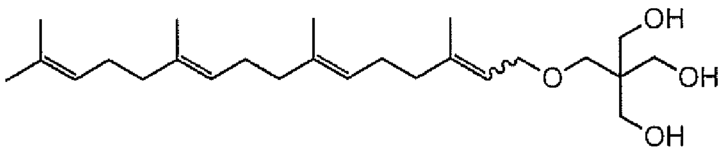

The title compound was synthesized using the same procedure and the same relative amounts of reagents as employed in Example 28, but with 6.18 g (20.0 mmol) of 1-chloro-3,7,11,15-tetramethylhexadeca-2,6,10,14-tetraene and 4.08 g (30.0 mmol) of pentaerythritol instead of 8.77 g (36.4 mmol) of 1-chloro-3,7,11-trimethyldodeca-2,6,10-triene and 5.03 g (54.6 mmol) of glycerol, respectively. The compound was obtained (1.62 g, 20% yield) as a pale yellow transparent liquid, which has the following $^1$H-NMR spectrum and viscosity:
$^1$H-NMR spectrum (270 MHz, $CDCl_3$, TMS) $\delta$: 1.55-1.8 (m, 15H), 1.8-2.2 (m, 12H), 2.58 (t, J=5Hz, 3OH), 3.46 (s, 2H), 3.72 (d, J=5.2Hz, 6H), 3.98 (d, J=6.7Hz, 2H), 5.10 (m, 3H), 5.30 (t, J=6.8Hz, 1H).
Viscosity: 2.9 Pa·s (at shear velocity of 92 1/s).

[Example 47]

Synthesis of mono-O-(3,7,11,15-tetramethylhexadeca-2,6,10,14-tetraenyl)diglycerol

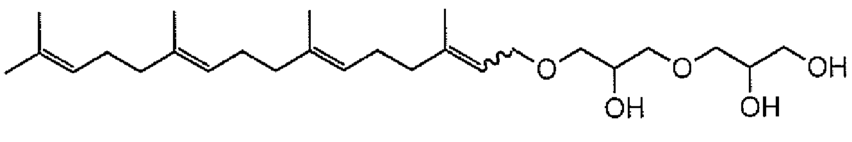

The title compound was synthesized using the same procedure and the same relative amounts of reagents as

employed in Example 28, but with 0.31 g (1.0 mmol) of 1-chloro-3,7,11,15-tetramethylhexadeca-2,6,10,14-tetraene and 0.25 g (1.5 mmol) of diglycerol instead of 8.77 g (36.4 mmol) of 1-chloro-3,7,11-trimethyldodeca-2,6,10-triene and 5.03 g (54.6 mmol) of glycerol, respectively. The compound was obtained (13.4 mg, 3.0% yield) as a pale yellow transparent liquid, which has the following $^1$H-NMR spectrum:

$^1$H-NMR spectrum (270 MHz, $CDCl_3$, TMS) δ: 1.5-1.8 (m, 15H), 1.85-2.2 (m, 12H), 3.4-4.5 (m, 12H), 5.09 (m, 3H), 5.33 (brs, 1H).

[Example 48]

Synthesis of mono-O-(5,9,13,17-tetramethyloctadeca-4,8,12,16-tetraenyl)glycerol

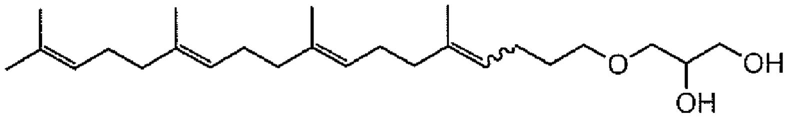

The title compound was synthesized using the same procedure and the same relative amounts of reagents as employed in Example 28, but with 11.8 g (25.0 mmol) of 5,9,13,17-tetramethyloctadeca-4,8,12,16-tetraen-1-yl p-toluenesulfonate and 3.45 g (37.5 mmol) of glycerol instead of 8.77 g (36.4 mmol) of 1-chloro-3,7,11-trimethyldodeca-2,6,10-triene and 5.03 g (54.6 mmol) of glycerol, respectively. The compound was obtained (309 mg, 3.1% yield) as a slightly yellow transparent liquid, which has the following $^1$H-NMR spectrum and viscosity:

$^1$H-NMR spectrum (270 MHz, $CDCl_3$, TMS) δ: 1.55-1.75 (m, 17H), 1.9-2.2 (m, 14H+OH), 2.58 (d, J=5Hz, OH), 3.4-3.6 (m, 4H), 3.68 (m, 2H), 3.85 (m, 1H), 5.14 (m, 4H).

Viscosity: 0.16 Pa·s (at shear velocity of 92 1/s).

[Example 49]

Synthesis of mono-O-(5,9,13,17-tetramethyloctadeca-4,8,12,16-tetraenyl)erythritol

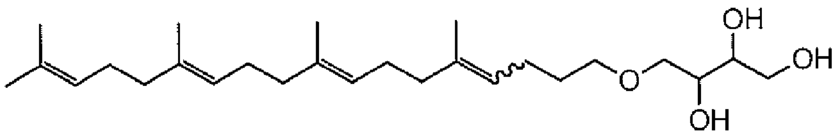

The title compound was synthesized using the same procedure and the same relative amounts of reagents as employed in Example 28, but with 7.09 g (15.0 mmol) of 5,9,13,17-tetramethyloctadeca-4,8,12,16-tetraen-1-yl p-toluenesulfonate and 2.75 g (22.5 mmol) of erythritol instead of 8.77 g (36.4 mmol) of 1-chloro-3,7,11-trimethyldodeca-2,6,10-triene and 5.03 g (54.6 mmol) of glycerol, respectively. The compound was obtained (265 mg, 4.2% yield) as a slightly yellow transparent liquid, which has the following $^1$H-NMR spectrum:

$^1$H-NMR spectrum (270 MHz, $CDCl_3$, TMS) δ: 1.5-1.8 (m, 17H), 1.9-2.2 (m, 14H), 2.42 (brs, OH), 2.74 (brs, OH), 2.85 (brs, OH), 3.4-3.65 (m, 4H), 3.65-3.9 (m, 4H), 5.11 (m, 4H).

[Example 50]

Synthesis of mono-O-(5,9,13,17-tetramethyloctadeca-4,8,12,16-tetraenyl)pentaerythritol

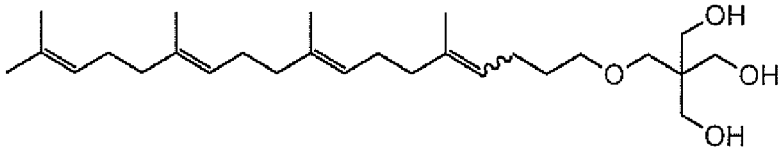

The title compound was synthesized using the same procedure and the same relative amounts of reagents as employed in Example 28, but with 11.8 g (25.0 mmol) of 5,9,13,17-tetramethyloctadeca-4,8,12,16-tetraen-1-yl p-toluenesulfonate and 5.11 g (37.5 mmol) of pentaerythritol instead of 8.77 g (36.4 mmol) of 1-chloro-3,7,11-trimethyldodeca-2,6,10-triene and 5.03 g (54.6 mmol) of glycerol, respectively. The compound was obtained (2.26 g, 21% yield) as a slightly yellow transparent liquid, which has the following $^1$H-NMR spectrum and viscosity:

$^1$H-NMR spectrum (270 MHz, $CDCl_3$, TMS) δ: 1.55-1.75 (m, 17H), 1.9-2.15 (m, 14H), 2.61 (brs, 3OH), 3.42 (t, J=6.5Hz, 2H), 3.46 (s, 2H), 3.72 (brs, 6H), 5.10 (brs, 4H).

Viscosity: 0.78 Pa·s (at shear velocity of 92 1/s).

[Example 51]

Synthesis of mono-O-(5,9,13,17-tetramethyloctadeca-4,8,12,16-tetraenyl)diglycerol

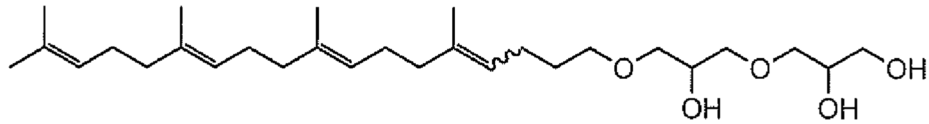

The title compound was synthesized using the same procedure and the same relative amounts of reagents as employed in Example 28, but with 11.8 g (25.0 mmol) of 5,9,13,17-tetramethyloctadeca-4,8,12,16-tetraen-1-yl p-toluenesulfonate and 6.25 g (37.5 mmol) of diglycerol instead of 8.77 g (36.4 mmol) of 1-chloro-3,7,11-trimethyldodeca-2,6,10-triene and 5.03 g (54.6 mmol) of glycerol, respectively. The compound was obtained (2.32 g, 20% yield) as a slightly yellow transparent liquid, which has the following $^1$H-NMR spectrum and viscosity:

$^1$H-NMR spectrum (270 MHz, $CDCl_3$, TMS) δ: 1.55-1.75 (m, 17H), 1.9-2.15 (m, 14H), 3.4-4.0 (m, 12H), 5.11 (m, 4H).

Viscosity: 1.4 Pa·s (at shear velocity of 92 1/s).

[Example 52]

Synthesis of 3,7,11,15-tetramethylhexadeca-2,6,10,14-tetraenyl glycerate

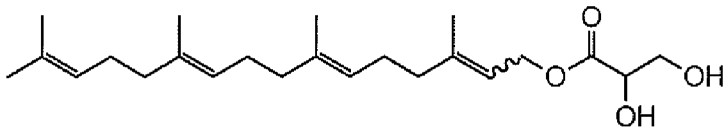

The title compound was synthesized using the same procedure and the same relative amounts of reagents as employed in Example 35, but with 5.81 g (20.0 mmol) of geranylgeraniol instead of 0.222 g (1.00 mmol) of farnesol. The compound was obtained (2.88 g, 39% yield in 2 steps) as a pale yellow transparent liquid, which has the following $^1$H-NMR spectrum and viscosity:

$^1$H-NMR spectrum (270 MHz, $CDCl_3$, TMS) δ: 1.5-1.8 (m, 15H), 1.9-2.2 (m, 12H+OH), 3.14 (m, OH), 3.88 (m, 2H), 4.26 (m, 1H), 4.75 (m, 2H), 5.10 (brs, 3H), 5.36 (t, J=7Hz, 1H).

Viscosity: 0.23 Pa·s (at shear velocity of 92 1/s).

[Example 53]

Synthesis of 5,9,13,17-tetramethyloctadeca-4,8,12,16-tetraenyl glycerate

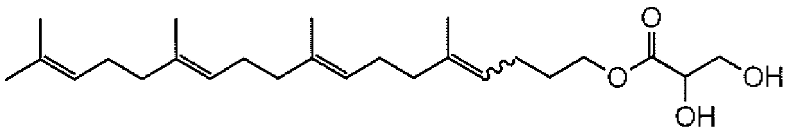

The title compound was synthesized using the same procedure and the same relative amounts of reagents as employed in Example 35, but with 6.37 g (20.0 mmol) of 5,9,13,17-tetramethyloctadeca-4,8,12,16-tetraen-1-ol instead of 0.222 g (1.00 mmol) of farnesol. The compound was obtained (2.99 g, 37% yield in 2 steps) as a pale yellow transparent liquid, which has the following $^1$H-NMR spectrum and viscosity:

$^1$H-NMR spectrum (270 MHz, $CDCl_3$, TMS) $\delta$: 1.55-1.8 (m, 17H), 1.95-2.15 (m, 14H+OH), 3.13 (d, J=5Hz, OH), 3.88 (m, 2H), 4.2-4.3 (m, 3H), 5.10 (m,4H).

Viscosity: 0.21 Pa·s (at shear velocity of 92 1/s).

[Example 54]

Synthesis of mono-O-(3,7,11-trimethyldodec-2-enoyl)glycerol

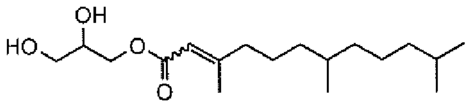

0.90 g (3.5 mmol) of methyl 3,7,11-trimethyldodec-2-enoate was slowly added dropwise to a solution of 0.59 g (6.4 mmol) of glycerol and 0.88 g (6.4 mmol) of potassium carbonate in dry N,N-dimethylformamide (3 mL) at 100°C. After the reaction mixture was stirred at 100°C for 18 hours, 1M hydrochloric acid was added. The resulting solution was extracted with ether, and the extract was washed with saturated sodium bicarbonate aqueous solution and saturated brine, successively, and dried over anhydrous sodium sulfate. After filtration, the filtrate was concentrated, and the resulting residue was purified by silica gel column chromatography (ethyl acetate/hexane mixture) to obtain 318 mg of the title compound (29% yield) as a transparent liquid. $^1$H-NMR and viscosity of the obtained compound were measured. The results were as follows.

$^1$H-NMR spectrum (300 MHz, $CDCl_3$, TMS) $\delta$: 0.80-0.95 (m, 9H), 1.00-1.80 (m,12H), 1.91 and 2.16 (s, 3H, 3-$CH_3$), 2.10-2.20 (m, 2H), 2.61 (brs, OH), 3.50-4.00 (m, 3H), 4.10-4.30 (m, 2H), 5.70 (brs, 1H).

Viscosity: 0.45 Pa·s (at shear velocity of 92 1/s).

Mono-O-(3,7,11-trimethyldodec-2-enoyl)glycerol synthesized was also referred to below as C15 glycerin ester.

[Example 55]

Synthesis of mono-O-(3,7,11,15-tetramethylhexadec-2-enoyl)pentaerythritol

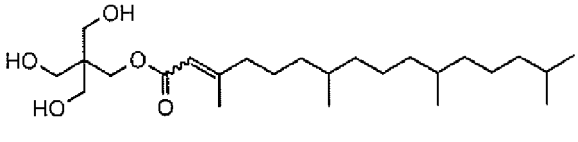

1.0 g (3.1 mmol) of methyl 3,7,11,15-tetramethylhexadec-2-enoate was slowly added dropwise to a solution of 0.84 g (6.2 mmol) of pentaerythritol and 0.85 g (6.2 mmol) of potassium carbonate in dry N,N-dimethylformamide (3 mL) at 80°C. After stirring at 100°C for 12 hours, 1M hydrochloric acid was added to the reaction solution. The resulting solution was extracted with ether, and the extract was washed with saturated sodium bicarbonate aqueous solution and saturated brine, successively, and dried over anhydrous sodium sulfate. After filtration, the filtrate was concentrated, and the resulting residue was purified by silica gel column chromatography (ethyl acetate/hexane mixture) to obtain 537 mg of the title compound (37% yield) as a transparent viscous liquid. $^1$H-NMR and viscosity of the obtained compound were measured. The results were as follows.

$^1$H-NMR spectrum (300 MHz, $CDCl_3$, TMS) $\delta$: 0.80-0.95 (m, 12H), 1.00-1.60 (m, 19H), 1.90-2.20 (m, 5H), 2.71

(brs, 30H), 3.65 (s, 6H), 4.25 (brs, 2H), 5.70 (brs, 1H).

Viscosity: 10.6 Pa·s (at shear velocity of 92 1/s).

Mono-O-(3,7,11,15-tetramethylhexadec-2-enoyl)pentaerythritol synthesized was also referred to below as C20 pentaerythritol ester.

[Example 56]

Synthesis of mono-O-(5,9,13-triomethyltetradec-4-enyl)pentaerythritol

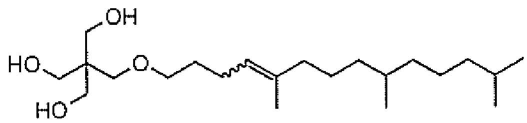

0.82 mL (5.9 mmol) of triethylamine, 0.90 g (4.7 mmol) of p-toluenesulfonyl chloride and 38 mg (0.39 mmol) of trimethylamine hydrochloride were added to a solution of 1.0 g (3.9 mmol) of 5,9,13-trimethyltetradec-4-en-1-ol in dry methylene chloride (3 mL) at 0°C, sequentially, followed by stirring for 1 hour at room temperature. 0.12 mL (1.0 mmol) of N,N-dimethyl-1,3-propanediamine was added to the reaction mixture at 0°C. After stirring for 30 min, the mixture was diluted with ethyl acetate. The solution was washed with water, 1M hydrochloric acid, saturated sodium bicarbonate aqueous solution, and saturated brine, successively, and dried over anhydrous sodium sulfate. After filtration, the filtrate was concentrated to obtain (5,9,13-trimethyltetradec-4-enyl)tosylate as a crude product.

0.26 g (55%, 5.9 mmol) of sodium hydride was added to a solution of 0.80 g (5.9 mmol) of pentaerythritol in dry N,N-dimethylformamide (6 mL) with cooling on ice. After stirring for 30 min at 50°C, the above-mentioned (5,9,13-trimethyltetradec-4-enyl)tosylate was added dropwise, and the mixture was further stirred for 18 hours at the same temperature. After addition of water at 0°C, the reaction mixture was extracted with ethyl acetate. The extract was washed with water, 1M hydrochloric acid, saturated sodium bicarbonate aqueous solution, and saturated brine, successively, and dried over anhydrous sodium sulfate. After filtration, the filtrate was concentrated, and the resulting residue was purified by silica gel column chromatography (ethyl acetate/hexane mixture) to obtain 375 mg of the title compound (26% yield in 2 steps) as a transparent liquid. $^1$H-NMR and viscosity of the obtained compound were measured. The results were as follows.

$^1$H-NMR spectrum (300 MHz, $CDCl_3$, TMS) $\delta$: 0.8-0.9 (m, 9H), 1.00-1.70 (m, 17H), 1.90-2.10 (m, 4H), 2.59 (brs, 30H), 3.42 (t, J=6.4Hz, 2H), 3.47 (s, 2H), 3.73 (d, J=4.5Hz, 6H), 5.09 (t, J=7.0Hz, 1H).

Viscosity: 0.98 Pa·s (at shear velocity of 92 1/s).

Mono-O-(5,9,13-trimethyltetradec-4-enyl)pentaerythriol synthesized was also referred to below as C17 pentaerythritol ether.

[Example 57]

Synthesis of mono-O-(3,7,11,15-tetramethylhexadec-2-enyl)erythritol

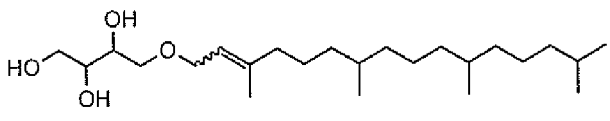

0.90 g (6.7 mmol) of N-chlorosuccinimide was suspended in methylene chloride (8 mL). After addition of 0.52 mL (7.1 mmol) of dimethylsulfide at 0°C, the solution was stirred for 20 min. After addition of 1.0 g (3.4 mmol) of phytol, the mixture was stirred for 1 hour at 0°C, and further stirred for 6 hours at room temperature. After addition of sodium bicarbonate aqueous solution, the reaction mixture was extracted with methylene chloride. The extract was washed with saturated brine, and dried over anhydrous sodium sulfate. After filtration, the filtrate was concentrated to obtain 3,7,11,15-tetramethylhexadec-2-ene-1-chloride as a crude product.

0.20 g (60%, 5.1 mmol) of sodium hydride was added to a solution of 0.62 g (5.1 mmol) of erythritol in dry N,N-dimethylformamide/tetrahydrofuran (1:1, 4 mL) with colling on ice. After stirring for 30 min at 50°C, the above-mentioned 3,7,11,15-tetramethylhexadec-2-ene-1-chloride was added dropwise and further stirred for 20 hours at the same temperature. After addition of water at 0°C, the reaction mixture was extracted with ether. The extract was washed with water, 1M hydrochloric acid, saturated sodium bicarbonate aqueous solution, and saturated brine, successively, and dried over anhydrous sodium sulfate. After filtration, the filtrate was concentrated, and the resulting residue was purified by silica gel column chromatography (ethyl acetate/hexane mixture) to obtain the title compound as a transparent liquid. The results of $^1$H-NMR measurement of the obtained compound are as shown below.

$^1$H-NMR spectrum (300 MHz, $CDCl_3$, TMS) δ: 0.80-0.90 (m, 12H), 1.00-1.80 (m, 22H), 2.00 (t, J=8.6Hz, 2H), 2.34 (brs, 1H, OH), 2.68 (brd, 1H, OH), 2.78 (brd, 1H, OH), 3.50-3.90 (m, 6H), 4.00-4.20 (m, 2H), 5.32 (brs, 1H).

Mono-O-(3,7,11,15-tetramethylhexadec-2-enyl)erythritol synthesized was also referred to below as C20 erythritol ether.

[Example 58]

Synthesis of mono-O-(5,9,13,17-tetramethyloctadec-4-enyl)glycerol

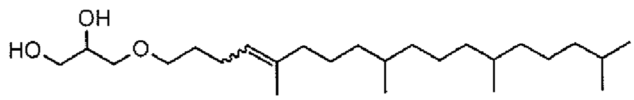

1.28 mL (9.23 mmol) of triethylamine, 1.06 g (5.56 mmol) of p-toluenesulfonyl chloride, and 43 mg (0.45 mmol) of trimethylamine hydrochloride were added to a solution of 1.50 g (4.63 mmol) of 5,9,13,17-tetramethyloctadec-4-en-1-ol in dry methylene chloride (9 mL) at 0°C, sequentially, followed by stirring for 3 hours at room temperature. 0.14 mL (1.1 mmol) of N,N-dimethyl-1,3-propanediamine was added to the reaction mixture at 0°C. After stirring for 3 hours, the mixture was diluted with ethyl acetate. The resulting solution was washed with water, 1M hydrochloric acid, saturated sodium bicarbonate aqueous solution, and saturated brine, successively, and dried over anhydrous sodium sulfate. After filtration, the filtrate was concentrated to obtain (5,9,13,17-tetramethyloctadec-4-enyl)tosylate as a crude product.

0.37 g (60%, 9.2 mmol) of sodium hydride was added to a solution of 0.851 g (9.24 mmol) of glycerol in dry N,N-dimethylformamide (6 mL) with cooling on ice. After stirring for 1 hour at 50°C, the above-mentioned (5,9,13,17-tetramethyloctadec-4-enyl)tosylate was added dropwise thereto, and the mixture was further stirred for 20 hours at 60°C. After addition of water at 0°C, the reaction mixture was extracted with ethyl acetate. The extract was washed with water, 1M hydrochloric acid, saturated sodium bicarbonate aqueous solution, and saturated brine, successively, and dried over anhydrous sodium sulfate. After filtration, the filtrate was concentrated, and the resulting residue was purified by silica gel column chromatography (methanol/methylene chloride mixture) to obtain 494 mg of the title compound (19% yield in 2 steps) as a transparent liquid. $^1$H-NMR and viscosity of the obtained compound were measured. The results were as follows.

$^1$H-NMR spectrum (300 MHz, $CDCl_3$, TMS) δ: 0.80-0.90 (m, 12H), 1.00-1.70 (m, 24H), 1.90-2.10 (m, 4H), 2.19 (dd, J=4.8, 7.2Hz, 1H, OH), 2.63 (d, J=5.1Hz, 1H, OH), 3.40-3.90 (m, 7H), 5.10 (t, J=7.2Hz, 1H).

Viscosity: 0.44 Pa·s (at shear velocity of 92 1/s).

Mono-O-(5,9,13,17-tetramethyloctadec-4-enyl)glycerol synthesized was also referred to below as C22 glycerin ether.

[Example 59]

Formation of liquid crystals and analysis thereof

(1) Liquid crystal gels

Each of the compounds synthesized in Example 32, 33, 43, 45, 49, and 50 and water were homogeneously mixed in accordance with the same procedure as in Example 9 to obtain a sample of each compound/water system being a gel in appearance. SAXS analysis of these gel samples was performed in the same manner as in Example 9.

For the compounds synthesized in Examples 32, 33, and 45, at least 3 scattering peaks were observed. The peak value ratios indicated the following ratio peculiar to the cubic liquid crystal belonging to the crystallographic space group Pn3m:

$$\sqrt{2}:\sqrt{3}:\sqrt{4}:\sqrt{6}:\sqrt{8}:\sqrt{9}\ .$$

Thus, the compound/water system samples were each confirmed to form cubic liquid crystals that belong to the crystallographic space group Pn3m. The results of SAXS analysis are shown in Figure 7 for the compound of Example 32/water system, in Figure 8 for the compound of Example 33/water system, and in Figure 9 for the compound of Example 45/water system.

For the compounds synthesized in Examples 43, 49, and 50, at least 3 scattering peaks were observed. The peak value ratios indicated the following ratio peculiar to the reverse hexagonal liquid crystal:

$$1:\sqrt{3}:2\ .$$

Thus, the compound/water system samples were each confirmed to form reverse hexagonal liquid crystals. The results of SAXS analysis are shown in Figure 10 for the compound of Example 43/water system, in Figure 11 for the compound of Example 49/water system, and in Figure 12 for the compound of Example 50/water system.

(2) O/W Dispersion (Liquid crystal emulsion)

Pluronic (Unilube 70DP-950B, NOF Corporation) and ethanol were added to C17 glycerin ester, followed by stirring for 1 hour using a stirrer chip. Then, distilled water was added and the mixture was further stirred for 1 hour to prepare an o/w dispersion. The mixture ratio (weight ratio) of C17 glycerin ester: Pluronic: ethanol: distilled water was 13.5: 3.4: 1.9: 80.2.

SAXS analysis of the o/w dispersion prepared was performed in the same manner as in Example 9. The result of SAXS analysis is shown in Figure 13. From Figure 13, the o/w dispersion was shown to be a liquid crystal emulsion of cubic liquid crystal that belongs to the crystallographic space group Pn3m.

[Example 60]

Measurement of viscosity of gels

The viscosity was measured for the gels prepared by addition of water to the compounds synthesized in Examples 45 and 50, and C17 glycerin ester synthesized in Example 13. Specifically, for the gel samples obtained by mixing homogeneously according to the same procedure as in Example 9, the shear viscosity was measured at a temperature of 25°C, using a viscosity and viscoelasticity measuring apparatus (Gemini II, Malvern Instruments Ltd.; cone plate φ25, cone angle 1°).

The measurement results at shear velocity of 10 1/s and 105 1/s are shown in Table 7.

Table 7

| Compound | Shear velocity (1/s) | Viscosity (Pas·sec) |
|---|---|---|
| Example 45 | 10<br>105 | 314<br>4.3 |
| Example 50 | 10<br>105 | 81<br>14 |
| C17 glycerin ester | 10<br>105 | 1878<br>166 |

When compared with the viscosities of the lipid compounds themselves (see Examples 45 and 50, and Examples 13), the viscosities of the gels were greatly increased. It was verified that liquid crystal gels were actually formed.

[Example 61]

Preparation of pump spray agent without ethanol

An o/w dispersion (o/w dispersion 1) of C17 glycerin ester as a lipid was prepared as a pump spray agent

sample, using the same procedure as employed in Example 18(1), but without using ethanol. The mixture ratio (weight ratio) of C17 glycerin ester: Pluronic: distilled water was 6: 2: 92.

Furthermore, an o/w dispersion (o/w dispersion 2) of C17 glycerin ester as a lipid was prepared as a pump spray agent sample, using the same procedure as employed in Example 18(2), but without using ethanol. The mixture ratio (weight ratio) of C17 glycerin ester: Pluronic: distilled water: sodium hyaluronate was 13.5: 3.375: 83.025: 0.1.

[Example 62]

Evaluation of adhesion preventing effect

The o/w dispersion 1 prepared in Example 61 was applied to the suture site of the rat peritoneum by using it as a pump spray agent in the same manner as in Example 21, and then adhesion evaluation was performed. Adhesion evaluation was also performed for an untreated rat group in the same manner as in Example 21. Adhesion evaluation was also performed using o/w dispersion 2 prepared in Example 61 in the same manner as in Example 21. In addition, o/w dispersion 1 was applied to 9 subjects, o/w dispersion 2 was applied to 12 subjects, and adhesion evaluation was performed.

As a result, the average score of adhesion evaluation for o/w dispersion 1 was 2.22, whereas the average score of adhesion evaluation for the untreated group was 2.89. On the other hand, the average score of adhesion evaluation for o/w dispersion 2 was 2.08, whereas the average score of adhesion evaluation for the untreated group was 2.83. Thus, it was shown that the dispersions without ethanol also exhibited a good adhesion preventing effect.

[Example 63]

Analysis of tissue sections

In test groups, test sample 18 and test sample 13 prepared in Example 18 were applied separately to the suture sites in the same manner as in Example 18(3). In the untreated group, an incision was made, but no sample was applied to the suture site. Furthermore, in a comparative group, Seprafilm was applied in the same manner as in Comparative Example 3.

Tissue observations of the tissue sections of the suture sites 7 days after surgery were as follows. Herein, +, ++, and +++ indicate that the levels of fibrosis/inflammation/angiogenesis increase in the following order: + < ++ < +++.

Table 8

| | | | | | | |
|---|---|---|---|---|---|---|
| Test sample 13 (C17 glycerin ester; dimethicone formulation) | | | | | | |
| fibrosis | +: | 1 case | ++: | 6 cases | +++: | 3 cases |
| inflammation | +: | 1 case | ++: | 4 cases | +++: | 5 cases |
| angiogenesis | +: | 1 case | ++: | 5 cases | +++: | 4 cases |
| Test sample 18 (C17 glycerin ester; o/w dispersion) | | | | | | |
| fibrosis | +: | 1 case | ++: | 4 cases | +++: | 5 cases |
| inflammation | +: | 1 case | ++: | 5 cases | +++: | 4 cases |
| angiogenesis | +: | 1 case | ++: | 5 cases | +++: | 4 cases |
| Seprafilm (comparative group) | | | | | | |
| fibrosis | +: | 1 case | ++: | 7 cases | +++: | 2 cases |
| inflammation | +: | 2 cases | ++: | 5 cases | +++: | 3 cases |
| angiogenesis | +: | 1 case | ++: | 4 cases | +++: | 5 cases |
| Untreated group | | | | | | |
| fibrosis | +: | 1 case | ++: | 5 cases | +++: | 4 cases |
| inflammation | +: | 1 case | ++: | 5 cases | +++: | 4 cases |
| angiogenesis | +: | 1 case | ++: | 5 cases | +++: | 4 cases |

Almost the same, some degrees of fibrosis, inflammation, and angiogenesis were locally observed in all groups. Thus, it was shown that the lipid compounds themselves according to the present invention do not inhibit biological

reactions (inflammation reactions), and do not elicit inflammation. It was considered that the inflammation was not caused by applying the test samples or Seprafilm, but by an invasion by the peritoneum incision or a foreign-body reaction with silk sutures. Although the inflammation was observed, the adhesion preventing effects were verified as shown in Example 21 by application of test samples 13 and 18. This indicates that a coating of the liquid crystal gels containing the lipid compounds according to the present invention serves a physical barrier function to reduce adhesions derived from inflammation.

[Example 64]

Test of bioadhesive property

The lipid compounds according to the present invention (adhesion preventing agents) form coatings of non-lamellar liquid crystals together with water. It was expected that the liquid crystal coatings formed on affected areas by the adhesion preventing agents according to the present invention would remain on the affected areas for a certain period without run off, due to the bioadhesive property. The bioadhesive property of the non-lamellar liquid crystals is important when the non-lamellar liquid crystals exhibit adhesion preventing effects.

Accordingly, the bioadhesive property of the liquid crystal gels formed by the lipid compounds according to the present invention was evaluated in rat peritoneal sections.

Specifically, first, four corners of a rat peritoneal section (square of about 1.5 cm x 1.5 cm) were pinned on a rubber plate in a dish, and 1 mL of PBS (+) (commercially available from Aldrich) was applied thereto, followed by allowing to stand for 5 minutes. After draining PBS (+) on the peritoneum, an evaluation lipid (20 mg) was applied thereto. Then, 1mL of PBS (+) was added slowly onto the applied part, followed by allowing to stand for 10 minutes to form a liquid crystal gel on the peritoneum. The peritoneal section prepared in this way and the PBS (+) solution in the dish were placed into No. 7 screw tube containing 5 mL of PBS (+) and a stirring bar, followed by stirring (400rpm) gently for 1 hour at 37°C in water bath.

After removal of the peritoneum section, 2.5 mL of ethyl acetate was added to the remaining aqueous solution in the No. 7 screw tube, and liquid separation was performed for extraction. After the extract was dried over sodium sulfate, filtered, and the filtrate was concentrated under reduced pressure, and the resulting residue was diluted with 0.1 mL of ethyl acetate. TLC analysis of the solution was performed for detecting the evaluation lipid.

The evaluation lipids and the results of the TLC analysis are shown in the Table below.

Table 9

| Evaluation lipid | TLC analysis |
|---|---|
| Ester | |
| C15 glycerin ester (Example 54) | S |
| saturated C17 glycerin ester (Example 8) | N |
| C17 glycerin ester (Example 13) | N |
| glyceryl farnesylacetate (Example 1) | S |
| mono-O-(5,9,13,17-tetramethyloctadeca-4,8,12,16-tetraenoyl)glycerol (Example 4) | S |
| C20 pentaerythritol ester (Example 55) | N |
| mono-O-(5,9,13,17-tetramethyloctadeca-4,8,12,16-tetraenoyl)erythritol (Example 5) | N |
| C22 diglycerin ester (Example 14) | N |
| Ether or Glycoside | |
| C22 glycerin ether (Example 58) | N |
| C17 pentaerythritol ether (Example 56) | N |
| mono-O-(5,9,13,17-tetramethyloctadeca-4,8,12,16-tetraenyl)pentaerythritol (Example 50) | N |
| C20 erythritol ether (Example 57) | N |
| mono-O-(3,7,11,15-tetramethylhexadeca-2,6,10,14-tetraenyl)erythritol (Example 45) | S |

(continued)

| Evaluation lipid | TLC analysis |
|---|---|
| β-XP (Example 15) | N |
| N: not substantially detected<br>S: detected only in a small amount | |

As shown in Table 9, the evaluation lipids were not substantially detected, or detected only in a small amount in TLC analysis. It was shown by the results that most of the applied evaluation lipids according to the present invention remained on the peritoneal sections. Therefore, it was verified that the lipid compounds according to the present invention form a coating of the liquid crystal gels having high bioadhesive properties.

For comparison, 18.0 mg of 5% sodium hyaluronate aqueous solution (gel) instead of a lipid was applied to a rat peritoneal section, and PBS (+) was applied thereon, in the same manner as described above. The gel of 5% sodium hyaluronate aqueous solution was prepared by dissolving sodium hyaluronate (hyaluronic acid FCH (FCH-80), Kikkoman Biochemifa Company) in sterile water. Observation of the treated peritoneum section was perfomed. As a result, the gel did not remain in appearance, and the sample was not recovered at all by scraping with a spatula.

Industrial Applicability

The adhesion preventing agent according to the present invention can be applied to an area at risk of tissue adhesion via a simple method such as spraying or spreading, in order to prevent adhesion. This enables an application by spraying, spreading or the like using a simple container, without the use of conventional, hard-to-use adhesion preventing agents available in film or sheet forms. Therefore, the present invention makes the use of adhesion preventing agents easier, for example, in endoscopic surgery and laparoscopic surgery. Further, since the compounds of the present invention has low viscosity, they can be advantageously used in spray agents, injections, and the like. The compounds of the present invention are low-molecular-weight compounds and can be filter-sterilized, and therefore the present invention is also advantageous in simplifying steps for producing an adhesion preventing agent.

All publications, patents, and patent applications cited herein are incorporated herein by reference in their entirety.

**Claims**

1. An adhesion preventing agent comprising an amphipathic compound having the following general formula (I):

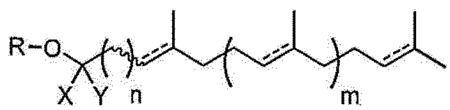

(I)

wherein X and Y each denotes a hydrogen atom or together denote an oxygen atom, n denotes an integer from 0 to 2, m denotes the integer 1 or 2, the designation:



denotes a single bond or double bond, and R denotes a hydrophilic group having two or more hydroxyl groups.

2. The adhesion preventing agent according to claim 1, wherein n denotes the integer 1 or 2 in the formula.

3. The adhesion preventing agent according to claim 1 or 2, wherein R in the formula denotes a hydrophilic group generated by removal of one hydroxyl group from any one selected from the group consisting of glycerol, erythritol, pentaerythritol, diglycerol, glycerin acid, triglycerol, xylose, sorbitol, ascorbic acid, glucose, galactose, mannose, dipentaerythritol, maltose, mannitol, and xylitol.

4. The adhesion preventing agent according to any one of claims 1 to 3, wherein R in the formula denotes a hydrophilic

group generated by removal of one hydroxyl group from glycerol, erythritol, diglycerol, or xylose.

5. The adhesion preventing agent according to any one of claims 1 to 4, wherein the amphipathic compound is any one of the following compounds:

mono-O-(5,9,13-trimethyltetradec-4-enoyl)glycerol,
mono-O-(5,9,13,17-tetramethyloctadec-4-enoyl)diglycerol,
mono-O-(5,9,13-trimethyltetradecanoyl)glycerol,
mono-O-(5,9,13,17-tetramethyloctadecanoyl)erythritol,
1-O-(3,7,11,15-tetramethylhexadecyl)-β-D-xylopyranoside, and
mono-O-(5,9,13-trimethyltetradec-4,8,12-trienoyl)glycerol.

6. The adhesion preventing agent according to any one of claims 1 to 5, further comprising a pharmaceutically acceptable carrier.

7. The adhesion preventing agent according to claim 6, wherein the carrier is a liquid carrier and/or a gas carrier.

8. The adhesion preventing agent according to claim 7, wherein the liquid carrier comprises at least one selected from the group consisting of silicone oil, alcohol, and an aqueous medium.

9. The adhesion preventing agent according to any one of claims 1 to 8, further comprising a pharmaceutically acceptable surfactant.

10. The adhesion preventing agent according to any one of claims 1 to 9, comprising hyaluronic acid or a salt thereof.

11. An amphipathic compound having the following general formula (II) or a salt thereof:

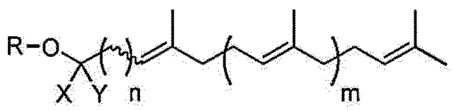

(I I)

wherein X and Y each denotes a hydrogen atom or together denote an oxygen atom, n denotes an integer from 0 to 2, m denotes the integer 1 or 2, and R denotes a hydrophilic group generated by removal of one hydroxyl group from any one selected from the group consisting of glycerol, erythritol, pentaerythritol, diglycerol, glyceric acid, and xylose.

12. The compound or salt thereof according to claim 11, wherein n denotes 1 or 2 and m denotes 2 in the formula.

13. The compound or salt thereof according to claim 11, wherein the compound is any one of the following compounds:

mono-O-(5,9,13,17-tetramethyloctadec-4,8,12,16-tetraenoyl)glycerol,
mono-O-(5,9,13,17-tetramethyloctadec-4,8,12,16-tetraenoyl)erythutol,
mono-O-(5,9,13,17-tetramethyloctadec-4,8,12,16-tetraenoyl)pentaerythritol,
mono-O-(5,9,13-trimethyltetradec-4,8,12-trienyl)erythritol,
mono-O-(5,9,13-trimethyltetradec-4,8,12-trienyl)pentaerythritol,
mono-O-(3,7,11,15-tetramethylhexadec-2,6,10,14-tetraenoyl)pentaerythritol,
mono-O-(3,7,11,15-tetramethylhexadec-2,6,10,14-tetraenyl)erythritol,
mono-O-(5,9,13,17-tetramethyloctadec-4,8,12,16-tetraenyl)erythritol, and
mono-O-(5,9,13,17-tetramethyloctadec-4,8,12,16-tetraenyl)pentaerythritol.

14. A method for preventing adhesion of an affected area, comprising applying the adhesion preventing agent according to any one of claims 1 to 10 to the affected area.

Fig. 1

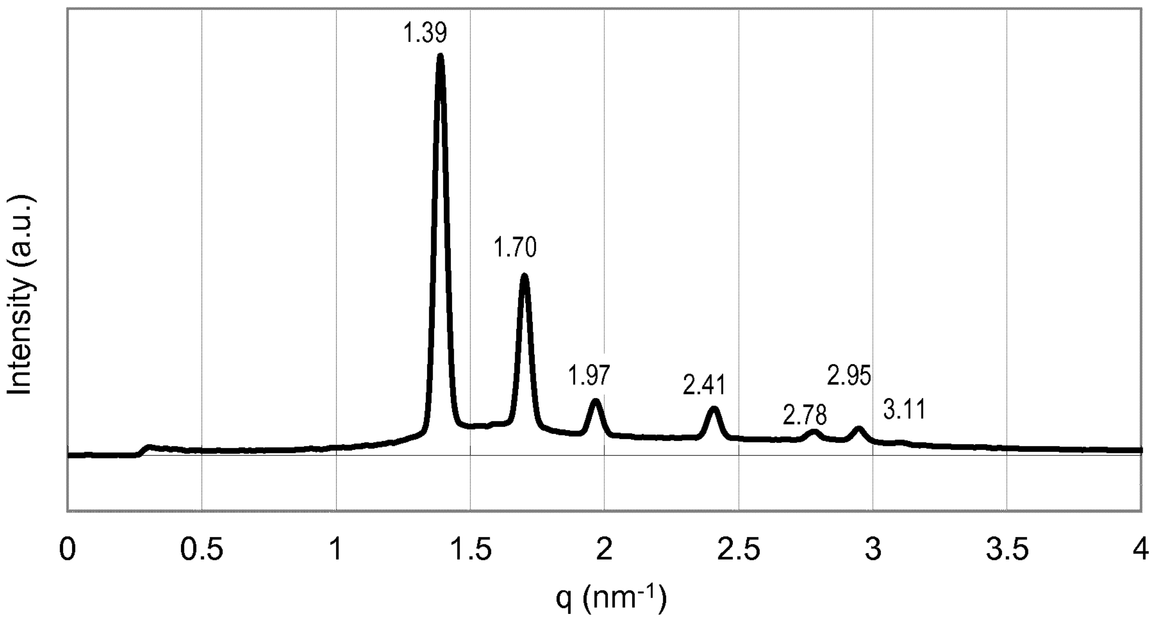

Fig. 2

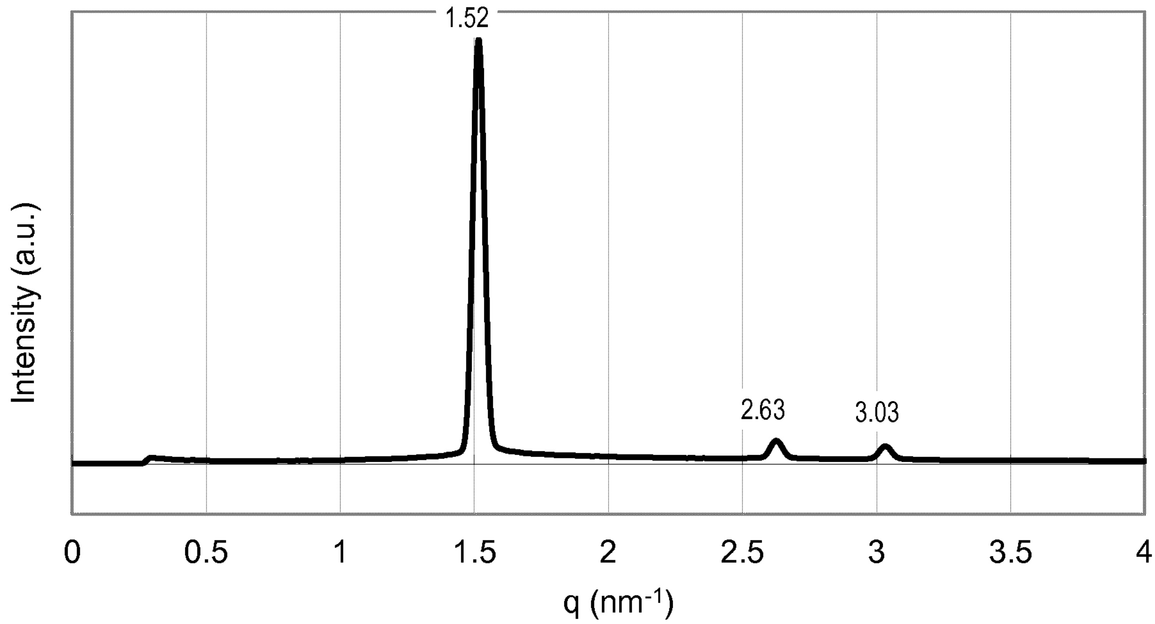

Fig. 3

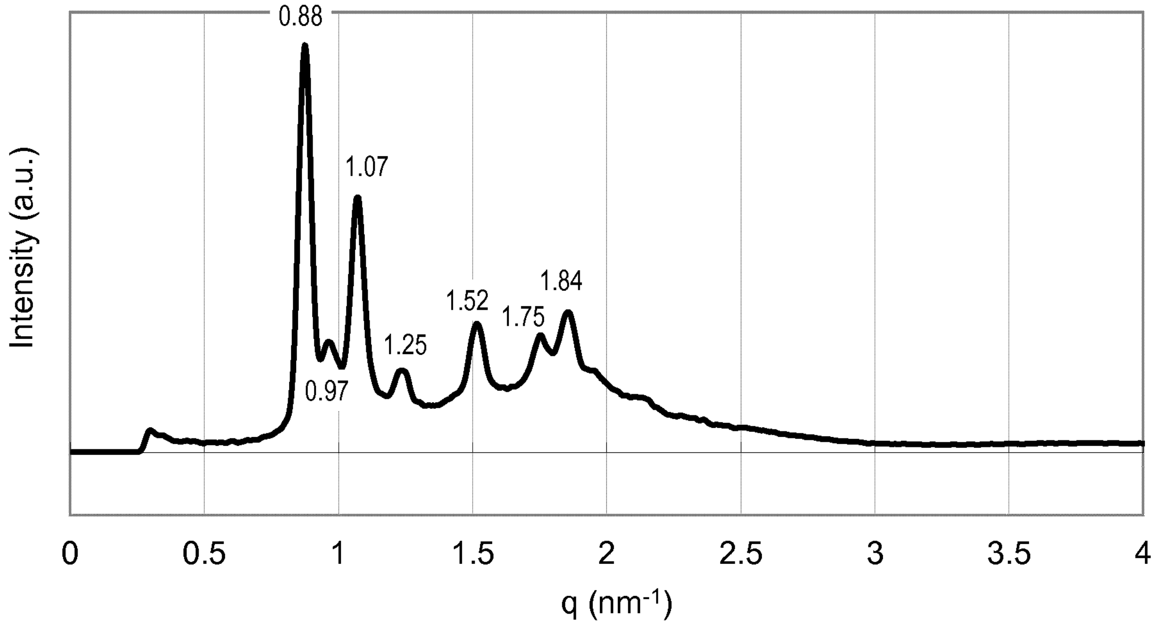

Fig. 4

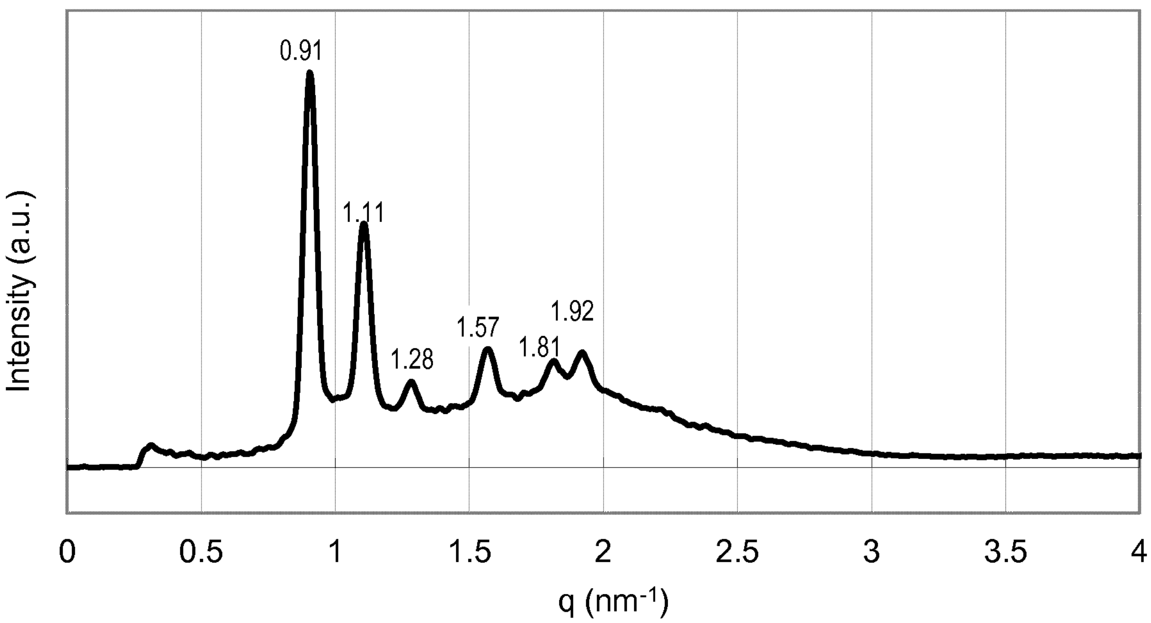

Fig. 5

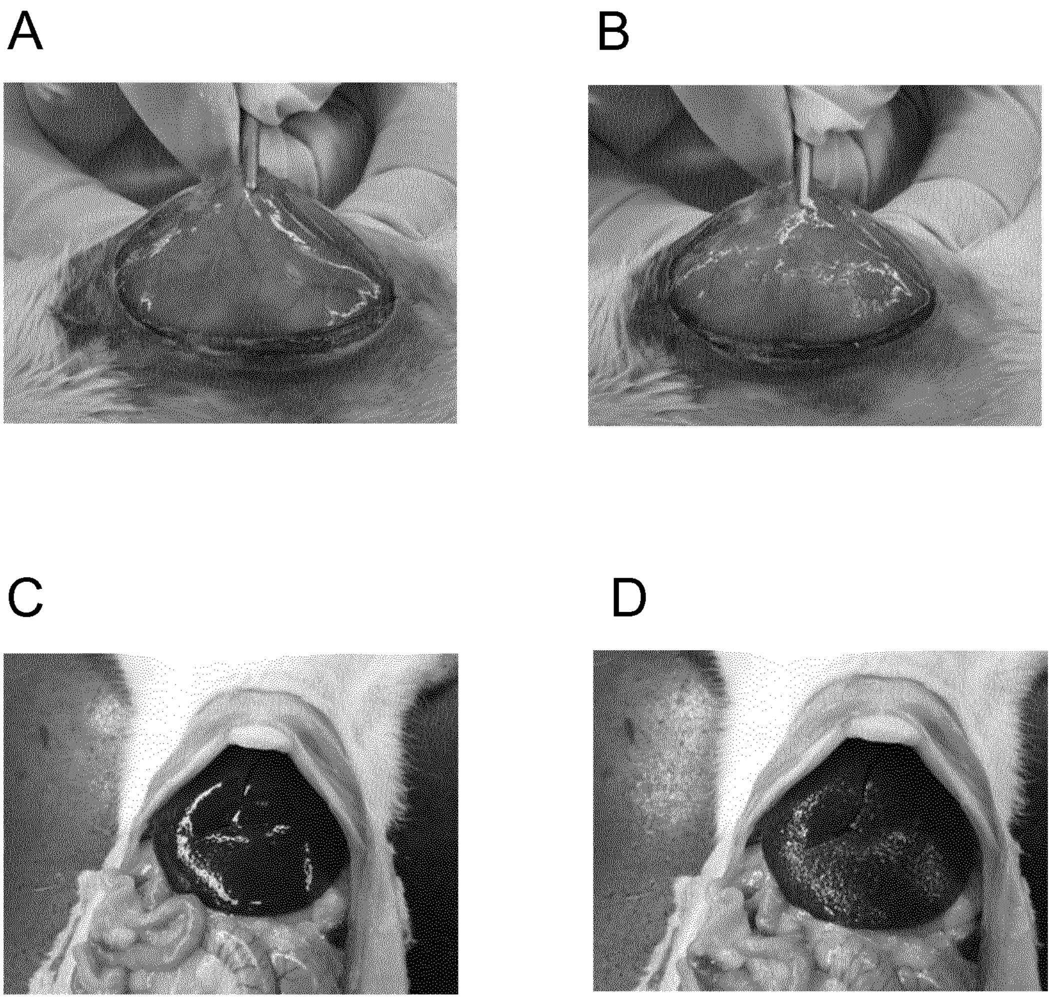

Fig. 6

A

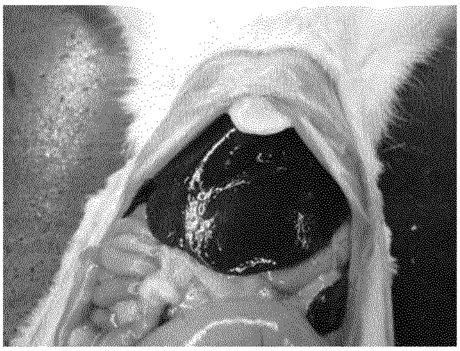

B

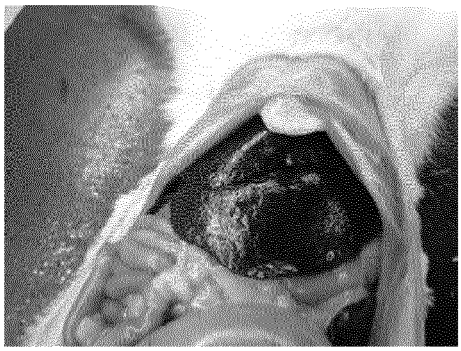

Fig. 7

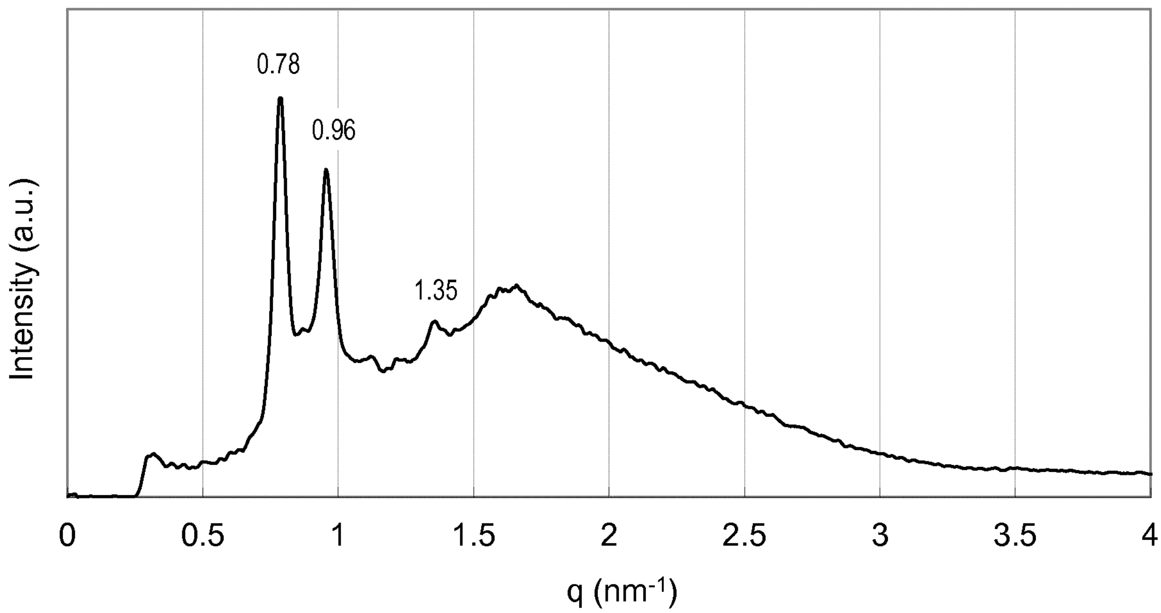

Fig. 8

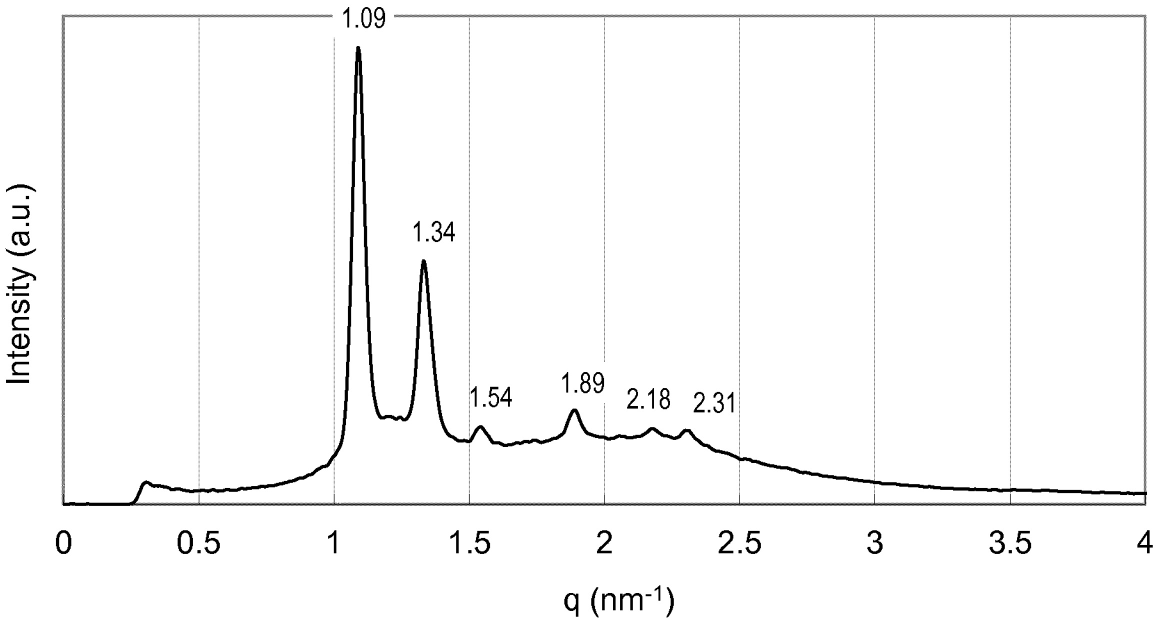

Fig. 9

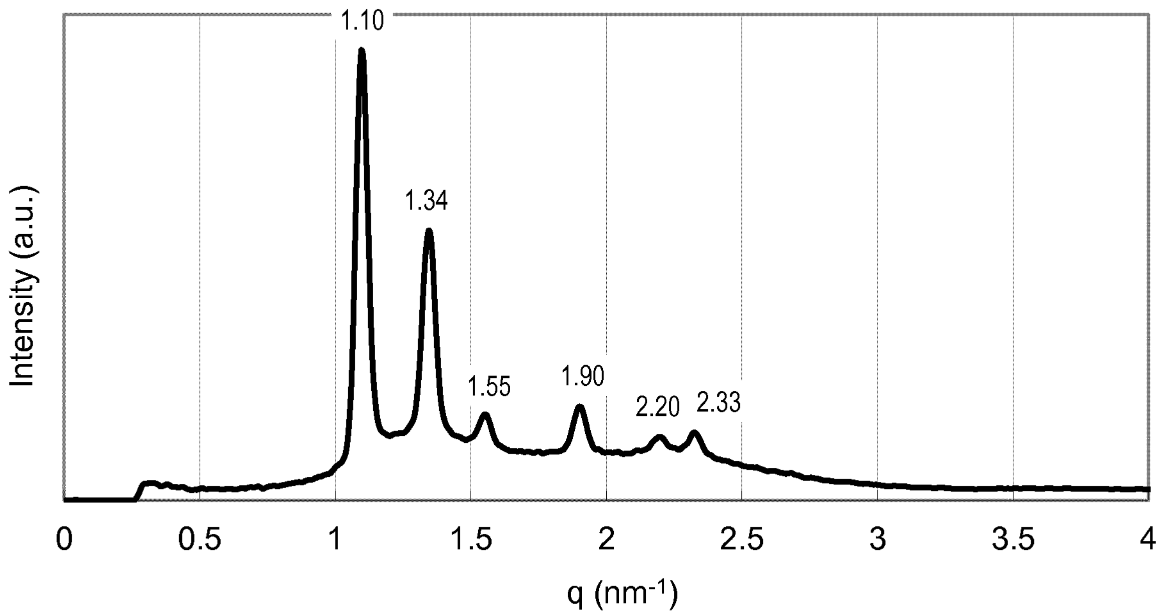

Fig. 10

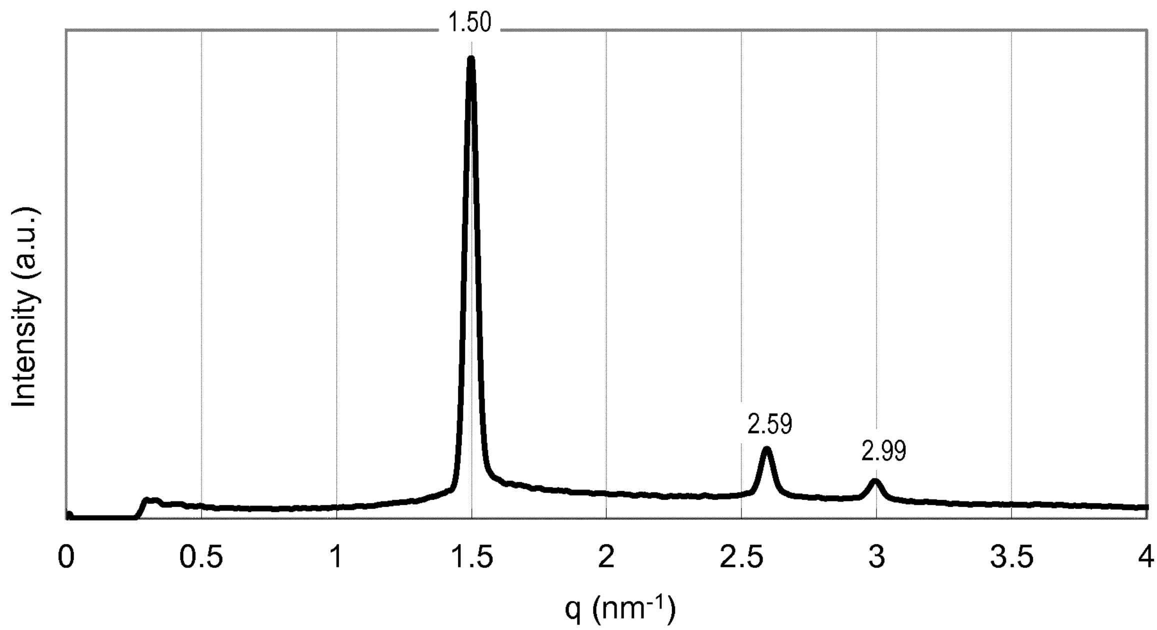

Fig. 11

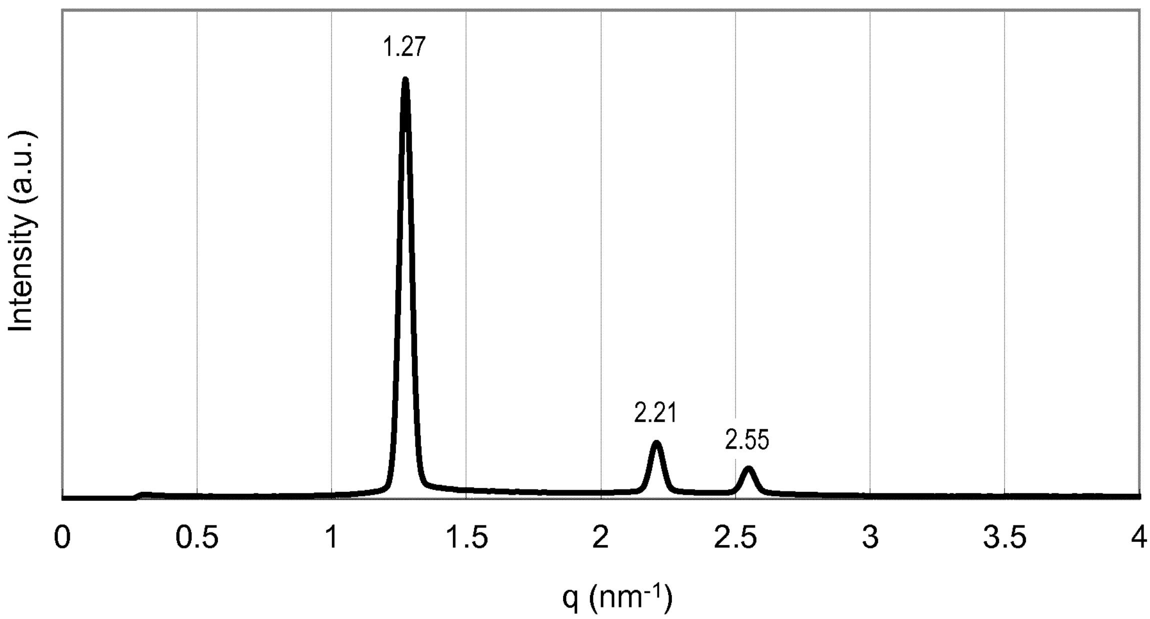

Fig. 12

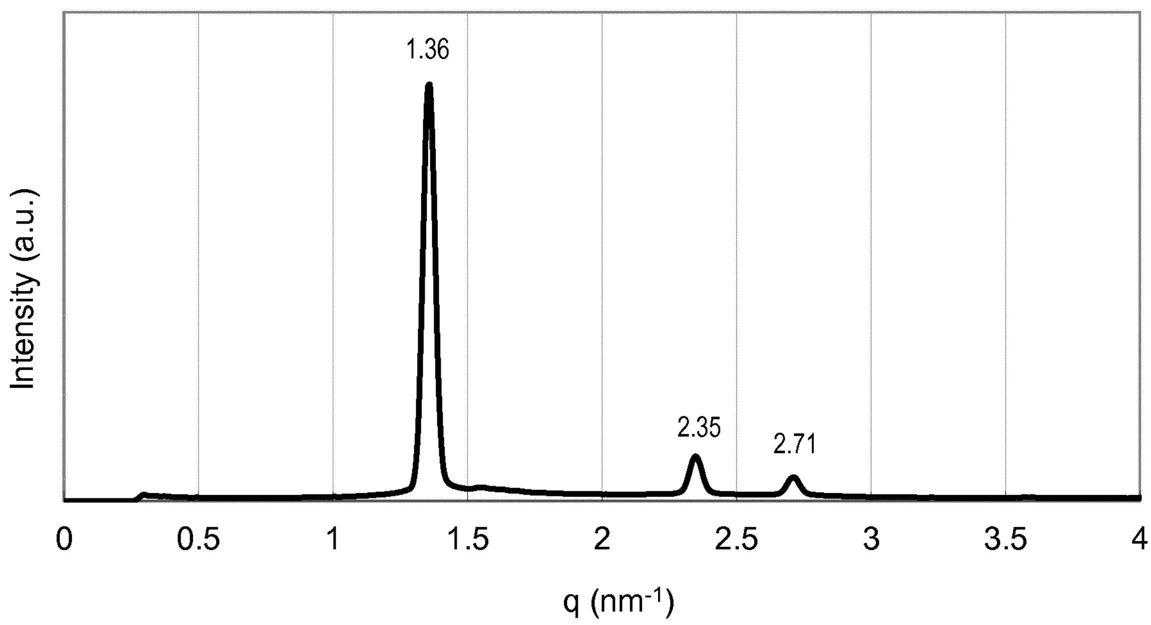

Fig. 13

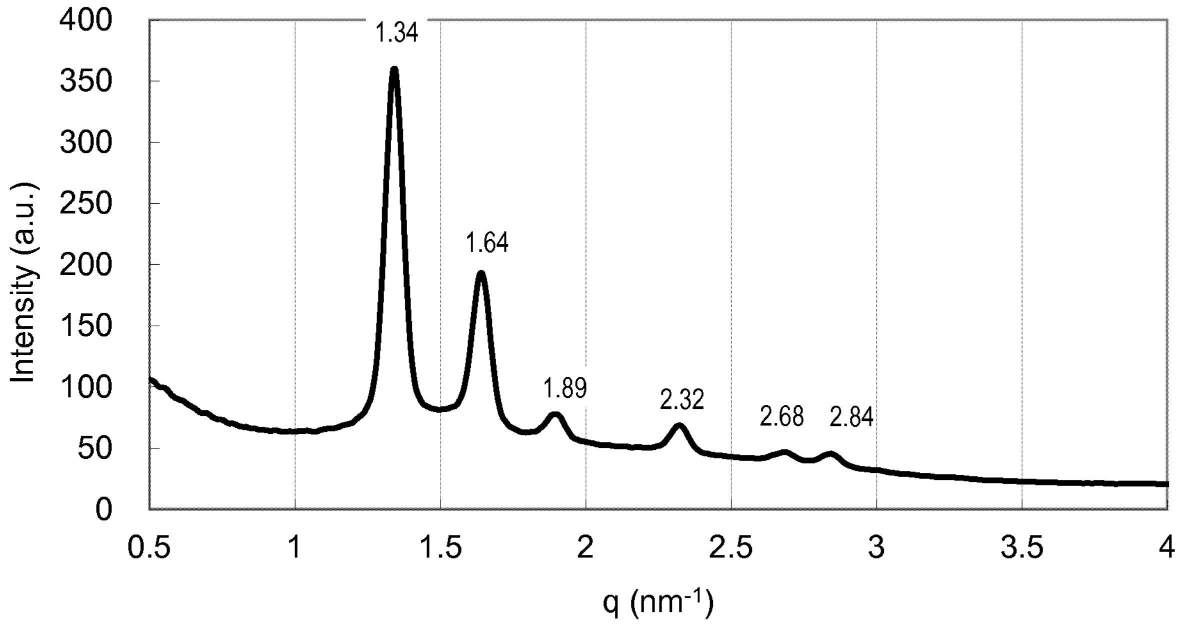
400
350
300
250
200
150
100
50
0
Intensity (a.u.)
1.34
1.64
1.89
2.32
2.68
2.84
0.5
1
1.5
2
2.5
3
3.5
4
q (nm-1)

## INTERNATIONAL SEARCH REPORT

International application No.
PCT/JP2014/059785

A. CLASSIFICATION OF SUBJECT MATTER
*A61L31/00*(2006.01)i, *A61K31/23*(2006.01)i, *A61K31/232*(2006.01)i, *A61K47/10*(2006.01)i, *A61K47/26*(2006.01)i, *A61K47/34*(2006.01)i, *A61K47/36*(2006.01)i, *A61K47/44*(2006.01)i, *A61L33/00*(2006.01)i,
According to International Patent Classification (IPC) or to both national classification and IPC

B. FIELDS SEARCHED
Minimum documentation searched (classification system followed by classification symbols)
A61L31/00, A61K31/23, A61K31/232, A61K47/10, A61K47/26, A61K47/34, A61K47/36, A61K47/44, A61L33/00, A61P41/00, C07C69/587, A61K8/37

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>A | JP 2004-59470 A (Kanebo, Ltd.),<br>26 February 2004 (26.02.2004),<br>entire text; particularly, example 3; claim 1, general formula (3)<br>(Family: none) | 11<br>1-10,12,13 |
| X<br>A | JP 60-28946 A (Kao Soap Co., Ltd.),<br>14 February 1985 (14.02.1985),<br>entire text; particularly, table 2, uppermost part, compounds; claim 1, general formula (1)<br>(Family: none) | 11<br>1-10,12,13 |
| X<br>A | JP 60-28945 A (Kao Soap Co., Ltd.),<br>14 February 1985 (14.02.1985),<br>entire text; particularly, table 2, uppermost part, compounds; claim 1, general formula (1)<br>(Family: none) | 11<br>1-10,12,13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 June, 2014 (16.06.14) | 24 June, 2014 (24.06.14) |
| Name and mailing address of the ISA/<br>Japanese Patent Office<br>Facsimile No. | Authorized officer<br>Telephone No. |

Form PCT/ISA/210 (second sheet) (July 2009)

**INTERNATIONAL SEARCH REPORT**

International application No. PCT/JP2014/059785

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Appendino, G. et al, Homologues and isomers of noladin ether, a putative novel endocannabinoid: interaction with rat cannabinoid CB1 receptors, Bioorganic & Medicinal Chemistry Letters, 2003, Vol.13, No. 1, pp.43-46, entire text, particularly, Table 1, Entry 7I | 11<br>1-10,12,13 |
| X<br>A | Ungur, N. et al, Absolute stereochemistry of natural sesquiterpenoid diacylglycerols, Tetrahedron: Asymmetry, Vol.10, No.7, 1999, pp. 1263-1273, entire text, particularly, R in 1h, 2h, 1i, 2i of Scheme 2 is a compound of 1d-1n | 11<br>1-10,12,13 |
| X<br>A | Lugemwa, FN. et al, Estradiol β-D-xyloside, an efficient primer for heparan sulfate biosynthesis, Journal of Biological Chemistry, 1991, Vol.266, No.11, pp.6674-7, entire text, particularly, page 6675, left column, Result, line 7 | 11<br>1-10,12,13 |
| A | WO 2006/043705 A1 (National Institute of Advanced Industrial Science and Technology), 27 April 2006 (27.04.2006), entire text; particularly, claims<br>& US 2008/0113923 A1 & EP 1813287 A1<br>entire text; particularly, claims | 1-13 |
| A | WO 2011/078383 A1 (Cytopathfinder Inc.), 30 June 2011 (30.06.2011), entire text; particularly, claims<br>& JP 5207420 B & JP 2013-129660 A<br>& US 2012/0264923 A1 & EP 2518040 A1<br>entire text; particularly, claims | 1-13 |
| P,A | Kenjiro HIRAI et al., "Hi-Lamella Ekisho Shishitsu Spray ni yoru Yuchaku Boshi Koka no Kento", Journal of Japan Surgical Society, 05 March 2014 (05.03.2014), vol.115, special extra issue (2), page 346, entire text | 1-13 |

Form PCT/ISA/210 (continuation of second sheet) (July 2009)

**INTERNATIONAL SEARCH REPORT**

International application No.
PCT/JP2014/059785

Continuation of A. CLASSIFICATION OF SUBJECT MATTER
(International Patent Classification (IPC))

*A61P41/00*(2006.01)i, *C07C69/587*(2006.01)i, *A61K8/37*(2006.01)n

(According to International Patent Classification (IPC) or to both national classification and IPC)

Form PCT/ISA/210 (extra sheet) (July 2009)

**INTERNATIONAL SEARCH REPORT**

International application No.
PCT/JP2014/059785

**Box No. II Observations where certain claims were found unsearchable (Continuation of item 2 of first sheet)**

This international search report has not been established in respect of certain claims under Article 17(2)(a) for the following reasons:

1. [x] Claims Nos.: 14
because they relate to subject matter not required to be searched by this Authority, namely:
Claim 14 involves "a method for treatment of the human body or animal body by surgery or therapy".

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010119994 A
- WO 2005087289 A
- WO 2006077362 A
- WO 2006043705 A
- WO 2011078383 A
- JP 2013096499 A
- JP S5129431 A
- JP S5129434 A
- JP S5129435 A
- JP S5129436 A
- JP S5134108 A
- JP 2008528463 A
- WO 0047079 A